(19) Europäisches Patentamt
European Patent Office
Office européen des brevets

(11) EP 4 759 804 A1

(12) **EUROPEAN PATENT APPLICATION**
published in accordance with Art. 153(4) EPC

(43) Date of publication:
**17.06.2026 Bulletin 2026/25**

(21) Application number: **24851443.2**

(22) Date of filing: **26.06.2024**

(51) International Patent Classification (IPC):
***C07D 231/18*** (2006.01) ***A01N 47/24*** (2006.01)
***A01P 3/00*** (2006.01) ***C07D 231/20*** (2006.01)
***C07D 233/90*** (2006.01) ***C07D 249/04*** (2006.01)
***C07D 401/12*** (2006.01) ***C07D 405/12*** (2006.01)
***C07D 409/12*** (2006.01) ***C07D 417/12*** (2006.01)

(52) Cooperative Patent Classification (CPC):
**A01N 47/24; A01P 3/00; C07D 231/18; C07D 231/20; C07D 233/90; C07D 249/04; C07D 401/12; C07D 405/12; C07D 409/12; C07D 417/12**

(86) International application number:
**PCT/JP2024/023210**

(87) International publication number:
**WO 2025/033011 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.08.2023 JP 2023131664**
**22.01.2024 JP 2024007491**
**19.04.2024 JP 2024068592**

(71) Applicant: **Nippon Soda Co., Ltd.**
**Tokyo 100-7010 (JP)**

(72) Inventors:
- **KOBAYASHI, Hiroyuki**
**Odawara-shi, Kanagawa 250-0280 (JP)**
- **NISHIKI, Haruka**
**Odawara-shi, Kanagawa 250-0280 (JP)**
- **KASHIWA, Shuntaro**
**Odawara-shi, Kanagawa 250-0280 (JP)**
- **SHIBAYAMA, Kotaro**
**Odawara-shi, Kanagawa 250-0280 (JP)**
- **SUZUKI, Hiroto**
**Odawara-shi, Kanagawa 250-0280 (JP)**
- **NAKAMURA, Yuka**
**Odawara-shi, Kanagawa 250-0280 (JP)**
- **SAIGA, Tomoyuki**
**Odawara-shi, Kanagawa 250-0280 (JP)**
- **KUBOTA, Kento**
**Odawara-shi, Kanagawa 250-0280 (JP)**

(74) Representative: **Mewburn Ellis LLP**
**Aurora Building**
**Counterslip**
**Bristol BS1 6BX (GB)**

(54) **FIVE-MEMBERED HETEROARYL COMPOUND AND AGRICULTURAL AND HORTICULTURAL BACTERICIDE**

(57) This five-membered heteroaryl compound is represented formula (Z-1) (wherein $Q^1$ and $Q^2$ each independently represent a phenyl group or the like; $B^1$, $B^2$ and $B^3$ each independently represent an oxygen atom, a nitrogen atom, $NR^1$, or $CR^2$; $R^1$ represents an alkyl group or the like; $R^2$ represents a hydrogen atom or the like; $Z^a$ represents an oxygen atom or the like; T represents a partial structure (T-1) or (T-2); Z represents an oxygen atom or the like; $R^6$ represents a hydrogen atom or the like; $Z^b$ represents an alkoxy group or the like; $R^7$ represents $R^cCO$- or the like; $R^c$ represents an alkyl group or the like; $R^4$ and $R^5$ each independently represent a hydrogen atom or the like; and n represents an integer of 1 to 3).

EP 4 759 804 A1

[Chemical Formula 1]

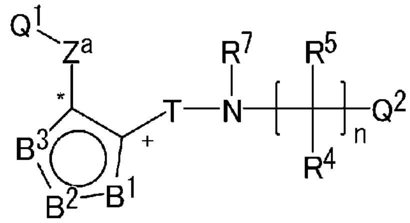

(Z-I)

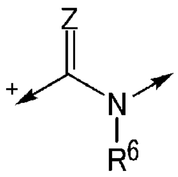

(T-1)

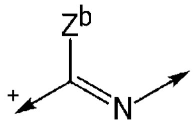

(T-2)

**Description**

TECHNICAL FIELD

The present invention relates to a five-membered heteroaryl compound and agricultural and horticultural fungicide. The present invention more specifically relates to a five-membered heteroaryl compound that exhibits excellent fungicidal activity against pathogens that affect agricultural and horticultural plants, has excellent safety, and can be synthesized in an industrially advantageous manner, as well as an agricultural and horticultural fungicide containing the same as an active ingredient.

Priority is claimed on Japanese Patent Application No. 2023-131664, filed August 10, 2023, Japanese Patent Application No. 2024-007491, filed January 22, 2024, and Japanese Patent Application No. 2024-068592, filed April 19, 2024, the content of which is incorporated herein by reference.

BACKGROUND ART

Various compounds that exhibit fungicidal activity against pathogens that affect agricultural and horticultural plants have been proposed. Not only sufficiently high efficacy, but also difficulty in causing drug resistance, no chemical damage to plants or soil pollution, and low toxicity to livestock and fish are required, so as to make such compounds usable practically as agricultural and horticultural fungicides.

Incidentally, Patent Document 1 discloses compounds of formula (A) and the like.

[Chemical Formula 1]

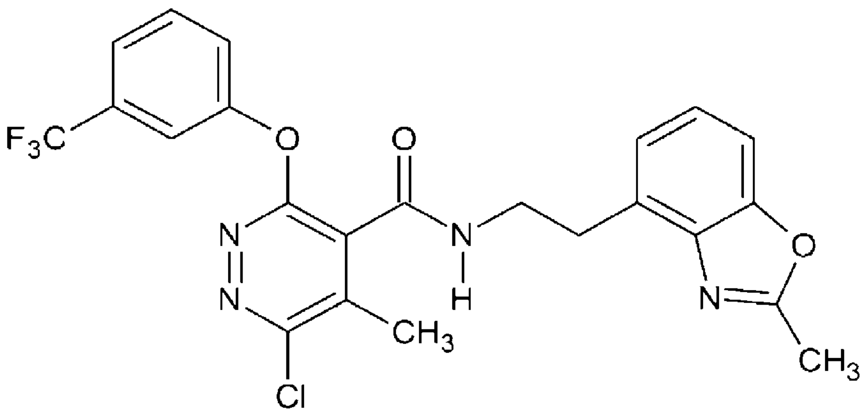

(A)

Citation List

Patent Document

Patent Document 1: Japanese Translation of PCT International Application Publication No. 2022-508277

SUMMARY OF INVENTION

Technical Problem

An object of the present invention is to provide a five-membered heteroaryl compound that exhibits excellent fungicidal activity against pathogens that affect agricultural and horticultural plants, has excellent safety, and can be synthesized in an industrially advantageous manner and an agricultural and horticultural fungicide containing the same as an active ingredient.

Solution to Problem

As a result of intensive research to solve the above-mentioned problems, the present invention encompassing the following aspects has been completed.

(1) A compound of formula (Z-1) or a salt thereof.

[Chemical Formula 2]

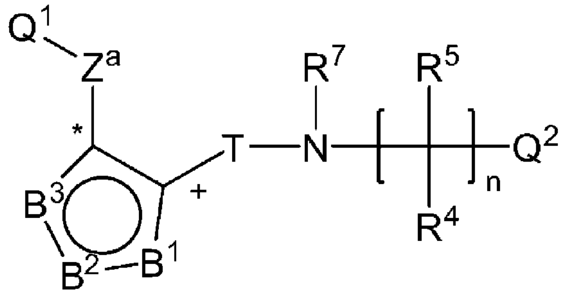

(Z − I)

(In the formula (Z-1),

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,

$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group, and

in a partial structure of the following formula (III):

[Chemical Formula 3]

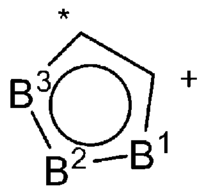

(III)

$B^1$ is an oxygen atom, a nitrogen atom, a group of formula: $NR^1$, or a group of formula: $CR^2$,

$B^2$ is an oxygen atom, a nitrogen atom, a group of formula: $NR^1$, or a group of formula: $CR^2$,

$B^3$ is an oxygen atom, a nitrogen atom, a group of formula: $NR^1$, or a group of formula: $CR^2$,

one of $B^1$, $B^2$ and $B^3$ is an oxygen atom, or a group of formula: $NR^1$, and remaining two thereof are a nitrogen atom or a group of formula: $CR^2$,

$R^1$ is each independently a substituted or unsubstituted C1-6 alkyl group, a substituted or unsubstituted C3-6 cycloalkyl group, or a substituted or unsubstituted five-membered or six-membered heterocyclyl group,

$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,

carbon atoms marked with * and + in the partial structure correspond to carbon atoms marked with * and + in the formula (Z-1),

$Z^a$ is an oxygen atom or a sulfur atom,

T is a partial structure (T-1) or a partial structure (T-2),

[Chemical Formula 4]

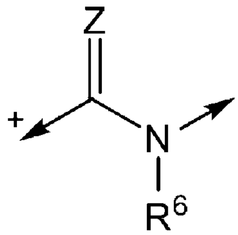 (T−1) 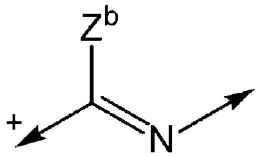 (T−2)

in the partial structure (T-1) or the partial structure (T-2), an arrow marked with + is bonded to a carbon atom marked with + in the partial structure of the formula (III), and the other arrow is bonded to a nitrogen atom of NR7 in the formula (Z-1),
in the partial structure (T-1), Z is an oxygen atom or a sulfur atom,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
in the partial structure (T-2), $Z^b$ is a C1-6 alkoxy group or a C1-6 alkylthio group,
$R^7$ is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a cyano group,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3.)

(2) The compound or the salt thereof according to (1) mentioned above, wherein the formula (Z-1) is formula (I).

[Chemical Formula 5]

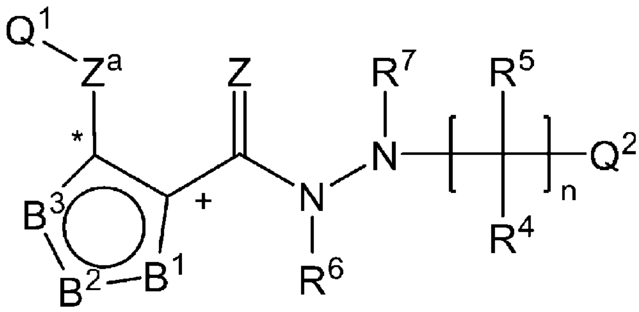

(I)

(In the formula (I),

$Q^1$, $Q^2$, $B^1$, $B^2$, $B^3$, $Z^a$, $R^4$, $R^5$, $R^7$ and n are identical to $Q^1$, $Q^2$, $B^1$, $B^2$, $B^3$, $Z^a$, $R^4$, $R^5$, $R^7$ and n in the formula (Z-1), and
Z and $R^6$ are identical to Z and $R^6$ in the partial structure (T-1).)

(3) The compound or the salt thereof according to (2) mentioned above, wherein

$Z^a$ is an oxygen atom,
Z is an oxygen atom,
$R^6$ is a hydrogen atom,
$R^7$ is a formyl group, a group of formula: $R^c$-CO-, a group of formula: $R^c$-CS-, a group of formula: $R^c$-CO-CO-, or a group of formula: $R^c$-$SO_2$-, and
n is 1.

(4) An agricultural and horticultural fungicide comprising at least one selected from the group consisting of the compound and the salt thereof of any one of (1) to (3) mentioned above, as an active ingredient.
(5) A method for producing a compound of formula (I-1), the method including a step in which a compound of formula (im-1) and a compound of formula (im-2) are reacted in the presence of an acid scavenger.

[Chemical Formula 6]

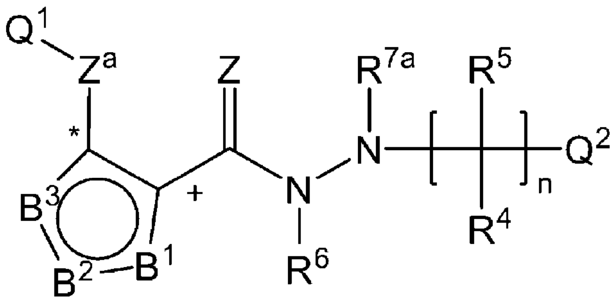

(I-1)

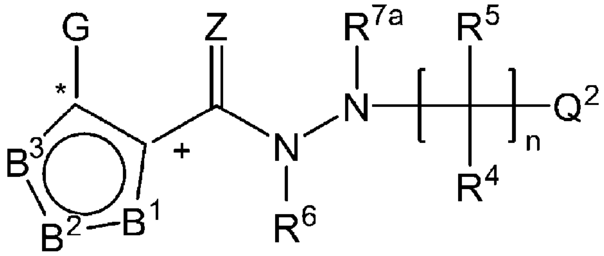

(im-1)

$Q^1-Z^a-H$ (im-2)

(In the formulae,

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in the following partial structure:

[Chemical Formula 7]

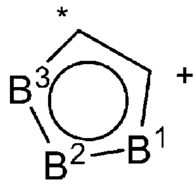

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,
$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-1) or the formula (im-1),
$Z^a$ is an oxygen atom or a sulfur atom,
Z is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted

naphthyl group, or a substituted or unsubstituted five-membered or six-membered heterocyclyl group,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group, and
G is a halogeno group.)

(6) A method for producing a compound of formula (I-2), the method including a step in which a compound of formula (im-3) and a compound of formula (im-4) are reacted in the presence of an organic base and a copper catalyst with oxygen as an oxidizing agent.

[Chemical Formula 8]

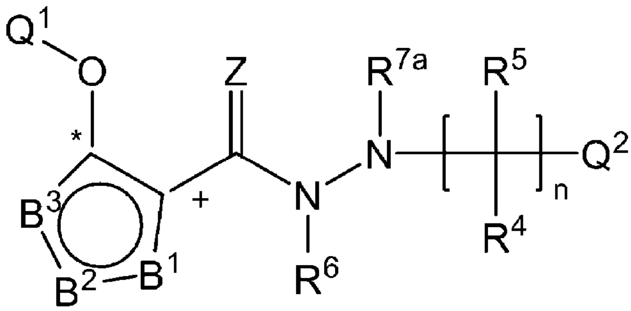

(I－2)

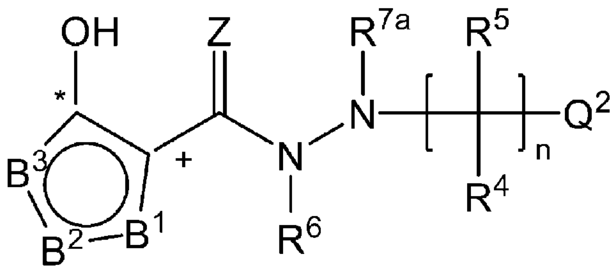

(im－3)

$Q^1—B(OH)_2$ (im－4)

(In the formulae,
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in the following partial structure:

[Chemical Formula 9]

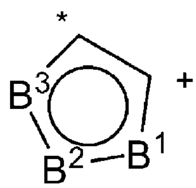

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,
$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-2) or the formula (im-3),
Z is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
$R^{7a}$ is a formyl group, a group of formula: $R^c$-CO-, a group of formula: $R^c$-CS-, a group of formula: $R^c$-CO-CO-, or a group of formula: $R^c$-$SO_2$-, and
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group.)

(7) A method for producing a compound of formula (I-3), the method including a compound of formula (im-5) and a compound of formula (im-6) are reacted in the presence of a condensing agent when $U^1$ in the formula (im-5) is a hydroxy group or in the presence of an acid scavenger when $U^1$ is a halogeno group.

[Chemical Formula 10]

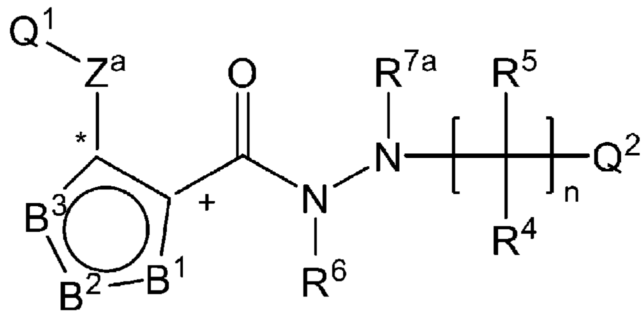

(I-3)

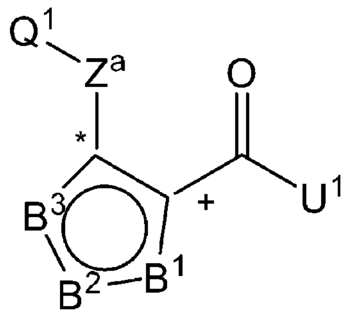

(im 5)

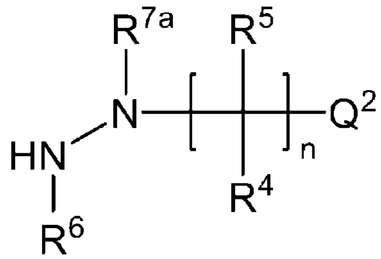

(im-6)

(In the formulae,

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in the following partial structure:

[Chemical Formula 11]

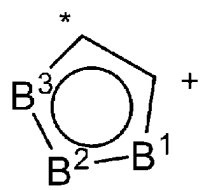

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,

$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-3) or the formula (im-5),
$Z^a$ is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group, and
$U^1$ is a hydroxy group or a halogeno group.)

(8) A compound of formula (im-5A) or a salt thereof.

[Chemical Formula 12]

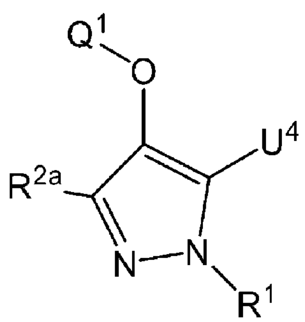

(im-5A)

(In the formula (im-5A),

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$R^1$ is substituted or unsubstituted C1-6 alkyl group, a substituted or unsubstituted C3-6 cycloalkyl group, or a substituted or unsubstituted five-membered or six-membered heterocyclyl group,
$R^{2a}$ is a C1-6 haloalkyl group, a cyano group, or a halogeno group, and
$U^4$ is a carboxyl group, a C1-6 alkoxycarbonyl group, or a halogenocarbonyl group.)

(9) A compound of formula (im-5B) or a salt thereof.

[Chemical Formula 13]

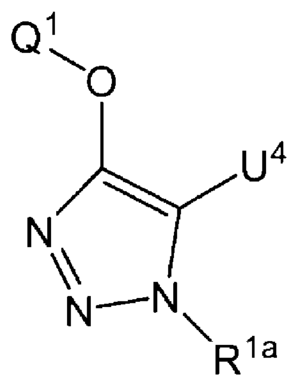

(im-5B)

(In the formula (im-5B),

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$R^{1a}$ is a substituted or unsubstituted C2-6 alkyl group, or a substituted or unsubstituted C3-6 cycloalkyl group, and
$U^4$ is a carboxyl group, a C1-6 alkoxycarbonyl group, or a halogenocarbonyl group.)

(10) A compound of formula (im-25) or a salt thereof.

[Chemical Formula 14]

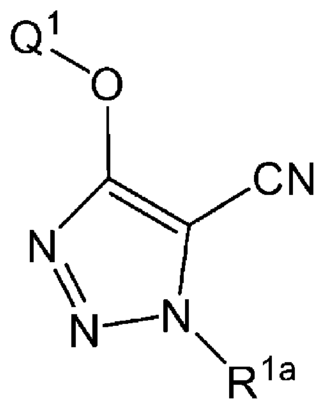

(im-25)

(In the formula (im-25),

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group, and

$R^{1a}$ is a substituted or unsubstituted C2-6 alkyl group, or a substituted or unsubstituted C3-6 cycloalkyl group.)

Advantageous Effects of Invention

The five-membered heteroaryl compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively prevent diseases in agricultural and horticultural plants.

DESCRIPTION OF EMBODIMENTS

The five-membered heteroaryl compound according to the present invention is a compound of formula (Z-1) (hereinafter, may be indicated as compound (Z-1)) or a salt of the compound (Z-1).

[Chemical Formula 15]

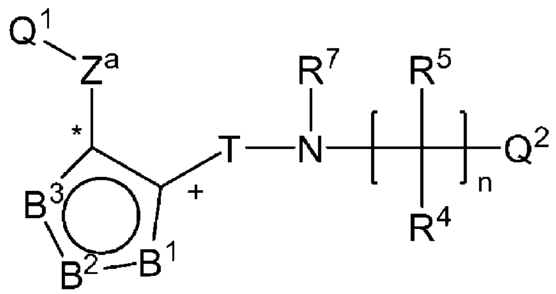

(Z − I)

In the present invention, the term "unsubstituted" refers to a group consisting of a mother nucleus. In the case where only the name of a group serving as a mother nucleus is provided without accompanying the term "substituted", this refers to "unsubstituted" unless specifically indicated otherwise.

On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.

The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.

There are no particular limitations on "substituents" provided that they are chemically available and achieve the effects of the present invention.

Specific examples of groups that can be "substituents" include the following groups:

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;

C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;

C2-6 alkynyl groups such as an ethynyl group, a 1-propynyl group, a 2-propynyl group, a 1-butynyl group, a 2-butynyl group, a 3-butynyl group, and a 1-methyl-2-propynyl group;

C3-6 cycloalkyl groups such as a cyclopropyl group, a cyclobutyl group, a cyclopentyl group, and a cyclohexyl group;

aryl groups such as a phenyl group, a tolyl group, a xylyl group, a trimethylphenyl group, and a naphthyl group;

phenyl C1-6 alkyl groups such as a benzyl group and a phenethyl group;

three-membered to six-membered heterocyclyl groups;

three-membered to six-membered heterocyclyl C1-6 alkyl groups;

a hydroxy group;

C1-6 alkoxy groups such as a methoxy group, an ethoxy group, a n-propoxy group, an i-propoxy group, a n-butoxy

group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
a phenoxy group, a naphthoxy group;
phenyl C1-6 alkoxy groups such as a benzyloxy group, and a phenethyloxy group;
five-membered or six-membered heteroaryloxy groups such as a thiazolyloxy group, and a pyridyloxy group;
five-membered or six-membered heteroaryl C1-6 alkyloxy groups such as a thiazolylmethyloxy group, and a pyridylmethyloxy group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
a benzoyl group;
C1-6 alkoxycarbonyl groups such as a methoxycarbonyl group, an ethoxycarbonyl group, a n-propoxycarbonyl group, an i-propoxycarbonyl group, a n-butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
an anilino group, a naphthylamino group;
phenyl C1-6 alkylamino groups such as a benzylamino group, and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as an aminocarbonyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
a phenylthio group, a naphthylthio group;
five-membered or six-membered heteroarylthio groups such as a thiazolylthio group, and a pyridylthio group;
C1-6 alkylsulfinyl groups such as a methylsulfinyl group, an ethylsulfinyl group, and a t-butylsulfinyl group;
C1-6 haloalkylsulfinyl groups such as a trifluoromethylsulfinyl group, and a 2,2,2-trifluoroethylsulfinyl group;
a phenylsulfinyl group;
five-membered or six-membered heteroarylsulfinyl groups such as a thiazolylsulfinyl group, and a pyridylsulfinyl group;

C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
a phenylsulfonyl group;
five-membered or six-membered heteroarylsulfonyl groups such as a thiazolylsulfonyl group, and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
a triphenylsilyl group;
a cyano group; and a nitro group.

Any hydrogen atom of the "substituent" may be substituted with a group having a different structure. Examples of such a "substituent" include C1-6 alkyl groups, C1-6 haloalkyl groups, C1-6 alkoxy groups, C1-6 haloalkoxy groups, halogeno groups, a cyano group, and a nitro group.

The "three-membered to six-membered heterocyclyl group" contains, as a ring member atom, one to four hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "three-membered to six-membered heterocyclyl group" include three-membered to six-membered saturated heterocyclyl groups, five-membered or six-membered heteroaryl groups, and five-membered or six-membered partially-unsaturated heterocyclyl groups.

Examples of the "three-membered to six-membered saturated heterocyclyl groups" include an aziridinyl group, an epoxy group, an azetidinyl group, an oxetanyl group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the "five-membered heteroaryl groups" include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the "six-membered heteroaryl groups" include a pyridyl group, a pyrazinyl group, a pyrimidinyl group, a pyridazinyl group, and a triazinyl group.

Examples of the "five-membered or six-membered partially-unsaturated heterocyclyl groups" include: five-membered partially-unsaturated heterocyclyl groups such as a pyrrolinyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

$Q^1$ in the formula (Z-1) is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group.

$Q^2$ in the formula (Z-1) is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group.

The heterocyclyl group contains, as a ring member atom, at least one hetero atom selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings.

Examples of the "five-membered or six-membered heterocyclyl group" as $Q^1$ or $Q^2$ include five-membered or six-membered saturated heterocyclyl groups, five-membered or six-membered heteroaryl groups, and five-membered or six-membered partially-unsaturated heterocyclyl groups.

Examples of the "five-membered or six-membered saturated heterocyclyl group" as $Q^1$ or $Q^2$ include a pyrrolidinyl group, a tetrahydrofuranyl group, a tetrahydropyranyl group, a thiazolidinyl group, a piperidyl group, a piperazinyl group, a morpholinyl group, a dioxolanyl group, and a dioxanyl group.

Examples of the "five-membered heteroaryl group" as $Q^1$ or $Q^2$ include a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, and a tetrazolyl group.

Examples of the "six-membered heteroaryl group" as $Q^1$ or $Q^2$ include a pyridyl group, a pyrazinyl group, a pyrimidinyl group, a pyridazinyl group, and a triazinyl group.

Examples of the "five-membered or six-membered partially-unsaturated heterocyclyl group" as $Q^1$ or $Q^2$ include:

five-membered partially-unsaturated heterocyclyl groups such as a pyrrolynyl group, a dihydrofuranyl group, a dihydrothiophenyl group, an imidazolynyl group, a pyrazolynyl group, an oxazolynyl group, an isoxazolynyl group, a thiazolynyl group, and an isothiazolynyl group; and six-membered partially-unsaturated heterocyclyl groups such as a dihydropyranyl group.

Examples of the "nine-membered heterocyclyl group" as $Q^1$ or $Q^2$ include an indolyl group, an isoindolyl group, a benzofuranyl group, a benzothienyl group, an indazolyl group, a benzoimidazolyl group, a benzoxazolyl group, a benzoisoxazolyl group, a benzothiazolyl group, a benzoisothiazolyl group, and a benzodioxolanyl group.

Examples of the "ten-membered heterocyclyl group" as $Q^1$ or $Q^2$ include a quinolynyl group, an isoquinolynyl group, a cinnolinyl group, a phthalazinyl group, a quinazolinyl group, and a quinoxalinyl group,

As a substituent on the "five-membered or six-membered heterocyclyl group", "phenyl group", "naphthyl group" or "nine-membered or ten-membered heterocyclyl group" as $Q^1$ or $Q^2$, a halogeno group, a C1-6 alkyl group, a C1-6 haloalkyl group, a hydroxy group, a C1-6 alkoxy group, a C1-6 haloalkoxy group, a C1-6 alkoxy group substituted with a C1-6 alkoxy group, a C1-6 alkylcarbonyl group, a C1-6 alkylthio group, a C1-6 alkylsulfonyl group, a C3-6 cycloalkyl group, a phenyl group, a phenyloxy group, a five-membered heteroaryl group, a six-membered heteroaryl group, a cyano group, a nitro group, a tri C1-6 alkylsilyl group; an amino group substituted with a C1-6 alkyl or C1-6 alkylcarbonyl group; a carboxamide group substituted with a C1-6 alkyl; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups and C1-6 haloalkoxy groups is preferable.

Two substituents on the "five-membered or six-membered heterocyclyl group", "phenyl group", "naphthyl group" or "nine-membered or ten-membered heterocyclyl group" of $Q^1$ or $Q^2$ may form together a divalent organic group, or two substituents on a phenyl group substituted with two substituents, a five-membered heteroaryl group substituted with two substituents, or a six-membered heteroaryl group substituted with two substituents may form together a divalent organic group. Examples of the divalent organic group include C3-6 alkylene groups such as a trimethylene group and a tetramethylene group; and C1-3 alkylene dioxy groups such as a methylenedioxy group, and a dimethylenedioxy group.

For example, it is preferable that two substituents on a phenyl group form together a trimethylene group to form an indanyl group as $Q^1$ or $Q^2$.

Among these, $Q^1$ in formula (Z-1) is preferably a substituted or unsubstituted phenyl group, a substituted or unsubstituted naphthyl group, a substituted or unsubstituted five-membered or six-membered heterocyclyl group, or a substituted or unsubstituted nine-membered heterocyclyl group, more preferably a substituted or unsubstituted phenyl group, a substituted or unsubstituted naphthyl group, a substituted or unsubstituted thiazolyl group, a substituted or unsubstituted pyridyl group or a substituted or unsubstituted benzodioxolanyl group, even more preferably a substituted or unsubstituted phenyl group or a substituted or unsubstituted thiazolyl group, and particularly preferably a substituted or unsubstituted phenyl group.

As a substituent on the "five-membered or six-membered heterocyclyl group", "phenyl group", "naphthyl group" or "nine-membered or ten-membered heterocyclyl group" as $Q^1$, a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C1-6 alkylsulfonyl group, a C3-6 cycloalkyl group, a phenyl group, a phenyloxy group, a cyano group, a nitro group, a tri C1-6 alkylsilyl group; an amino group substituted with a C1-6 alkyl or C1-6 alkylcarbonyl group; and/or a carboxamide group substituted with a C1-6 alkyl is more preferable, a halogeno group, a C1-6 alkyl group, a C1-6 haloalkyl group, a C1-6 alkoxy group, a C1-6 haloalkoxy group, a C1-6 alkylcarbonyl group, a C1-6 alkylthio group, a C3-6 cycloalkyl group, a cyano group, a nitro group; and/or an amino group substituted with a C1-6 alkyl group is even more preferable, a halogeno group, a C1-6 alkyl group, a C1-6 haloalkyl group, a C1-6 alkoxy group, a C1-6 haloalkoxy group, a C1-6 alkylthio group, a C3-6 cycloalkyl group, a cyano group, and/or an amino group substituted with a C1-6 alkyl group is still more preferable, and a halogeno group, a C1-6 alkyl group, a C1-6 haloalkyl group, a C1-6 alkoxy group, a C1-6 haloalkoxy group, a C1-6 alkylthio group, a C3-6 cycloalkyl group, a cyano group, and/or an amino group substituted with a C1-6 alkyl group is particularly preferable.

Among these, as a substituent on the "phenyl group", at least one selected from halogeno groups, C1-6 alkyl groups, C1-6 haloalkyl groups, C1-6 alkoxy groups, C1-6 haloalkoxy groups, C1-6 alkylthio groups, C3-6 cycloalkyl groups, a cyano group and amino groups substituted with C1-6 alkyl groups are preferable, and at least one selected from halogeno groups, C1-6 alkyl groups, C1-6 haloalkyl groups, C1-6 alkoxy groups, C1-6 haloalkoxy groups, C1-6 alkylthio groups, C3-6 cycloalkyl groups, a cyano group and amino groups substituted with C1-6 alkyl groups is more preferable.

As a substituent on the "five-membered or six-membered heterocyclyl group", a C1-6 haloalkyl group is more preferable. As a substituent on the "thiazolyl group", a C1-6 haloalkyl group is more preferable.

In a case where the "five-membered or six-membered heterocyclyl group", "phenyl group", "naphthyl group" or "nine-membered or ten-membered heterocyclyl group" as $Q^1$ has a substituent, the number of the substituent is preferably

1 or 2.

$Q^1$ may be an indanyl group in which two substituents on a phenyl group form together a trimethylene group.

$Q^2$ in the formula (Z-1) is more preferably a substituted or unsubstituted phenyl group, a substituted or unsubstituted naphthyl group, a substituted or unsubstituted thienyl group, a substituted or unsubstituted thiazolyl group, a substituted or unsubstituted pyridyl group, a substituted or unsubstituted benzodioxolanyl group, a substituted or unsubstituted benzothiazolyl group, or a substituted or unsubstituted quinolynyl group, and even more preferably a substituted or unsubstituted phenyl group.

As a substituent on the "phenyl group", "naphthyl group", "five-membered or six-membered heterocyclyl group", or "nine-membered or ten-membered heterocyclyl group" as $Q^2$, a halogeno group, a C1-6 alkyl group, a C1-6 haloalkyl group, a C1-6 alkoxy group, a C1-6 haloalkoxy group, a C1-6 alkoxy group substituted with a C1-6 alkoxy group, a C1-6 alkylthio group, a C1-6 alkylsulfonyl group, a cyano group, or an amino group substituted with a C1-6 alkyl group is more preferable, and a halogeno group, a C1-6 alkyl group, a C1-6 haloalkyl group, a C1-6 alkoxy group, a C1-6 haloalkoxy group, a C1-6 alkoxy group substituted with C1-6 alkoxy group, a C1-6 alkylthio group, a cyano group, or an amino group substituted with a C1-6 alkyl group is even more preferable.

In a case where the "phenyl group", "naphthyl group", "five-membered or six-membered heterocyclyl group" or "nine-membered or ten-membered heterocyclyl group" as $Q^2$ has a substituent, the number of the substituent is preferably 1 or 2, and more preferably 2.

It is also preferable that $Q^2$ be an indanyl group in which two substituents on a phenyl group form together a trimethylene group.

Each symbol in the partial structure of the following formula (III) means, as follows.

[Chemical Formula 16]

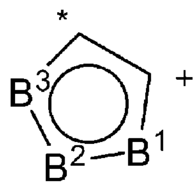

(III)

Carbon atoms marked with * and + in the partial structure correspond to carbon atoms marked with * and + in the formula (Z-1).

$B^1$ is an oxygen atom, a nitrogen atom, a group of formula: $NR^1$, or a group of formula: $CR^2$.

$B^2$ is an oxygen atom, a nitrogen atom, a group of formula: $NR^1$, or a group of formula: $CR^2$

$B^3$ is an oxygen atom, a nitrogen atom, a group of formula: $NR^1$, or a group of formula: $CR^2$.

One of $B^1$, $B^2$ and $B^3$ is an oxygen atom, or a group of formula: $NR^1$, and the remaining two thereof are a nitrogen atom or a group of formula: $CR^2$.

Specific examples of partial structure of formula (III) include the following structures.

[Chemical Formula 17]

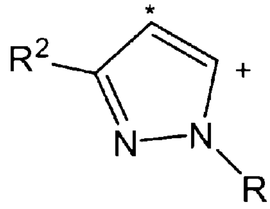

(III-1)

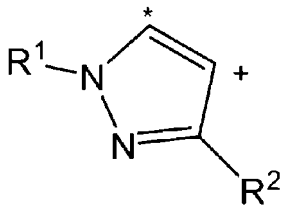

(III-2)

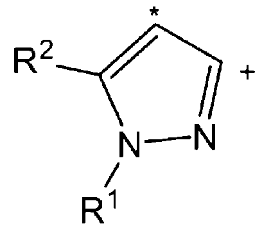

(III-3)

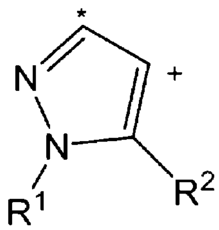

(III-4)

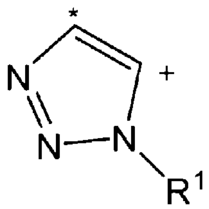

(III-5)

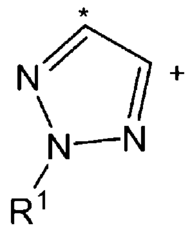

(III-6)

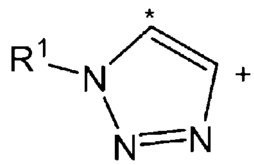

(III-7)

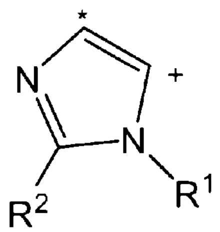

(III-8)

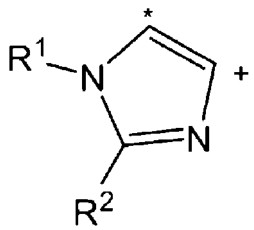

(III-9)

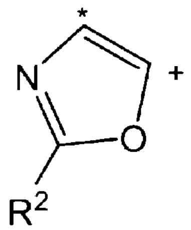

(III-10)

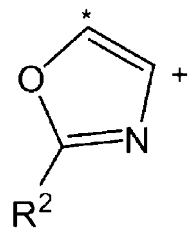

(III-11)

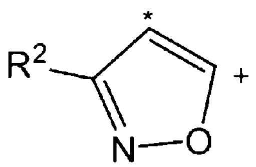

(III-12)

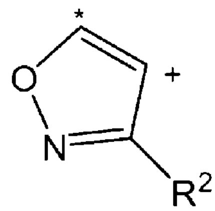

(III-13)

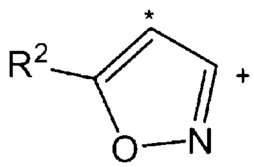

(III-14)

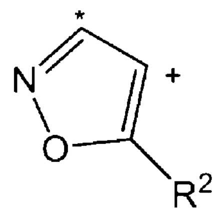

(III-15)

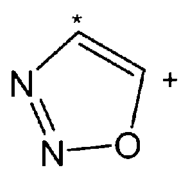

(III-16)

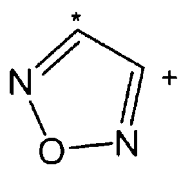

(III-17)

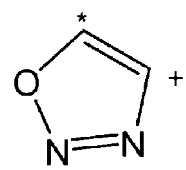

(III-18)

$R^1$ is each independently a substituted or unsubstituted C1-6 alkyl group, a substituted or unsubstituted C3-6 cycloalkyl group, or a substituted or unsubstituted five-membered or six-membered heterocyclyl group.

$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl

group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group.

The "C1-6 alkyl group" as $R^1$ and $R^2$ may be a linear chain or a branched chain. Examples of the "C1-6 alkyl group" as $R^1$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "C3-6 cycloalkyl group" as $R^1$ and $R^2$ include a cyclopropyl group, a cyclobutyl group, a cyclopentyl group, and a cyclohexyl group.

Examples of the "C2-6 alkenyl group" as $R^2$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" as $R^2$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkoxy group" as $R^2$ include a methoxy group, an ethoxy group, a n-propoxy group, an i-propoxy group, a n-butoxy group, a s-butoxy group, an i-butoxy group, and a t-butoxy group.

Examples of the "C1-6 alkylcarbonyl group" as $R^2$ include an acetyl group, a propionyl group, a n-propylcarbonyl group, an i-propylcarbonyl group, a n-butylcarbonyl group, and a t-butylcarbonyl group.

Examples of the "C1-6 alkoxycarbonyl group" as $R^2$ include a methoxycarbonyl group, an ethoxycarbonyl group, a n-propoxycarbonyl group, an i-propoxycarbonyl group, a n-butoxycarbonyl group, and a t-butoxycarbonyl group.

Examples of the "C1-6 alkylcarbonyloxy group" as $R^2$ include an acetyloxy group, a propionyloxy group, a n-propylcarbonyloxy group, an i-propylcarbonyloxy group, a n-butylcarbonyloxy group, and a t-butylcarbonyloxy group.

Examples of the "C1-6 alkoxycarbonyloxy group" as $R^2$ include a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group.

Examples of the "C1-6 alkylthio group" as $R^2$ include a methylthio group, an ethylthio group, a n-propylthio group, a n-butylthio group, a n-pentylthio group, a n-hexylthio group, an i-propylthio group, and an i-butylthio group.

Examples of the "C1-6 alkylsulfinyl group" as $R^2$ include a methylsulfinyl group, an ethylsulfinyl group, and a t-butylsulfinyl group.

Examples of the "C1-6 alkylsulfonyl group" as $R^2$ include a methylsulfonyl group, an ethylsulfonyl group, and a t-butylsulfonyl group.

As a substituent on the "C1-6 alkyl group", "C2-6 alkenyl group", "C2-6 alkynyl group", "C1-6 alkoxy group", "C1-6 alkylcarbonyl group", "C1-6 alkoxycarbonyl group", "C1-6 alkylcarbonyloxy group", "C1-6 alkoxycarbonyloxy group", "C1-6 alkylthio group", "C1-6 alkylsulfinyl group", or "C1-6 alkylsulfonyl group" as $R^1$ or $R^2$, a halogeno group such as a fluoro group, a chloro group, a bromo group, or an iodo group, a hydroxy group, a C1-6 alkoxy group such as a methoxy group, an ethoxy group, a n-propoxy group, an i-propoxy group, a n-butoxy group, a s-butoxy group, an i-butoxy group, or a t-butoxy group, a C1-6 haloalkoxy group such as a 2-chloro-n-propoxy group, a 2,3-dichlorobutoxy group, or a trifluoromethoxy group, a C3-6 cycloalkyl group, a phenyl group, a five-membered heteroaryl group, a six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups, and C1-6 haloalkoxy groups is preferable, and at least one selected from halogeno groups, C1-6 alkoxy groups and C3-6 cycloalkyl groups is more preferable.

The "five-membered or six-membered heterocyclyl group" as $R^1$ and $R^2$ is a group that contains, as a ring member atom, one, two, three or four hetero atoms selected from the group consisting of a nitrogen atom, an oxygen atom and a sulfur atom. When at least two hetero atoms are contained, the hetero atoms may be identical to or different from each other.

Examples of the "five-membered or six-membered heterocyclyl group" include the same five-membered or six-membered heterocyclyl group as $Q^1$ or $Q^2$, and specifically include a pyrrolidinyl group, a tetrahydrofuranyl group, a thiazolidinyl group, a tetrahydro-2H-pyranyl group, a piperidyl group, a piperazinyl group, a morpholinyl group, a dioxolanyl group, a dioxanyl group; a pyrrolynyl group, a dihydrofuranyl group, an imidazolynyl group, a pyrazolynyl group, an oxazolynyl group, an isoxazolynyl group; a dihydropyranyl group; a pyrrolyl group, a furyl group, a thienyl group, an imidazolyl group, a pyrazolyl group, an oxazolyl group, an isooxazolyl group, a thiazolyl group, an isothiazolyl group, a triazolyl group, an oxadiazolyl group, a thiadiazolyl group, a tetrazolyl group; a pyridyl group, a pyrazinyl group, a pyrimidinyl group, a pyridazinyl group, and triazinyl group. The "five-membered or six-membered heterocyclyl group" is preferably a pyrazolynyl group, a thiazolyl group, or a pyridyl group, and more preferably a thiazolyl group or a pyridyl group.

Examples of the "five-membered or six-membered heterocyclyloxy group" as $R^2$ include a pyrrolidinyloxy group, a tetrahydrofuranyloxy group, a thiazolidinyloxy group, a tetrahydro-2H-pyranyloxy group, a piperidyloxy group, a piperazinyloxy group, a morpholinyloxy group, a dioxolanyloxy group, a dioxanyloxy group; a pyrrolynyloxy group, a dihydrofuranyloxy group, an imidazolynyloxy group, a pyrazolynyloxy group, an oxazolynyloxy group, an isooxazolynyloxy group; and a dihydropyranyloxy group.

As a substituent on the "C3-6 cycloalkyl group", "phenyl group", "naphthyl group", "five-membered or six-membered heterocyclyl group", "phenyloxy group", "naphthyloxy group", or "five-membered or six-membered heterocyclyloxy group" as $R^1$ and $R^2$, a halogeno group, a C1-6 alkyl group, a C1-6 haloalkyl group, a hydroxy group, a C1-6 alkoxy group, a C1-6 haloalkoxy group, a C3-6 cycloalkyl group, a phenyl group, a five-membered heteroaryl group, a six-membered heteroaryl group, a cyano group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl group substituted with at least one substituent selected from C1-6 alkyl groups, C1-6 alkoxy groups, halogeno groups, C1-6 haloalkyl groups, and C1-6 haloalkoxy groups; or a six-membered heteroaryl group substituted with at least one substituent selected from C1-6 alkyl groups, C1-6 alkoxy groups, halogeno groups, C1-6 haloalkyl groups, and C1-6 haloalkoxy groups is preferable, and a halogeno group is more preferable.

The "amino group" as $R^2$ is a group of $NH_2$. The "substituted amino group" is a group formed by substituting a hydrogen atom in a group of $NH_2$ with another group.

The "aminocarbonyl group" as $R^2$ is a group of $NH_2CO$. The "substituted aminocarbonyl group" is a group formed by substituting a hydrogen atom in a group of $NH_2CO$ with another group.

As a substituent on the "amino group", or "aminocarbonyl group" as $R^2$, a C1-6 alkyl group, a C1-6 haloalkyl group, a C1-6 alkoxycarbonyl group, a C3-6 cycloalkyl group, a phenyl group, a five-membered heteroaryl group, a six-membered heteroaryl group; a phenyl group substituted with at least one substituent selected from C1-6 alkyl groups, C1-6 alkoxy groups, halogeno groups, C1-6 haloalkyl groups, and C1-6 haloalkoxy groups; a five-membered heteroaryl groups substituted with at least one substituent selected from C1-6 alkyl groups, C1-6 alkoxy groups, halogeno groups, C1-6 haloalkyl groups, and C1-6 haloalkoxy groups; or a six-membered heteroaryl groups substituted with at least one substituent selected from C1-6 alkyl groups, C1-6 alkoxy groups, halogeno groups, C1-6 haloalkyl groups, and C1-6 haloalkoxy groups is preferable, a C1-6 alkyl group or a C1-6 alkoxycarbonyl group is more preferable, and a C1-6 alkyl group is even more preferable.

Examples of the "halogeno group" as $R^2$ include a fluoro group, a chloro group, a bromo group, and an iodo group.

Among these, $R^1$ is each independently and preferably a C1-6 alkyl group; a C1-6 alkyl group substituted with a halogeno group, C1-6 alkoxy group or C3-6 cycloalkyl group; a C3-6 cycloalkyl group; or a five-membered or six-membered heterocyclyl group, more preferably a C1-6 alkyl group; a C1-6 alkyl group substituted with a halogeno group, C1-6 alkoxy group or C3-6 cycloalkyl group; a C3-6 cycloalkyl group; a thiazolyl group or a pyridyl group, and even more preferably a C1-6 alkyl group; a C1-6 alkyl group substituted with a halogeno group, C1-6 alkoxy group or C3-6 cycloalkyl group; or a C3-6 cycloalkyl group.

$R^2$ is each independently and preferably a hydrogen atom, a substituted or unsubstituted C1-6 alkyl group, a substituted or unsubstituted C3-6 cycloalkyl group, a cyano group, or a halogeno group, and more preferably a hydrogen atom; a C1-6 alkyl group; a C3-6 cycloalkyl group; a cyano group; or a halogeno group.

The partial structure of formula (III) is preferably a partial structure of the formula (III-1), a partial structure of the formula (III-2), a partial structure of the formula (III-4), a partial structure of the formula (III-5), a partial structure of the formula (III-6), a partial structure of the formula (III-9), or a partial structure of the formula (III-15), more preferably a partial structure of the formula (III-1), a partial structure of the formula (III-2), a partial structure of the formula (III-5) or a partial structure of the formula (III-15), and even more preferably a partial structure of the formula (III-1) or a partial structure of the formula (III-5).

The partial structure of the formula (III-1) is preferably a partial structure of the following formula (III-1-a).

In the following formula (III-1-a), $R^{2a}$ is a hydrogen atom, a C1-6 alkyl group, a C3-6 cycloalkyl group, a cyano group or a halogeno group, and more preferably a hydrogen atom, a C1-6 alkyl group, a C3-6 cycloalkyl group or a halogeno group.

[Chemical Formula 18]

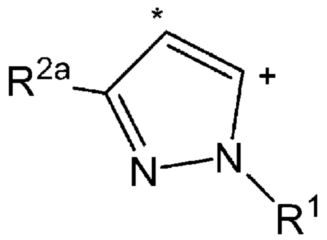

(III-1-a)

$Z^a$ in the formula (Z-1) is an oxygen atom or a sulfur atom.

$Z^a$ in the formula (Z-1) is preferably an oxygen atom.

T in the formula (Z-1) is a partial structure (T-1) or a partial structure (T-2).

[Chemical Formula 19]

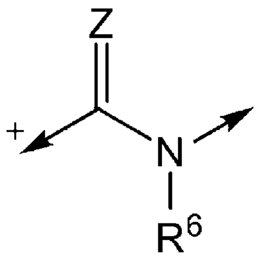

(T-1)

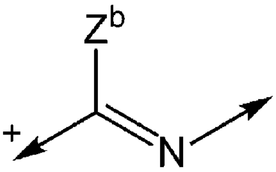

(T-2)

**In** the partial structure (T-1) or the partial structure (T-2), an arrow marked with + is bonded to a carbon atom marked with + in the partial structure of the formula (III), and the other arrow is bonded to a nitrogen atom of NR7 in the formula (Z-1).

Z in the partial structure (T-1) is an oxygen atom or a sulfur atom, and preferably an oxygen atom.

$R^6$ in the partial structure (T-1) is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group.

Examples of the "substituted or unsubstituted C1-6 alkyl group", "substituted or unsubstituted C2-6 alkenyl group", "substituted or unsubstituted C2-6 alkynyl group", "substituted or unsubstituted C1-6 alkylcarbonyl group", "substituted or unsubstituted C1-6 alkoxycarbonyl group", "substituted or unsubstituted C3-6 cycloalkyl group", "substituted or unsubstituted phenyl group", "substituted or unsubstituted naphthyl group", and "substituted or unsubstituted five-membered or six-membered heterocyclyl group" as $R^6$ include the same groups as those mentioned as $R^2$.

Among these, $R^6$ in the partial structure (T-1) is preferably a hydrogen atom or an unsubstituted C1-6 alkyl group, and more preferably a hydrogen atom.

In the partial structure (T-2), $Z^b$ is a C1-6 alkoxy group or a C1-6 alkylthio group.

Examples of the "C1-6 alkoxy group" and "C1-6 alkylthio group" as $Z^b$ include the same groups as those mentioned as $R^2$.

Among these, $Z^b$ in the partial structure (T-2) is preferably a methoxy group or a methylthio group.

Among these, T in the formula (Z-1) is preferably the partial structure (T-1).

$R^4$ in the formula (Z-1) is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group.

$R^5$ in the formula (Z-1) is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group.

Examples of the " substituted or unsubstituted C1-6 alkyl group", " substituted or unsubstituted C2-6 alkenyl group", " substituted or unsubstituted C2-6 alkynyl group", "substituted or unsubstituted C3-6 cycloalkyl group", "substituted or unsubstituted phenyl group", "substituted or unsubstituted naphthyl group", "substituted or unsubstituted five-membered or six-membered heterocyclyl group", and "halogeno group" as $R^4$ and $R^5$ include the same groups as those mentioned as examples of $R^2$.

$R^4$ and $R^5$ on an identical carbon atom may form together a divalent organic group. Examples of the divalent organic group include C2-5 alkylene groups such as a dimethylene group, a trimethylene group, and a tetramethylene group.

Among these, $R^4$ and $R^5$ in the formula (Z-1) are each preferably a hydrogen atom or a C1-6 alkyl group, and more preferably a hydrogen atom.

$R^7$ in the formula (Z-1) is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a cyano group.

Examples of the "substituted or unsubstituted C1-6 alkyl group", "substituted or unsubstituted C2-6 alkenyl group", "substituted or unsubstituted C2-6 alkynyl group", "substituted or unsubstituted C3-6 cycloalkyl group", "substituted or unsubstituted phenyl group", "substituted or unsubstituted naphthyl group", and "substituted or unsubstituted five-membered or six-membered heterocyclyl group as $R^7$ include the same groups as those mentioned as $R^2$.

$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group.

Examples of the "substituted or unsubstituted C1-6 alkyl group", "substituted or unsubstituted C1-6 alkoxy group", "substituted or unsubstituted amino group", "substituted or unsubstituted C3-6 cycloalkyl group", "substituted or unsubstituted C1-6 alkylthio group", "substituted or unsubstituted phenyl group", "substituted or unsubstituted naphthyl group", "substituted or unsubstituted five-membered or six-membered heterocyclyl group", and "substituted or unsubstituted five-membered or six-membered heterocyclyloxy group" as $R^c$ include the same groups as those mentioned as $R^2$.

Examples of the "C3-6 cycloalkoxy group" as $R^c$ include a cyclopropyloxy group, a cyclobutyloxy group, and a cyclopentyloxy group.

Examples of the "three-membered or four-membered heterocyclyl group" as $R^c$ include an aziridinyl group, an epoxy group, an azetidinyl group, and an oxetanyl group.

Examples of the "three-membered or four-membered heterocyclyloxy group" as $R^c$ include an aziridinyloxy group, an epoxyoxy group, a 3-azetidinyloxy group, and a 3-oxetanyloxy group.

Preferable examples of a substituent on the "C3-6 cycloalkoxy group", "three-membered or four-membered heterocyclyl group", or "three-membered or four-membered heterocyclyloxy group" as $R^c$ include halogeno groups, C1-6 alkyl groups, C1-6 haloalkyl groups, a hydroxy group, C1-6 alkoxy groups, C1-6 haloalkoxy groups, C3-6 cycloalkyl groups, a phenyl group, five-membered heteroaryl groups, six-membered heteroaryl groups, a cyano group; phenyl groups substituted with at least one substituent selected from C1-6 alkyl groups, C1-6 alkoxy groups, halogeno groups, C1-6 haloalkyl groups, and C1-6 haloalkoxy groups; five-membered heteroaryl groups substituted with at least one substituent selected from C1-6 alkyl groups, C1-6 alkoxy groups, halogeno groups, C1-6 haloalkyl groups, and C1-6 haloalkoxy groups; and six-membered heteroaryl groups substituted with at least one substituent selected from C1-6 alkyl groups, C1-6 alkoxy groups, halogeno groups, C1-6 haloalkyl groups and C1-6 haloalkoxy groups.

$R^7$ is preferably a hydrogen atom; a C1-6 alkyl group; a C1-6 haloalkyl group; a formyl group; a group of formula: $R^c$-CO-, a group of formula: $R^c$-CS-, a group of formula: $R^c$-CO-CO-, a group of formula: $R^c$-$SO_2$-; or a phenyl group substituted with a C1-6 alkyl group, more preferably a formyl group, a group of formula: $R^c$-CO-, a group of formula: $R^c$-CS-, a group of formula: $R^c$-CO-CO-, or a group of formula: $R^c$-$SO_2$-, and even more preferably a group of formula: $R^c$-CO-.

$R^c$ is each independently and preferably a C1-6 alkyl group; a C1-6 haloalkyl group; a C1-6 alkyl group substituted with a C1-6 alkoxy group; a C1-6 alkoxy group; a C1-6 haloalkoxy group; a C1-6 alkoxy group substituted with a C3-6 cycloalkyl group; a C1-6 alkoxy group substituted with a phenyl group; a C3-6 cycloalkoxy group; a C1-6 alkylthio group; an amino group substituted with a C1-6 alkyl group; a C3-6 cycloalkyl group; a phenyl group; a pyrazolyl group substituted with a C1-6 alkyl group; an oxetanyl group; or an oxetanyloxy group, and more preferably a C1-6 alkyl group; a C1-6 alkyl group substituted with a C1-6 alkoxy group; a C1-6 alkoxy group; a C1-6 alkoxy group substituted with a C3-6 cycloalkyl group; a C1-6 alkoxy group substituted with a phenyl group; a C3-6 cycloalkoxy group; or an oxetanyloxy group.

n indicates the repeating number of structural unit in square brackets and is an integer of 1 to 3.

n is preferably 1 or 2, and more preferably 1.

The five-membered heteroaryl compound according to the present invention is preferably a compound of formula (I) (hereinafter, may be referred to as compound (I)) or a salt thereof. Each symbol in formula (I) is identical to that in the formula (Z-1).

[Chemical Formula 20]

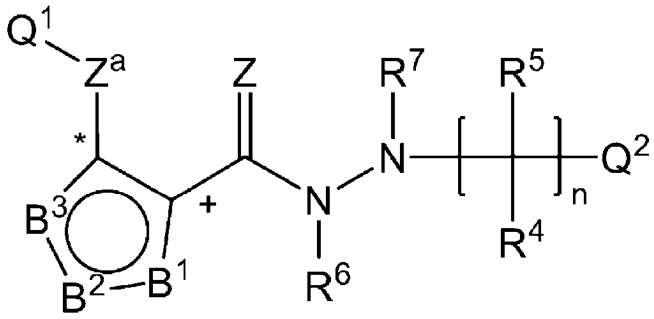

(I)

One of examples of the five-membered heteroaryl compound according to the present invention is a compound of the following formula (I-A) or a salt thereof.

Each symbol in the formula (I-A) is identical to that in the formula (Z-1), and $R^{7a}$ is a formyl group, a group of formula: $R^c$-CO-, a group of formula: $R^c$-CS-, a group of formula: $R^c$-CO-CO-, or a group of formula: $R^c$-$SO_2$-.

$R^c$ is identical to that defined in $R^7$.

$R^{7a}$ is preferably a group of formula: $R^c$-CO-.

[Chemical Formula 21]

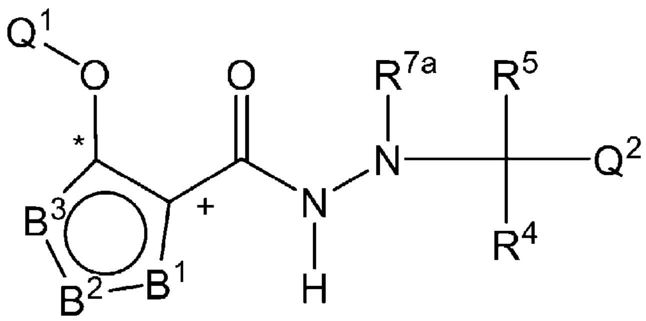

(I-A)

The salt of the compound (Z-I) is not particularly limited as long as the salt is an agriculturally and horticulturally acceptable salt. Examples of the salt of the compound (Z-I) include salts of inorganic acids such as hydrochloric acid and sulfuric acid; salts of organic acids such as acetic acid and lactic acid; salts of alkali metals such as lithium, sodium, and potassium; salts of alkaline earth metals such as calcium and magnesium; salts of transition metals such as iron and copper; ammonia; and salts of organic bases such as triethylamine, tributylamine, pyridine, and hydrazine.

The method for producing the compound (Z-I) or the salt of the compound (Z-I) is not particularly limited. For example, the compound (Z-I) or the salt of the compound (Z-I) can be obtained by a conventionally-known production method as described in Examples or the like. In addition, the salt of the compound (Z-I) can be obtained from the compound (Z-I) by a conventionally-known method.

Method for producing the compound (I)

Production Method 1

A compound of formula (I-1) can be produced by a method including a reaction step of a compound of formula (im-1) and a compound of formula (im-2), as shown in Scheme 1.

[Chemical Formula 22]

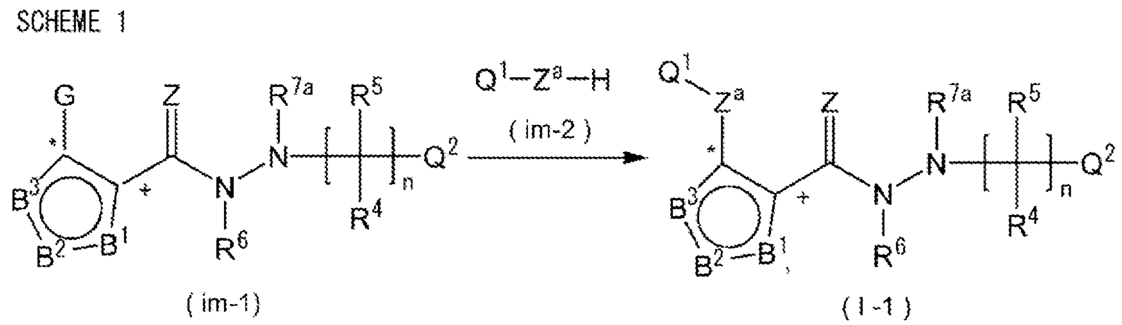


Among the compounds (I), the compound of formula (I-1) is a compound in which $R^7$ is "a formyl group, a group of formula: $R^c$-CO-, a group of formula: $R^c$-CS-, a group of formula: $R^c$-CO-CO-, or a group of formula: $R^c$-$SO_2$-" (hereinafter, may be indicated as $R^{7a}$).

$R^{7a}$ is preferably a group of formula: $R^c$-CO-.

The compound of formula (im-1) is a compound in which G is a halogeno group, and the other symbols are identical to those defined in the compound of formula (1-1).

The compound of formula (im-2) is a compound in which symbols are identical to those defined in the compound of formula (I-1).

The compound of formula (I-1) can be produced by reacting the compound of formula (im-1) with the compound of formula (im-2) by a conventionally-known method. The reaction may be carried out in the presence of an acid scavenger.

Examples of the acid scavenger include common inorganic bases and organic bases. Alkali metal carbonates, alkaline earth metal acetates, tertiary amines or aromatic amines are preferably used.

Although the reaction temperature is appropriately determined, the reaction temperature is approximately 60°C to 120°C, for example. Although the reaction time is appropriately determined, the reaction time is approximately 1 hour to 24 hours, for example.

The compound of formula (im-2) may be a commercially available product, or may be produced by a conventionally-known method.

The compound of formula (im-1) may be produced by at least one method described in the present specification (see Production Method 4).

Production Method 2

A compound of formula (I-2) can be produced by a method including a reaction step of a compound of formula (im-3) and a compound of formula (im-4), as shown in Scheme 2.

[Chemical Formula 23]

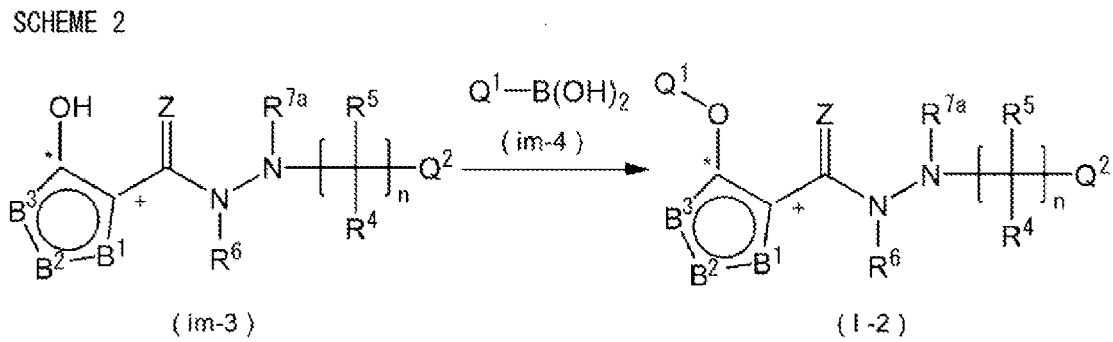


Among the compounds (I), the compound of formula (1-2) is a compound in which $Z^a$ is oxygen, and $R^7$ is "a formyl group, a group of formula: $R^c$-CO-, a group of formula: $R^c$-CS-, a group of formula: $R^c$-CO-CO-, or a group of formula: $R^c$-$SO_2$-" (hereinafter, may be indicated as $R^{7a}$).

$R^{7a}$ is preferably a group of formula: $R^c$-CO-.

The compound of formula (im-3) is a compound in which symbols are identical to those defined in the compound of formula (1-2).

The compound of formula (im-4) is a compound in which $Q^1$ is identical to that defined in the compound of formula (I-2).

The compound of formula (I-2) can be produced by reacting the compound of formula (im-3) and the compound of formula (im-4) in the presence of an organic base and a copper catalyst with oxygen as an oxidizing agent. This reaction is known as Chan-Lam-Evans coupling.

Although the copper catalyst is not particularly limited, a divalent copper catalyst such as copper(II) chloride, copper(II) bromide, copper(II) oxide, copper(II) acetate, copper(II) sulfate, copper(II) bis(2,2,6,6-tetramethyl-3,5-heptanedionate), or copper(II) trifluoromethanesulfonate is used, and copper(II) acetate is preferably used.

Although the organic base is not particularly limited, a tertiary amine such as triethylamine or an aromatic amine such as pyridine is used, and an aromatic amine such as pyridine is preferably used.

Although the reaction temperature is appropriately determined, the reaction temperature is approximately 0°C to 40°C, for example. Although the reaction time is appropriately determined, the reaction time is approximately 6 hours to 48 hours, for example.

The compound of formula (im-4) may be a commercially available product, or may be produced by a conventionally-known method.

The compound of formula (im-3) may be produced by at least one method described in the present specification (see Production Method 5).

Production Method 3

A compound of formula (1-3) can be produced by a method including a reaction step of a compound of formula (im-5) and a compound of formula (im-6), as shown in Scheme 3.

[Chemical Formula 24]

SCHEME 3

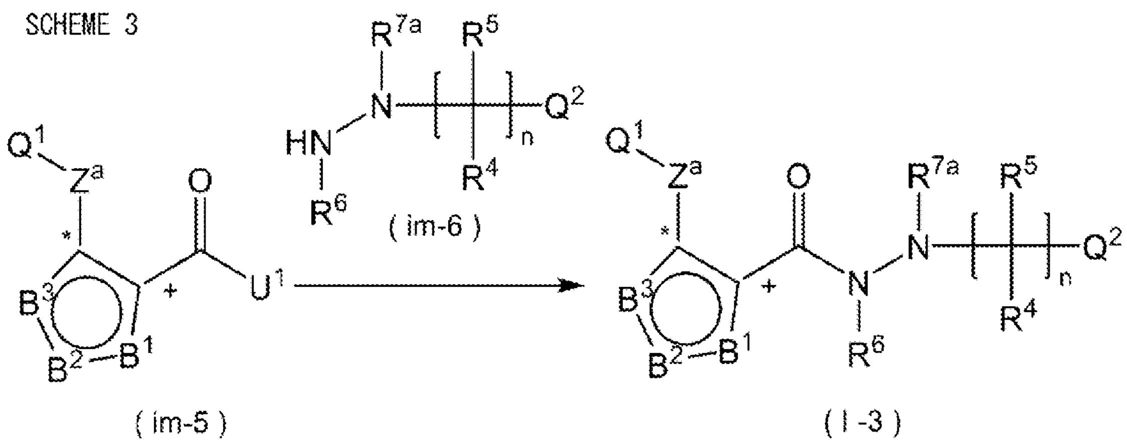

Among the compounds (I), the compound of formula (I-3) is a compound in which Z is an oxygen atom, and $R^7$ is "a formyl group, a group of formula: $R^c$-CO-, a group of formula: $R^c$-CS-, a group of formula: $R^c$-CO-CO-, or a group of formula: $R^c$-$SO_2$-" (hereinafter, may be indicated as $R^{7a}$).

$R^{7a}$ is preferably a group of formula: $R^c$-CO-.

The compound of formula (im-5) is a compound in which $U^1$ is a hydroxy group or a halogeno group, and preferably a hydroxy group, and the other symbols are identical to those defined in the compound of formula (I).

The compound of formula (im-6) is a compound in which symbols are identical to those defined in the compound of formula (I-3).

The compound of formula (I-3) can be produced by reacting the compound of formula (im-5) in which $U^1$ is a hydroxy group with the compound of formula (im-6) in the presence of a condensing agent.

Although examples of the condensing agent include halogenating agents (such as phosgene, phosphorus tribromide, phosphorus trichloride, phosphorus pentachloride, phosphorus trichloride oxide, oxalyl chloride, and thionyl chloride), dehydrating agents (such as ethyl chloroformate, methyl chloroformate, isopropyl chloroformate, isobutyl chloroformate, and methanesulfonyl chloride), and carbonyldiimidazole, the condensing agent is not limited thereto.

Although the reaction temperature is appropriately determined, the reaction temperature is approximately 0°C to

40°C, for example. Although the reaction time is appropriately determined, the reaction time is approximately 1 hour to 24 hours, for example.

Alternatively, the compound of formula (I-3) can be produced by reacting the compound of formula (im-5) in which $U^1$ is a hydroxy group with an organic base in the presence of a condensing agent. 1-Hydroxybenzotriazole may also be used in combination.

Although the organic base is not particularly limited, a tertiary amine such as triethylamine or diisopropylethylamine is preferably used, for example.

Although the condensing agent is not particularly limited, a carbodiimide such as N,N'-dicyclohexylcarbodiimide (DCC), a carbodiimide salt such as 1-ethyl-3-(3-dimethylaminopropyl)carbodiimide hydrochloride, 1-hydroxy-7-azobenzotriazole (HOAt), O-(7-azabenzotriazol-1-yl)-N,N,N',N'-tetramethyluronium hexafluorophosphate (HATU), propylphosphonic acid anhydride or the like may be used, for example, and a carbodiimide salt (1-ethyl3-(3-dimethylaminopropyl) carbodiimide hydrochloride), or HATU is preferably used.

Although the reaction temperature is appropriately determined, the reaction temperature is approximately 0°C to 40°C, for example. Although the reaction time is appropriately determined, the reaction time is approximately 1 hour to 24 hours, for example.

The compound of formula (I-3) can be produced by reacting the compound of formula (im-5) in which $U^1$ is a halogen atom with the compound of formula (im-6) by a conventionally-known method in the presence of an acid scavenger.

Examples of the acid scavenger include common inorganic bases and organic bases, and an alkali metal carbonate, alkaline earth metal acetate, tertiary amine or aromatic amine is preferably used.

The compound of formula (im-5) may be a commercially available product, or may be produced by a method described in documents (such as Japanese Translation of PCT International Application Publication No. 2022-508277 or Japanese Translation of PCT International Application Publication No 2022-514651). Alternatively, the compound of formula (im-5) may be produced by at least one method described in the present specification (see Production Methods 6 and 7).

The compound of formula (im-6) may be a commercially available product. Alternatively, the compound of formula (im-6) may be produced by at least one method described in the present specification (see Production Methods 8 and 9).

Herein, the compound of formula (I-3) can be converted to a compound in which Z is a sulfur atom by using a thionating agent.

Although examples of the thionating agent include sulfur, hydrogen sulfide acid, sodium sulfide, sodium hydrosulfide, boron trisulfide, bis(diethylaluminum)sulfide, ammonium sulfide, phosphorus pentasulfide, and Lawesson's reagent, the thionating agent is not limited thereto.

The reaction may be carried out in the presence of an inorganic base or an organic base, as needed. An alkali metal carbonate, an alkaline earth metal acetate, a tertiary amine or an aromatic amine is preferable.

Production Method 4

A compound of formula (1m-1a) can be produced by a method including a reaction step of a compound of formula (im-7) and the compound of formula (im-6), as shown in Scheme 4.

[Chemical Formula 25]

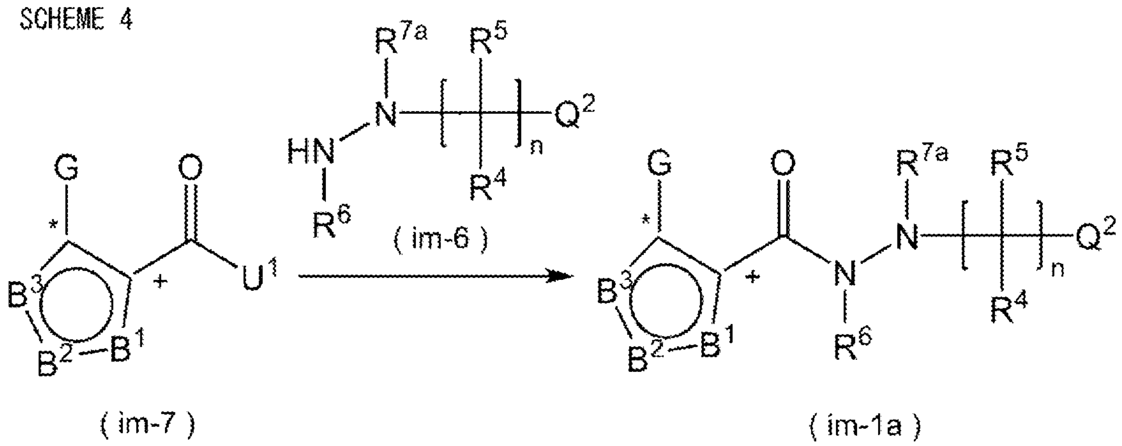


Among the compounds of formula (im-1), the compound of (1m-1a) is a compound in which Z is an oxygen atom.

The compound of formula (im-7) is a compound in which G is a halogeno group, $U^1$ is a hydroxy group or a halogeno group, and the other symbols are identical to those defined in the compound of formula (im-1).

Agents used in Production Method 4 and reaction conditions may be the same as those described in Production Method 3.

The compound of formula (im-7) may be a commercially available product.

The compound of formula (1m-1a) can be converted to a compound in which Z is a sulfur atom by using a thionating agent. As the method which allows conversion to proceed, the method described in Production Method 3 may be used.

Production Method 5

A compound of formula (1m-3a) can be produced by a method including a reaction step of a compound of formula (im-8) and the compound of formula (im-6), as shown in Scheme 5.

[Chemical Formula 26]

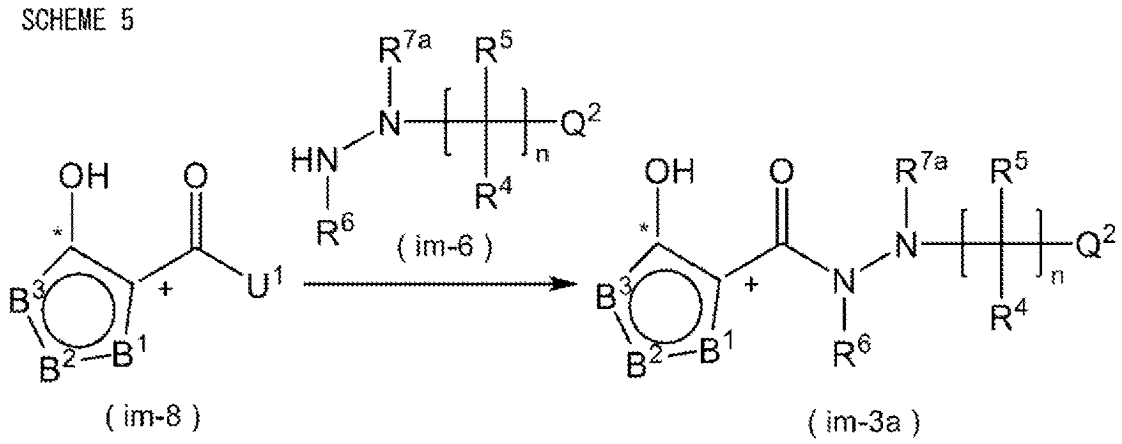


Among the compounds of formula (im-3), the compound of formula (im-3a) is a compound in which Z is an oxygen atom.

The compound of formula (im-8) is a compound in which $U^1$ is a hydroxy group or a halogeno group, and preferably a hydroxy group, and the other symbols are identical to those defined in the compound of formula (im-3).

Agents used in Production Method 5 and reaction conditions may be the same as those described in Production Method 3.

The compound of formula (im-8) may be a commercially available product. Alternatively, the compound of

formula (im-8) may be produced by at least one method described in the present specification (see Production Method 7).

The compound of formula (im-3a) can be converted to a compound in which Z is a sulfur atom by using a thionating agent. As the method which allows conversion to proceed, the method described in Production Method 3 may be used.

Production Method 6

The compound of formula (im-5) can be produced by a method including: a reaction step of a compound of formula (im-9) and the compound of formula (im-2); and a reaction step in which a compound of formula (im-10) obtained by the above-mentioned reaction step is converted to the compound of formula (im-5), as shown in Scheme 6.

[Chemical Formula 27]

SCHEME 6

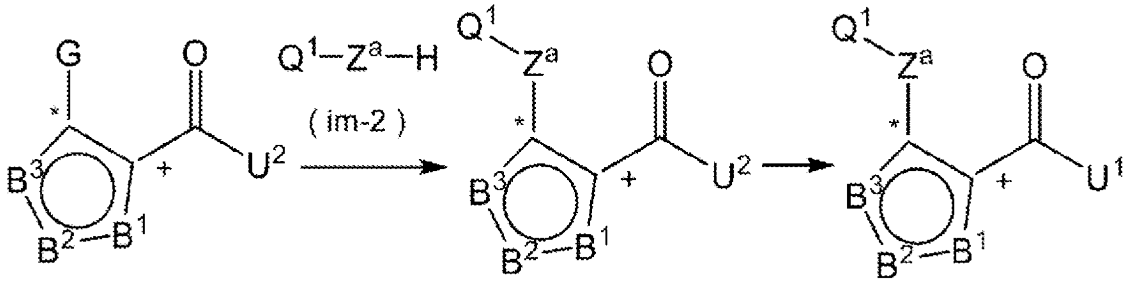


(im-9) (im-10) (im-5)

The compound of formula (im-9) is a compound in which $U^2$ is a C1-6 alkoxy group, G is a halogeno group, and the other symbols are identical to those defined in the compound of formula (im-5).

The compound of formula (im-10) is a compound in which $U^2$ is a C1-6 alkoxy group, and the other symbols are identical to those defined in the compound of formula (im-5).

The compound of formula (im-10) can be produced by reacting the compound of formula (im-9) with the compound of formula (im-2) by a conventionally-known method. The reaction may be allowed to proceed in the presence of an acid scavenger.

Examples of the acid scavenger include common inorganic bases and organic bases. An alkali metal carbonate, an alkaline earth metal acetate, a tertiary amine or an aromatic amine is preferable, and an alkali metal carbonate such as cesium carbonate or a tertiary amine such as dimethylglycine is more preferable.

In addition, a copper catalyst such as copper iodide may be used in combination.

Although the reaction temperature is appropriately determined, the reaction temperature is approximately 100°C to 200°C, for example. Although the reaction time is appropriately determined, the reaction time is approximately 0.5 hours to 6 hours, for example.

The compound of formula (im-5) in which $U^1$ is a hydroxy group can be produced by hydrolyzing the compound of formula (im-10) under acidic conditions or basic conditions.

For example, a method in which trifluoroacetic acid is used to realize acidic conditions, and a method in which lithium hydroxide is used in a mixed solvent system of tetrahydrofuran (THF) and water or a mixed solvent system of alcohol such as methanol, THF and water to realize basic conditions can be mentioned. Among these, the method in which basic conditions are realized is preferable.

Although the hydrolysis temperature is appropriately determined, the hydrolysis temperature is approximately 0°C to 60°C, for example. Although the hydrolysis time is appropriately determined, the hydrolysis time is approximately 1 hour to 24 hours, for example.

The compound of formula (im-5) in which $U^1$ is a halogeno group can be produced by reacting the compound of formula (im-5) in which $U^1$ is a hydroxy group with a halogenating agent.

The compound of formula (im-9) may be a commercially available product, or may be produced by a conventionally-known method.

Production Method 7

A compound of formula (im-5a) can be produced by a method including: a reaction step of a compound of formula (im-12) and a compound of formula (im-13); and a reaction step in which a compound of formula (im-10a) obtained by the above-mentioned reaction step is converted to the compound of formula (im-5a), as shown in Scheme 7.
In addition, a compound of formula (im-8a) can be produced by a method including a reaction step in which the compound of formula (im-12) is converted thereto.
The compound of formula (im-12) can also be produced by a method including a reaction step in which a compound of formula (im-11) is reacted with a boron compound and oxidized.

[Chemical Formula 28]

SCHEME 7

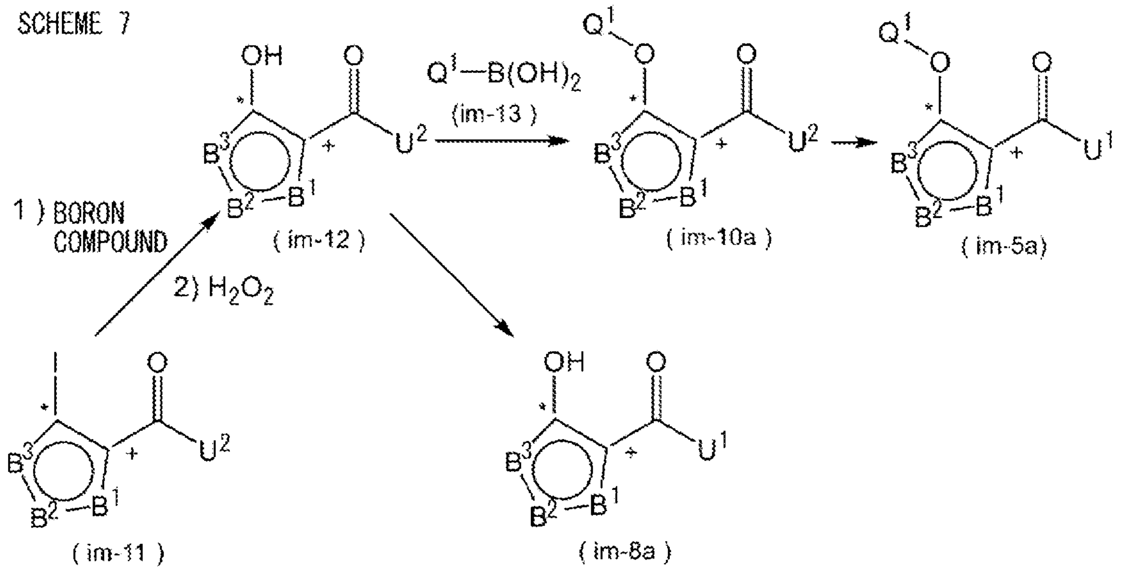


Among the compounds of formula (im-5), the compound of formula (im-5a) is a compound in which $Z^a$ is an oxygen atom.
The compound of formula (im-11) is a compound in which $U^2$ is a C1-6 alkoxy group, and the other symbols are identical to those defined in the compound of formula (Z-1).
The compound of formula (im-12) is a compound in which $U^2$ is a C1-6 alkoxy group, and the other symbols are identical to those defined in the compound of formula (Z-1).
The compound of formula (im-13) is a compound in which $Q^1$ is identical to that defined in the compound of formula (Z-1).
The compound of formula (im-10a) is a compound in which $U^2$ is a C1-6 alkoxy group, and the other symbols are identical to those defined in the compound of formula (Z-1).
The compound of formula (im-8a) is identical to the compound of formula (im-8).
The compound of formula (im-10a) can be produced by reacting the compound of formula (im-12) and the compound of formula (im-13) in the presence of an organic base and a copper catalyst with oxygen as an oxidizing agent. This reaction is known as Chan-Lam-Evans coupling.
Although the copper catalyst is not particularly limited, a divalent copper catalyst such as copper(II) chloride, copper(II) bromide, copper(II) oxide, copper(II) acetate, copper(II) sulfate, copper(II) bis(2,2,6,6-tetramethyl-3,5-heptanedionate), or copper(II) trifluoromethanesulfonate is used, and copper(II) acetate is preferably used.
Although the organic base is not particularly limited, a tertiary amine such as triethylamine or an aromatic amine such as pyridine may be used, for example, and an aromatic amine such as pyridine is preferably used.
Although the reaction temperature is appropriately determined, the reaction temperature is approximately 0°C to 40°C, for example. Although the reaction time is appropriately determined, the reaction time is approximately 6 hours to 48 hours, for example.
The compound of formula (im-5a) in which $U^1$ is a hydroxy group can be produced by hydrolyzing the compound of formula (im-10a) under acidic conditions or basic conditions.
For example, a method in which trifluoroacetic acid is used to realize acidic conditions, and a method in which

lithium hydroxide is used in a mixed solvent system of THF and water or a mixed solvent system of alcohol such as methanol, THF and water to realize basic conditions can be mentioned. Among these, the method in which basic conditions are realized is preferable.

Although the hydrolysis temperature is appropriately determined, the hydrolysis temperature is approximately 0°C to 60°C, for example. Although the hydrolysis time is appropriately determined, the hydrolysis time is approximately 1 hour to 24 hours, for example.

The compound of formula (im-5a) in which $U^1$ is a halogeno group can be produced by reacting the compound of formula (im-5a) in which $U^1$ is a hydroxy group with a halogenating agent.

The compound of formula (im-8a) in which $U^1$ is a hydroxy group can be produced by hydrolyzing the compound of formula (im-12) under acidic conditions or basic conditions.

For example, a method in which trifluoroacetic acid is used to realize acidic conditions, and a method in which lithium hydroxide is used in a mixed solvent system of THF and water to realize basic conditions can be mentioned. Among these, the method in which acidic conditions are realized is preferable.

Although the hydrolysis temperature is appropriately determined, the hydrolysis temperature is approximately 0°C to 60°C, for example. Although the hydrolysis time is appropriately determined, the hydrolysis time is approximately 1 hour to 24 hours, for example.

The compound of formula (im-8a) in which $U^1$ is a halogeno group can be produced by reacting the compound of formula (im-8a) in which $U^1$ is a hydroxy group with a halogenating agent.

The compound of formula (im-13) may be a commercially available product, or may be produced by a conventionally-known method.

The compound of formula (im-12) may be a commercially available product, or may be produced by a conventionally-known method.

Alternatively, the compound of formula (im-12) can be produced by reacting the compound of formula (im-11) with a boron compound in the presence of a palladium catalyst to convert the compound of formula (im-11) into an organoboron compound, followed by carrying out oxidization.

The method for converting into the organoboron compound is the so-called Miyaura-Ishiyama boronation reaction.

Although the palladium catalyst is not particularly limited, examples thereof include tetrakis(triphenylphosphine) palladium(0), bis(dibenzylideneacetone)palladium(0), tris(dibenzylideneacetone)dipalladium(0), bis(triphenylphosphine)palladium(II) dichloride, [1,1'-bis(diphenylphosphino)ferrocene]dichloropalladium(II), and [1,1'-bis(di-tert-butylphosphino)ferrocene]dichloropalladium(II), and [1,1'-bis(diphenylphosphino)ferrocene]dichloropalladium(II) is preferably used.

Although the boron compound is not particularly limited, examples thereof include bis(pinacolato)diboron, pinacolborane, and diboronic acid, and bis(pinacolato)diboron is preferably used.

The compound of formula (im-12) in which a carbon-oxygen bond is formed from a carbon-boron bond can be produced by oxidizing the obtained organoboron compound with hydrogen peroxide under basic conditions.

The compound of formula (im-11) may be a commercially available product, or may be produced by a conventionally-known method.

Production Method 8

A compound of formula (im-6a) can be produced by a method including: a reaction step of a compound of formula (im-19) and a compound of formula (im-20); and a reaction step in which a compound of formula (im-21) obtained by the reaction and a hydrazine, as shown in Scheme 8.

[Chemical Formula 29]

SCHEME 8

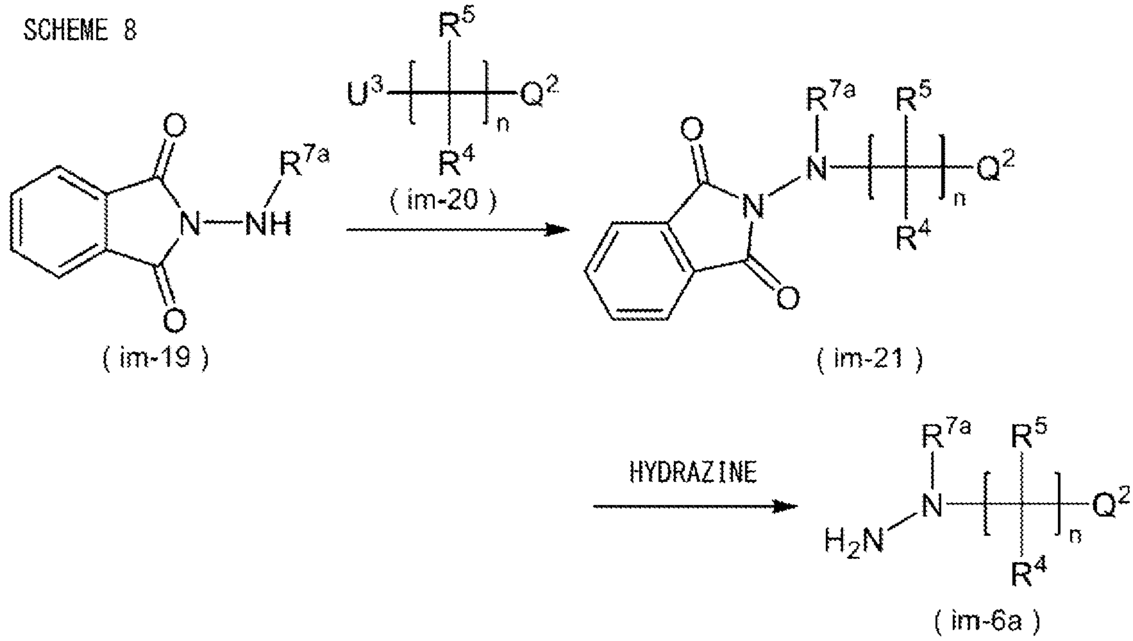


(im-19) (im-20) (im-21) (im-6a)

Among the compounds of the formula (im-6), the compound of formula (im-6a) is a compound in which $R^6$ is a hydrogen atom.

The compound of formula (im-19) is a compound in which $R^{7a}$ is identical to that defined in the compound of formula (im-6).

The compound of formula (im-20) is a compound in which $U^3$ is a hydroxy group or a halogeno group, and the other symbols are identical to those defined in the compound of formula (im-6).

The compound of formula (im-21) is a compound in which symbols are identical to those defined in the compound of formula (im-6).

The compound of formula (im-21) can be produced by reacting the compound of formula (im-20) in which $U^3$ is a hydroxy group with the compound of formula (im-19) in the presence of dialkyl azodicarboxylate and triphenylphosphine, the so-called Mitunobu reaction.

Although examples of the dialkyl azodicarboxylate include diethyl azodicarboxylate, and diisopropyl azodicarboxylate, the dialkyl azodicarboxylate is not limited thereto.

The compound of formula (im-21) can be produced by reacting the compound of formula (im-20) in which $U^3$ is a halogen atom with the compound of formula (im-19) in the presence of an acid scavenger by a conventionally-known method.

Although the reaction time is appropriately determined, the reaction time is approximately 1 hour to 24 hours, for example. Although the reaction temperature is appropriately determined, the reaction temperature is approximately 0°C to 100°C, for example.

Examples of the acid scavenger include common inorganic bases and organic bases. An alkali metal carbonate such as potassium carbonate, an alkaline earth metal acetate, a tertiary amine or an aromatic amine is preferable, and an alkali metal carbonate is more preferable.

The compound of formula (im-6a) can be produced by reacting the compound of formula (im-21) with a hydrazine.

Although the hydrazine is not particularly limited, a hydrazine monohydrate, methylhydrazine, or the like may be used, and hydrazine monohydrate is preferably used.

The compound of formula (im-19) may be a commercially available product, or may be produced by reacting a compound of formula: $NH_2$-NH-$R^{7a}$ (wherein $R^{7a}$ is identical to that defined in the compound of formula (im-6)) with phthalic anhydride.

The compound of formula (im-20) may be a commercially available product, or may be produced by a conventionally-known method.

Production Method 9

The compound of formula (im-6c) can be produced by a method including a reaction step of the compound of

formula (im-6a) and the compound of formula (im-23), as shown in Scheme 9.

[Chemical Formula 30]

SCHEME 9

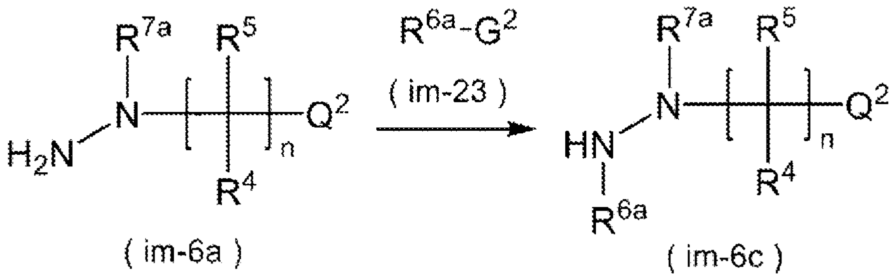


( im-6a ) ( im-6c )

Among the compounds of formula (im-6), the compound of formula (im-6c) is a compound in which $R^6$ is a group other than a hydrogen atom (hereinafter, may be indicated as $R^{6a}$).
The compound of formula (im-23) is a compound in which $G^2$ is a halogeno group, and $R^{6a}$ is identical to that defined in the formula (im-6c).
The compound of formula (im-6c) can be produced by reacting the compound of formula (im-6a) with the compound of formula (im-23). The reaction is preferably carried out in the presence of an inorganic base.
The compound of formula (im-23) may be a commercially available product, or may be produced by a conventionally-known method.

Production Method 10

A compound of formula (im-5c) can be produced by a method including: a reaction step of a compound of formula (im-24) and a compound of formula (im-2a); and a reaction step in which a compound of formula (im-25) obtained by the above-mentioned reaction step is converted to the compound of formula (im-5c), as shown in Scheme 10.

[Chemical Formula 31]

SCHEME 10

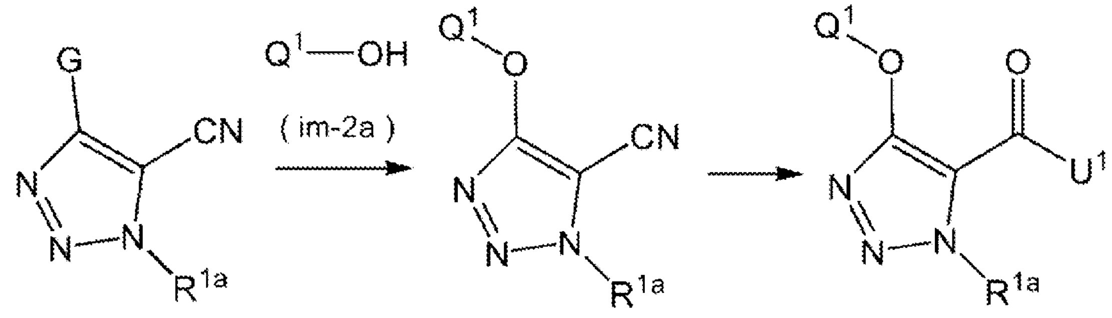


( im-24 ) ( im-25) ( im-5c)

The compound of formula (im-24) is a compound in which G is a halogeno group, and $R^{1a}$ is identical to that defined in a compound of formula (im-5B) shown below.
The compound of formula (im-2a) is a compound in which $Q^1$ is identical to that defined in the compound of formula (im-5B) shown below.
The compound of formula (im-25) is a compound in which $Q^1$ and $R^{1a}$ are identical to those defined in the compound of formula (im-5B) shown below.
The compound of formula (im-5c) is a compound in which $U^1$ is a hydroxy group or a halogeno group, and $Q^1$ and $R^{1a}$ are identical to those defined in the compound of formula (im-5B) shown below.
The compound of formula (im-25) can be produced by reacting the compound of formula (im-24) with the

compound of formula (im-2a) by a conventionally-known method. The reaction may be carried out in the presence of an acid scavenger.

Examples of the acid scavenger include common inorganic bases and organic bases. An alkali metal carbonate, an alkaline earth metal acetate, a tertiary amine or an aromatic amine is preferable, and an alkali metal carbonate such as potassium carbonate or cesium carbonate, or a tertiary amine such as dimethylglycine is more preferable.

Although the reaction temperature is appropriately determined, the reaction temperature is approximately 100°C to 200°C, for example. Although the reaction time is appropriately determined, the reaction time is approximately 0.5 hours to 6 hours, for example.

The compound of formula (im-5c) in which $U^1$ is a hydroxy group can be produced by hydrolyzing the compound of formula (im-25) under acidic conditions or basic conditions.

For example, a method in which trifluoroacetic acid is used to realize acidic conditions, and a method in which lithium hydroxide is used in a mixed solvent system of tetrahydrofuran (THF) and water or a mixed solvent system of alcohol such as methanol, THF and water to realize basic conditions can be mentioned. Among these, the method in which basic conditions are realized is preferable.

Although the hydrolysis temperature is appropriately determined, the hydrolysis temperature is approximately 0°C to 60°C, for example. Although the hydrolysis time is appropriately determined, the hydrolysis time is approximately 1 hour to 24 hours, for example.

Alternatively, in a case where an amide is obtained using an aqueous solution of alkali metal hydroxide such as sodium hydroxide in an alcohol solvent such as ethanol without hydrolyzing to a carboxylic acid, a carboxylic acid is formed by carrying out the reaction with sodium nitrite or nitrite ester in an acetic acid solvent.

Although the reaction temperature is appropriately determined, the reaction temperature is, for example, approximately 0°C to 100°C and preferably approximately 0°C to 10°C. Although the hydrolysis time is appropriately determined, the hydrolysis time is approximately 1 hour to 6 hours, for example.

The compound of formula (im-5c) in which $U^1$ is a halogeno group can be produced by reacting the compound of formula (im-5c) in which $U^1$ is a hydroxy group with a halogenating agent.

The compound of formula (im-24) may be a commercially available product, or may be produced by a conventionally-known method.

Production Method of compound (I')

Among the compounds (Z-1), a compound in which T in the formula (Z-1) is a partial structure (T-2) is a compound of the following formula (I'). Each symbol in formula (I') is identical to that in the formula (Z-1).

[Chemical Formula 32]

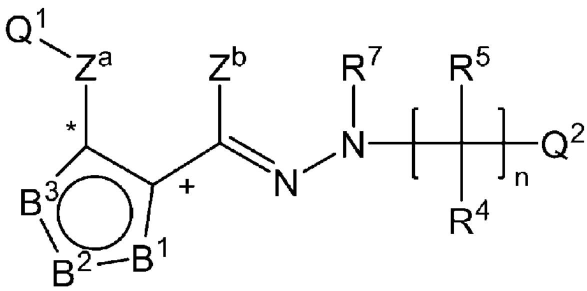

(I')

The compound of formula (I') (hereinafter, may be indicated as compound (I')) can be produced by thioamidating the compound (I) using a thionating agent such as Lawesson's reagent, followed by reacting the resultant with a corresponding alkyl halide, when $Z^b$ is a C1-6 alkylthio group.

The compound (I') can be produced by reacting the compound (I) with an oxonium salt such as trimethyloxonium tetrafluoroborate or triethyloxonium tetrafluoroborate, when $Z^b$ is a C1-6 alkoxy group.

Intermediate product

A compound of the following formula (im-5A) or a salt thereof may be an intermediate product of the compound (Z-1).

[Chemical Formula 33]

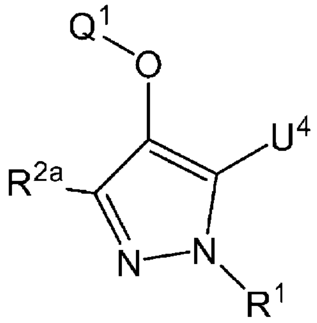

(im-5A)

In the formula (im-5A), $R^1$ and $Q^1$ are identical to those defined in the compound of formula (Z-1).

Among these, $R^1$ is preferably a C1-6 alkyl group.

$Q^1$ is preferably a substituted or unsubstituted phenyl group or a substituted or unsubstituted thiazolyl group.

As a substituent on the "phenyl group", a halogeno group, a C1-6 alkyl group, a C1-6 haloalkyl group, a C1-6 alkoxy group, a C1-6 alkylthio group, a C3-6 cycloalkyl group, or an amino group substituted with a C1-6 alkyl group is preferable, and a halogeno group, a C1-6 alkyl group or a C1-6 haloalkyl group is more preferable.

As a substituent on the "thiazolyl group", a C1-6 haloalkyl group is preferable.

$R^{2a}$ is a C1-6 haloalkyl group, a cyano group, or a halogeno group. Among these, $R^{2a}$ is preferably a cyano group or a halogeno group.

$U^4$ is a carboxyl group, a C1-6 alkoxycarbonyl group, or a halogenocarbonyl group. Among these, $U^4$ is preferably a carboxyl group or a C1-6 alkoxycarbonyl group.

Intermediate product

A compound of the following formula (im-5B) or a salt thereof may be an intermediate product of the compound (Z-1).

[Chemical Formula 34]

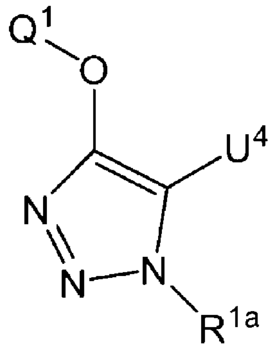

(im-5B)

In the formula (im-5B), $Q^1$ is identical to that defined in the formula (Z-1). Among these, $Q^1$ is preferably a substituted or unsubstituted phenyl group. As a substituent on the "phenyl group", a halogeno group, a C1-6 alkyl group, or a C1-6 haloalkyl group is preferable, and a C1-6 haloalkyl group is more preferable.

$R^{1a}$ is a substituted or unsubstituted C2-6 alkyl group or a substituted or unsubstituted C3-6 cycloalkyl group. As a substituent on the "C2-6 alkyl group" or "C3-6 cycloalkyl group", a halogeno group is preferable. Among these, $R^{1a}$ is preferably a t-butyl group.

$U^4$ is a carboxyl group, a C1-6 alkoxycarbonyl group, or a halogenocarbonyl group. Among these, $U^4$ is preferably a carboxyl group or a C1-6 alkoxycarbonyl group.

Intermediate product

A compound of the following formula (d-1) (hereinafter, may be referred to as compound (d-1)) or a compound of

the following formula (d-2) ((hereinafter, may be referred to as compound (d-2)) may be an intermediate product of the compound (Z-1).

[Chemical Formula 35]

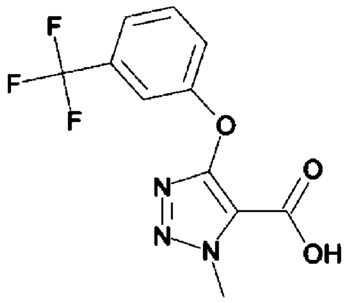

(d-1)

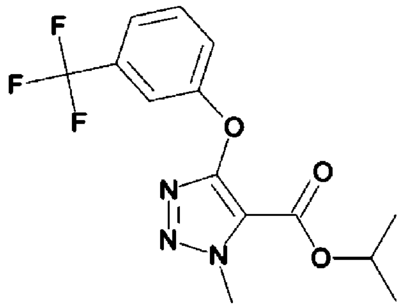

(d-2)

Intermediate product

A compound of the following formula (im-25) or a salt thereof may be an intermediate product of the compound (Z-1).

[Chemical Formula 36]

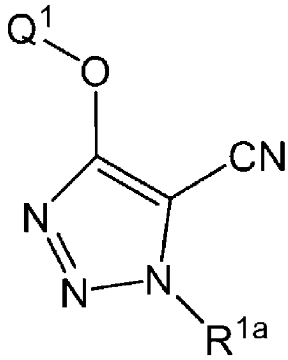

(im-25)

In the formula (im-25), $Q^1$ is identical to that defined in the compound of the formula (Z-1). Among these, $Q^1$ is preferably a substituted or unsubstituted phenyl group. As a substituent on the "phenyl group", a halogeno group, a C1-6 alkyl group, or a C1-6 haloalkyl group is preferable, a halogeno group or a C1-6 haloalkyl group is more preferable, and a C1-6 haloalkyl group is even more preferable.

$R^{1a}$ is a substituted or unsubstituted C2-6 alkyl group, or a substituted or unsubstituted C3-6 cycloalkyl group. As a substituent on the "C2-6 alkyl group" or "C3-6 cycloalkyl group", a halogeno group is preferable. Among these, $R^{1a}$ is preferably an unsubstituted C2-6 alkyl group, and more preferably a t-butyl group.

An agricultural and horticultural fungicide according to the present invention contains at least one selected from the group consisting of the compound (Z-I) and the salt of the compound (Z-I), as an active ingredient (hereinafter, may be indicated as an active ingredient (Z-I)). The amount of the active ingredient (Z-I) contained in the agricultural and horticultural fungicide according to the present invention is not particularly limited, as long as fungicidal effects are exhibited.

The agricultural and horticultural fungicide according to the present invention can be used to control plant diseases caused by a wide variety of filamentous fungi, such as fungi belonging to algae fungi (Oomycetes), sac fungi (Ascomycetes), imperfect fungi (Deuteromycetes), Basidiomycete fungi (Basidiomycetes) or zygomycete fungi (Zygomycetes).

Examples of plant diseases (pathogens) to be controlled include the following.

Sugar beet: brown spot disease (Cercospora beticola), black root disease (Aphanomyces cochlioides), root rot disease (Thanatephorus cucumeris), leaf rot disease (Thanatephorus cucumeris), rust disease (Uromyces betae), powdery mildew disease (Oidium sp.), spot blotch disease (Ramularia beticola), damping-off disease (Aphanomyces cochlioides, or Pythium ultimum), and the like.

Peanut: brown spot disease (Mycosphaerella arachidis), leaf mold (Ascochyta sp.), rust disease (Puccinia arachidis), damping-off disease (Pythium debaryanum), rust spot disease (Alternaria alternata), stem rot disease (Sclerotium rolfsii), black rust disease (Mycosphaerella berkeleyi), red crown rot disease (Calonectria ilicicola), and the like.

Cucumber: powdery mildew disease (Sphaerotheca fuliginea), downy mildew disease (Pseudoperonospora cubensis), gummy stem blight disease (Mycosphaerella melonis), wilt disease (Fusarium oxysporum), sclerotinia rot disease (Sclerotinia sclerotiorum), gray mold disease (Botrytis cinerea), anthracnose (Colletotrichum orbiculare), scab (Cladosporium cucumerinum), brown spot disease (Corynespora cassiicola), damping-off disease (Pythium debaryanum, Rhizoctonia solani Kuhn), Phomopsis root rot disease (Phomopsis sp.), Bacterial spot (Pseudomonas syringae pv. Lachrymans), and the like.

Tomato: gray mold disease (Botrytis cinerea), leaf mold disease (Cladosporium fulvum), late blight disease (Phytophthora infestans), verticillium wilt disease (Verticillium albo-atrum, Verticillium dahliae), powdery mildew disease (Oidium neolycopersici), early blight disease (Alternaria solani), leaf mold disease (Pseudocercospora fuligena), bacterial wilt disease (Ralstonia solanacearum), sclerotinia rot disease (Sclerotinia sclerotiorum), and the like.

Eggplant: gray mold disease (Botrytis cinerea), black rot disease (Corynespora melongenae), powdery mildew disease (Erysiphe cichoracearum), leaf mold disease (Mycovellosiella nattrassii), sclerotinia rot disease (Sclerotinia sclerotiorum), verticillium wilt disease (Verticillium dahliae), phomopsis blight disease (Phomopsis vexans), and the like.

Pepper: Phytophthora rot disease (Phytophthora capsici), gray mold disease (Botrytis cinerea), sclerotinia rot disease (Sclerotinia sclerotiorum), anthracnose (Colletotrichum aenigma, Colletotrichum capsici, Colletotrichum fructicola, Colletotrichum jiangxiense), powdery mildew disease (Leveillula taurica), and the like.

Strawberry: gray mold disease (Botrytis cinerea), powdery mildew disease (Sphaerotheca humuli), anthracnose (Colletotrichum acutatum, Colletotrichum fragariae), phytophthora rot disease (Phytophthora cactorum), soft rot disease (Rhizopus stolonifer), fusarium wilt disease (Fusarium oxysporum), verticillium wilt disease (Verticillium dahliae), sclerotinia rot disease (Sclerotinia sclerotiorum), and the like.

Onion: gray-mold neck rot disease (Botrytis allii), gray mold disease (Botrytis cinerea), leaf blight disease (Botrytis squamosa), downy mildew disease (Peronospora destructor), phytophthora porri disease (Phytophthora porri), leaf blight disease (Ciobrinia allii), small sclerotial neck rot disease (Botrytis squamosa), fusarium basal rot disease (Fusarium oxysporum), pink root rot disease (Pyrenochaeta terrestris), white rot disease (Sclerotium cepivorum), rust disease (Puccinia allii), southern blight disease (Sclerotium rolfsii) and the like.

Green onion: soft rot disease (Pectobacterium carotovorum), downy mildew disease (Peronospora destructor), leaf scorch disease (Pleospora allii), **bulb** black rot disease (Sclerotium cepivorum), rust disease (Puccinia allii), leaf blight disease (Botrytis squamosa), southern blight disease (Sclerotium rolfsii), pink root rot disease (Pyrenochaeta terrestris), and the like.

Cabbage: clubroot disease (Plasmodiophora brassicae), soft rot disease (Erwinia carotovora), black rot disease (Xanthomonas campesrtis pv. campestris), bacterial black spot disease (Pseudomonas syringae pv. maculicala, Pseudomonas syringae pv. alisalensis), downy mildew disease (Peronospora parasitica), sclerotinia rot disease (Sclerotinia sclerotiorum), black spot disease (Alternaria brassicicola), gray mold disease (Botrytis cinerea), black leg disease (Phoma lingam), Pythium rot disease (Pythium aphanidermatum, Pythium ultimum), white rust disease (Albugo macrospora),and the like.

Lettuce: bacterial rot disease (Pseudomonas cichorii, Pseudomonas marginalis), bacterial soft rot disease (Pectobacterium carotovorum), downy mildew disease (Bremia lactucae), gray mold disease (Botrytis cinerea), stem rot disease (Sclerotinia sclerotiorum), big vein disease (Mirafiori lettuce big-vein ophiovirus), root rot disease (Fusarium oxysporum), bottom rot disease (Rhizoctonia solani), powdery mildew disease (Golovinomyces orontii), and the like.

Common bean: sclerotinia rot disease (Sclerotinia sclerotiorum), gray mold disease (Botrytis cinerea), anthracnose (Colletotrichum lindemuthianum), angular spot disease (Phaeoisariopsis griseola), and the like.

Pea: Mycosphaerella blight disease (Mycosphaerella blight), gray mold disease (Botrytis cinerea), stem rot disease (Sclerotinia sclerotiorum), powdery mildew disease (Erysiphe pisi), and the like.

Apple: powdery mildew disease (Podosphaera leucotricha), scab disease (Venturia inaequalis), Monilinia disease (Monilinia mali), black spot disease (Mycosphaerella pomi), valsa canker disease (Valsa mali), alternaria blotch disease (Alternaria mali), rust disease (Gymnosporangium yamadae), ring rot disease (Botryosphaeria berengeriana), anthracnose (Glomerella cingulata, Colletotrichum acutatum), leaf srot disease (Diplocarpon mali), fly speck disease (Zygophiala jamaicensis), Sooty blotch disease (Gloeodes pomigena), violet root rot disease (Helicobasidium mompa), white root rot disease (Rosellinia necatrix), gray mold disease (Botrytis cinerea), fire blight disease (Erwinia amylovora), silver leaf disease (Chondrostereum purpureum), crown gall disease (Rhizobium radiobacter, Rhizobium rhizogenes) and the like.

Japanese apricot: scab disease (Cladosporium carpophilum), gray mold disease (Botrytis cinerea), brown rot disease (Monilinia mumecola), sooty blotch disease (Peltaster sp.), plum pockets disease (Taphrina pruni), cylindrosporium leaf spot disease (Phloeosporella padi), and the like.

Persimmon: powdery mildew disease (Phyllactinia kakicola), anthracnose (Gloeosporium kaki), angular leaf spot (Cercospora kaki), circular leaf spot (Mycosphaerella nawae), gray mold disease (Botrytis cinerea), fly speck disease (Zygophiala jamaicensis) and the like.

Peach: brown rot disease (Monilinia fructicola, Monilia fructigena), scab disease (Cladosporium carpophilum), phomopsis rot disease (Phomopsis sp.), bacterial shot hole disease (Xanthomonas campestris pv. pruni), leaf curl disease (Taphrina deformans), anthracnose (Colletotrichum gloeosporioides), cylindrosporium leaf spot disease (Phloeosporella padi), Coriolus versicolor disease (Coriolus versicolor) and the like.

Almond: brown rot disease (Monilinia laxa), spot blotch disease (Stigmina carpophila), scab disease (Cladosporium carpophilum), red leaf spot disease (Polystigma rubrum), alternaria blotch disease (Alternaria alternata), anthracnose (Colletotrichum gloeospoides), and the like.

Yellow peach: brown rot disease (Monilinia fructicola), anthracnose (Colletotrichum acutatum), black spot disease (Alternaria sp.), Monilinia Kusanoi disease (Monilinia kusanoi), Mycosphaerella leaf spot disease (Mycosphaerella cerasella), powdery mildew disease (Podosphaera tridactyla), and the like.

Grape: gray mold disease (Botrytis cinerea), powdery mildew disease (Uncinula necator), ripe rot disease (Glomerella cingulata, Colletotrichum acutatum), downy mildew disease (Plasmopara viticola), anthracnose (Elsinoe ampelina), brown spot disease (Pseudocercospora vitis), black rot disease (Guignardia bidwellii), white rot disease (Coniella castaneicola), rust disease (Phakopsora ampelopsidis), Cottony bunch disease (the pathogen thereof has not been identified), crown gall disease (Rhizobium radiobacter, Rhizobium vitis), and the like.

Pear: scab disease (Venturia nashicola), rust disease (Gymnosporangium asiaticum), black spot disease (Alternaria kikuchiana), ring rot disease (Botryosphaeria berengeriana), powdery mildew disease (Phyllactinia mali), cytospora canker disease (Phomopsis fukushii), brown spot blotch disease (Stemphylium vesicarium), anthracnose (Glomerella cingulata), and the like.

Tea: ring spot disease (Pestalotiopsis longiseta, P. theae), anthracnose (Colletotrichum theae-sinensis), net blister blight disease (Exobasidium reticulatum), bacterial shoot blight disease (Pseudomonas syringae), blister blight disease (Exobasidium vexans), and the like.

Citrus fruits: scab disease (Elsinoe fawcetti), blue mold disease (Penicillium italicum), common green mold disease (Penicillium digitatum), gray mold disease (Botrytis cinerea), melanose disease (Diaporthe citri), canker disease (Xanthomonas campestris pv.Citri), powdery mildew disease (Oidium sp.), brown rot disease (Phytophthora citrophthora), anthracnose (Colletotrichum fioriniae), and the like.

Kiwi fruit: bacterial bud rot disease (Pseudomonas marginalis, Pseudomonas syringae, Pseudomonas viridiflava), bacterial canker disease (Pseudomonas syringae), gray mold disease (Botrytis cinerea), soft rot disease (Botryosphaeria dothidea, Diaporthe sp., Lasiodiplodia theobromae), sooty spot disease (Pseudocercospora actinidiae), and the like.

Olive: anthracnose (Colletotrichum acutatum, Colletotrichum gloeosporioides), Peacock spot (Spilocaea oleaginea), and the like.

Chestnut: anthracnose (Colletotrichum gloeosporioides), and the like.

Wheat: powdery mildew disease (Blumeria graminis f.sp. tritici), red mold disease (Gibberella zeae, Fusarium avenaceum, Fusarium culmorum, Fusarium crookwellense, Microdochium nivale), red rust disease (Puccinia recondita), yellow rust disease (Puccinia striiformis), brown snow mold disease (Pythium iwayamai), pink snow mold disease (Monographella nivalis), eye spot disease (Pseudocercosporella herpotrichoides), leaf scorch disease (Septoria tritici), glume blotch disease (Leptosphaeria nodorum), typhulasnow blight disease (Typhula incarnata), sclerotinia snow blight disease (Myriosclerotinia borealis), damping-off disease (Gaeumannomyces graminis), ergot disease (Claviceps purpurea), stinking smut disease (Tilletia caries), loose smut disease (Ustilago nuda), blast disease (Pyricularia grisea), damping-off disease (Pythium spp., Fusarium spp., Rhizoctonia spp.), seedling blight disease (Pythium spp., Fusarium spp., Rhizoctonia spp.) and the like.

Barley: leaf spot disease (Pyrenophora graminea), net blotch disease (Pyrenophora teres), leaf blotch disease (Rhynchosporium secalis), loose smut disease (Ustilago tritici, U.nuda), damping-off disease (Pythium spp., Fusarium spp., Rhizoctonia spp.), seedling blight disease (Pythium spp., Fusarium spp., Rhizoctonia spp.) and the like.

Rice: blast disease (Pyricularia oryzae), sheath blight disease (Rhizoctonia solani), bakanae disease (Gibberella fujikuroi), brown spot disease (Cochliobolus miyabeanus), damping-off disease (Pythium graminicolum), bacterial leaf blight disease (Xanthomonas oryzae), bacterial seedling blight disease (Burkholderia plantarii), brown stripe disease (Acidovorax avenae), bacterial grain rot disease (Burkholderia glumae), Cercospora leaf spot disease (Cercospora oryzae), false smut disease (Ustilaginoidea virens), rice brown spot disease (Alternaria alternata, Curvularia intermedia), kernel discoloration of rice (Alternaria padwickii), pink coloring of rice grains (Epicoccam purpurascenns), and the like.

Tobacco: sclerotinia rot disease (Sclerotinia sclerotiorum), powdery mildew disease (Erysiphe cichoracearum), phytophthora rot disease (Phytophthora nicotianae), and the like.

Tulip: gray mold disease (Botrytis cinerea), Botrytis blight disease (Botrytis tulipae), leaf rot disease (Rhizoctonia solani), bulb rot disease (Fusarium oxysporum), bulb-coat rot disease (Rhizoctonia solani), and the like.

Rose: black spot disease (Diplocarpon rosae), powdery mildew disease (Erysiphe simulans, Podosphaera pannosa), gray mold disease (Botrytis cinerea), and the like.

Chrysanthemum: gray mold disease (Botrytis cinerea), rust disease (Puccinia horiana), downy mildew disease (Paraperonospora minor, Peronospora danica), Pythium root and stem rot disease (Pythium aphanidermatum, Pythium dissotocum, Pythium helicoides, Pythium oedochilum, Pythium sylvaticum), wilt disease (Rhizoctonia solani), Fusarium blight disease (Fusarium solani), and the like.

Gerbera: gray mold disease (Botrytis cinerea), powdery mildew disease (Podosphaera xanthii), and the like.

Lily: Botrytis blight disease (Botrytis elliptic a, Pestalotiopsis sp.), gray mold disease (Botrytis cinerea), and the like.

Sunflower: downy mildew disease (Plasmopara halstedii), sclerotinia rot disease (Sclerotinia sclerotiorum), gray mold disease (Botrytis cinerea), and the like.

Bent grass: Sclerotinia snow blight disease (Sclerotinia borealis), large patch (Rhizoctonia solani), brown patch (Rhizoctonia solani), Dollar spot (Sclerotinia homoeocarpa), blast disease (Pyricularia sp.), Pythium red blight disease (Pythium aphanidermatum), anthracnose (Colletotrichum graminicola), and the like.

Orchard grass: powdery mildew disease (Erysiphe graminis), and the like.

Soybean: purple stain disease (Cercospora kikuchii), downy mildew disease (Peronospora manshurica), phytophthora rot disease (Phytophthora sojae), rust disease (Phakopsora pachyrhizi), sclerotinia rot disease (Sclerotinia sclerotiorum), anthracnose (Colletotrichum truncatum), gray mold disease (Botrytis cinerea), Sphaceloma scab (Elsinoe glycines), black spot disease (Diaporthe phaseolorum var. sojae), damping-off disease (Pythium spp., Fusarium spp., Rhizoctonia spp.), seedling blight disease (Pythium spp., Fusarium spp., Rhizoctonia spp.), and the like.

Potato: Phytophthora rot disease (Phytophthora infestans), early blight disease (Aleternaria solani), scurf disease (Thanatephorus cucumeris), verticillium wilt disease (Verticillium albo-atrum, V. dahliae, V. nigrescens), blackleg disease (Pectobacterium atrosepticum), soft rot disease (Pectobacterium carotovorum), gray mold disease (Botrytis cinerea), scab (Streptomyces spp.), stem rot disease (Sclerotinia sclerotiorum), and the like.

Yam: leaf mold disease (hasibu-byo) (Cylindrosporium dioscoreae), anthracnose (Colletotrichum gloeosporioides), blue mold disease (Penicillium sclerotigenum), and the like.

Sweet potato: violet root rot disease (Helicobasidium mompa), wilt disease (Fusarium oxysporum), and the like.

Taro: Phytophthora rot disease (Phytophthora colocasiae), stem rot disease (Rhizoctonia solani), and the like.

Ginger: root rot disease (Pythium ultimum, Pythium myriotylum), leaf spot disease (Phyllosticta zingiberis), and the like.

Banana: Panama disease (Fusarium oxysporum), Sigatoka disease (Mycosphaerella fijiensis, M. musicola), and the like.

Mango: anthracnose (Colletotrichum aenigma), canker disease (Xanthomonas campestris), stem-end rot disease (Diaporthe pseudophoenicicola, Lasiodiplodia theobromae, Lasiodiplodia spp., Neofusicoccum parvum, Neofusicoccum sp.), gray mold disease (Botrytis cinerea), and the like.

Rape seed: sclerotinia rot disease (Sclerotinia sclerotiorum), root rot disease (Phoma lingam), black leaf spot disease (Alternaria brassicae), powdery mildew disease (Erysiphe cruciferarum, Erysiphe cichoracearum, Oidium matthiolae), downy mildew disease (Peronospora parasitica), and the like.

Coffee: rust disease (Hemileia vastatrix), anthracnose (Colletotrichum coffeanum), brown eye spot disease (Cercospora coffeicola), and the like.

Sugarcane: brown rust disease (Puccinia melanocephala), and the like.

Corn: zonate spot disease (Gloecercospora sorghi), rust disease (Puccinia sorghi), southern rust disease (Puccinia polysora), smut disease (Ustilago maydis), brown spot disease (Cochliobolus heterostrophus), northern leaf blight disease (Setophaeria turcica), damping-off disease (Pythium spp., Fusarium spp., Rhizoctonia spp.), seedling blight disease (Pythium spp., Fusarium spp., Rhizoctonia spp.), and the like.

Cotton: seedling blight disease (Pythium sp.), rust disease (Phakopsora gossypii), sour rot disease (Mycosphaerella areola), anthracnose (Glomerella gossypii), and the like.

Hop: downy mildew disease (Pseudoperonospora humuli), powdery mildew disease (Oidium sp., Podosphaera macularis), gray mold disease (Botrytis cinerea), and the like.

The agricultural and horticultural fungicide according to the present invention is preferably applied to cereals; vegetables; root vegetables; potatoes; trees, such as fruit-bearing trees, tea, coffee, or cacao; feed crops; lawn grasses; or plants such as cotton.

The agricultural and horticultural fungicide according to the present invention may be applied to any part of plants, such as leaves, stems, stalks, flowers, buds, fruits, seeds, sprouts, roots, tubers, tuberous roots, shoots, or slips. The agricultural and horticultural fungicide according to the present invention may also be applied to varieties, improved varieties, cultivars, mutants, hybrid bodies, or gene recombinants (GMO) of the plants.

The agricultural and horticultural fungicide according to the present invention may be used to carry out seed treatment, foliage application, soil application, or submerged application so as to control various diseases generated in

agricultural and horticultural crop plants encompassing flowers, lawn grasses, and herbages.

The agricultural and horticultural fungicide according to the present invention may further contain additional components in addition to the active ingredient (Z-I). Examples of the additional components include conventional carriers used to make formulations. Examples of other additional components include conventional fungicides, insecticides, acaricides, nematicides, soil pest control agents, plant regulatory agents, synergists, fertilizers, soil improvement agents, and animal feeds (hereinafter, may be indicated as active ingredient (II)). The agricultural and horticultural fungicide according to the present invention may exhibit synergistic effects by containing the active ingredient (Z-I) and the active ingredient (II).

Specific examples of the active ingredient (II) which may be mixed or used with the agricultural and horticultural fungicide according to the present invention are shown below.

(1) Nucleic acid biosynthesis inhibitors:

(a) RNA polymerase I inhibitors: benalaxyl, benalaxyl-M, furalaxyl, metalaxyl, metalaxyl-M, oxadixyl, clozylacon, and ofurace;
(b) Adenosine deaminase inhibitors: bupirimate, dimethirimol, and ethirimol;
(c) DNA/RNA synthesis inhibitors: hymexazol, and octhilinone;
(d) DNA topoisomerase II inhibitors: oxolinic acid.

(2) Mitotic inhibitors and cell division inhibitors:

(a) β-tubulin polymerization inhibitors: benomyl, carbendazim, chlorfenazole, fuberidazole, thiabendazole, thiophanate, thiophanate-methyl, diethofencarb, zoxamide, and ethaboxam;
(b) Cell division inhibitors: pencycuron;
(c) Spectrin-like protein delocalization inhibitors: fluopicolide, and fluopimomide.

(3) Respiration inhibitors:

(a) Complex I - NADH oxidation-reduction enzyme inhibitors: diflumetorimu, and tolfenpyrad;
(b) Complex II - succinate dehydrogenase inhibitors: benodanil, flutolanil, mepronil, isofetamide, fluopyram, fenfuram, furmecyclox, carboxin, oxycarboxin, thifluzamide, benzovindiflupyr, bixafen, fluxapyroxad, furametpyr, isopyrazam, penflufen, penthiopyrad, sedaxane, boscalid, pydiflumetofen, isoflucypram, pyraziflumid, and inpyrfluxam;
(c) Complex III - ubiquinol oxidase Qo inhibitors: azoxystrobin, coumoxystrobin, coumethoxystrobin, enoxastrobin, flufenoxystrobin, picoxystrobin, pyraoxystrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole, and mandestrobin;
(d) Complex **III** - ubiquinol reductase Qi inhibitors: cyazofamid, amisulbrom, and fenpicoxamid;
(e) Oxidative phosphorylation uncoupling agents: binapacryl, meptyldinocap, dinocap, fluazinam, and ferimzone;
(f) Oxidative phosphorylation inhibitors (ATP synthase inhibitors): fentin acetate, fentin chloride, and fentin hydroxide;
(g) ATP production inhibitors: silthiofam;
(h) Complex III: Qx (unknown) inhibitor of cytochrome bc1 (ubiquinone reductase): ametoctradin.

(4) Amino acid and protein synthesis inhibitors

(a) Methionine biosynthesis inhibitors: andoprim, cyprodinil, mepanipyrim, and pyrimethanil;
(b) Protein synthesis inhibitors: blasticidin-S, kasugamycin, kasugamycin hydrochloride, streptomycin, and oxytetracycline.

(5) Signal transduction inhibitors:

(a) Signal transduction inhibitors: quinoxyfen, and proquinazid;
(b) Inhibitors of MAP / histidine kinase in osmotic signal transduction: fenpiclonil, fludioxonil, chlozolinate, iprodione, procymidone, and vinclozolin.

(6) Lipids and cell membrane synthesis inhibitors:

(a) Inhibitors of phospholipid biosynthesis and methyltransferase: edifenphos, iprobenfos, pyrazophos, and isoprothiolane;
(b) Lipid peroxidation agent: biphenyl, chloroneb, dicloran, quintozene, tecnazene, tolclofos-methyl, and etridiazole;
(c) Agents that act on the cell membrane: iodocarb, propamocarb, propamocarb-hydrochloride, propamocarb-fosetylate, and prothiocarb;
(d) Microorganisms that disrupt the cell membrane of pathogens: bacillus subtilis bacteria, bacillus subtilis strain QST713, bacillus subtilis strain FZB24, bacillus subtilis strain MBI600, bacillus subtilis strain D747, and bacillus amyloliquefaciens;
(e) Agents that disrupt the cell membrane: melaleuca alternifolia (tea tree) extract.

(7) Inhibitors of sterol biosynthesis in the cell membrane:

(a) Inhibitors of demethylation at the C14 position in sterol biosynthesis: triforine, pyrifenox, pyrisoxazole, fenarimol, flurprimidol, nuarimol, imazalil, imazalil-sulphate, oxpoconazole, oxpoconazole fumarate, pefurazoate, prochloraz, triflumizole, viniconazole, azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, fluquinconazole, simeconazole, tebuconazole, tetraconazole, triadimefon, triadimenol, triticonazole, prothioconazole, voriconazole, and mefentrifluconazole;
(b) Inhibitors of Δ14 reductase and Δ8→Δ7-isomerase in sterol biosynthesis: aldimorph, dodemorph, dodemorph acetate, fenpropimorph, tridemorph, fenpropidin, piperalin, and spiroxamine;
(c) Inhibitors of 3-keto reductase in C4 demethylation in sterol biosynthesis: fenhexamid, and fenpyrazamine;
(d) Inhibitors of squalene epoxidase in sterol biosynthesis: pyributicarb, naftifine, and terbinafine.

(8) Cell wall synthesis inhibitors

(a) Trehalase inhibitor: validamycin;
(b) Chitin synthesis inhibitors: polyoxin, and polyoxorim;
(c) Cellulose synthase inhibitors: dimethomorph, flumorph, pyrimorph, benthiavalicarb, benthiavalicarb-isopropyl, iprovalicarb, valifenalate, and mandipropamid.

(9) Melanin biosynthesis inhibitors

(a) Melanin biosynthesis reductase inhibitors: phthalide, pyroquilon, and tricyclazole;
(b) Melanin biosynthesis anhydrase inhibitors: carpropamid, diclocymet, and fenoxanil.
(c) Melanin biosynthesis polyketide synthase inhibitors: tolprocarb.

(10) Host plant resistance inducer:

(a) Agents that act on the salicylic acid synthesis pathway: acibenzolar-S-methyl;
(b) Others: probenazole, tiadinil, isotianil, dichlobentiazox, ipfentrifluconazole, laminarin, reynoutria sachalinensis extract, phosphorous acid, and phosphite.

(11) Agents, action of which is unclear: cymoxanil, fosetyl-aluminum, phosphoric acid, phosphate, tecloftalam, triazoxide, flusulfamide, diclomezine, methasulfocarb, cyflufenamid, metrafenone, pyriofenone, dodine, dodine free base, and flutianil.
(12) Agents having multi-functionality: copper (copper salt), Bordeaux mixture, copper hydroxide, copper naphthalate, copper oxide, copper oxychloride, copper sulfate, sulfur, sulfur products, calcium polysulfide, ferbam, mancozeb, maneb, mancopper, metiram, polycarbamate, propineb, thiram, zineb, ziram, captan, captafol, folpet, chlorothalonil, dichlofluanid, tolylfluanid, guazatine, guazatine triacetate (another name: iminoctadine triacetate), iminoctadine trialbesilate, anilazine, dithianon, quinomethionate, and fluoroimide.
(13) Other agents: DBEDC, fluorofolpet, guazatine acetate, bis (8-quinolinolato) copper (II), propamidine, chloropicrin, cyprofuram, agrobacterium, bethoxazin, diphenylamine, methyl isothiocyanate (MITC), mildiomycin, capsaicin, cufraneb, cyprosulfamide, dazomet, debacarb, dichlorophen, difenzoquat, difenzoquat·methyl sulfonate, flumetover, fosetyl-calcium, fosetyl-sodium, irumamycin, natamycin, nitrothal isopropyl, oxamocarb, pyrrolnitrin, tebufloquin, tolnifanide, zarilamid, algophase, amicarthiazol, oxathiapiprolin, fluoxapiprolin, metiram zinc, benthiazole, trichlamide, uniconazole, oxyfenthiin, picarbutrazox, dichlobentiazox, quinofumelin, thiuram, ambam, agrobacterium radiobacter, coniothyrium minitans, pseudomonas fluorescens, pseudomonas rhodesiae, talaromyces flavus, trichoderma atroviride, non-pathogenic erwinia carotovora, bacillus simplex, variovorax paradoxus, lactobacillus plantarum, florylpicoxamid, pyrapropoyne, fluindapyr, aminopyrifen, pyridachlometyl, ipflufenoquin, and dipymetitrone.

Specific examples of the insecticides, acaricides, nematicides, soil insect pest control agents, and anthelmintic agents as the active ingredient (II) which may be mixed or used with the agricultural and horticultural fungicide according to the present invention are shown below.

(1) Acetylcholinesterase (AChE) inhibitors:

(a) Carbamate-based acetylcholinesterase (AChE) inhibitors:
alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, fenothiocarb, MIPC, MPMC, MTMC, aldoxycarb, allyxycarb, aminocarb, bufencarb, chloethocarb, metam-sodium, and promecarb;
(b) Organophosphate-based acetylcholinesterase (AChE) inhibitors:
acephate, azamethiphos, azinphos-ethyl, azinphos-methyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isocarbophos, Isopropyl=O-(methoxyaminothiophosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion, bromophos-ethyl, BRP, carbophenothion, cyanofenphos, CYAP, demeton-S methyl sulfone, dialifos, dichlofenthion, dioxabenzofos, etrimfos, fensulfothion, flupyrazofos, fonofos, formothion, fosmethilan, isazofos, iodofenphos, methacrifos, pirimiphos-ethyl, phosphocarb, propaphos, prothoate, and sulprofos;

(2) GABAergic chloride ion channel blockers:
acetoprole, chlordane, endosulfan, ethiprole, fipronil, pyrafluoprole, pyriprole, camphechlor, heptachlor, dienochlor, and flufiprole;
(3) Sodium channel modulators

(a) Pyrethroid-based sodium channel modulators:
acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, β-cyfluthrin, cyhalothrin, λ-cyhalothrin, γ-cyhalothrin, cypermethrin, α-cypermethrin, β-cypermethrin, θ-cypermethrin, ζ-cypermethrin, cyphenothrin [(1R)-trans isomer], deltamethrin, empenthrin [(EZ)-(1R)-isomer], esfenvalerate, etofenprox, fenpropathrin, fenvalerate, flucythrinate, flumethrin, τ-fluvalinate, halfenprox, imiprothrin, kadethrin, permethrin, phenothrin [(1R)-trans isomer], prallethrin, pyrethrum, pyrethrin, resmethrin, silafluofen, tefluthrin, tetramethrin, tetramethrin [(1R)-isomer], tralomethrin, transfluthrin, allethrin, pyrethrin I, pyrethrin II, profluthrin, dimefluthrin, bioethanomethrin, biopermethrin, transpermethrin, fenfluthrin, fenpirithrin, flubrocythrinate, flufenprox, metofluthrin, protrifenbute, pyresmethrin, terallethrin, κ- bifenthrin, chloroprallethrin, heptafluthrin, meperfluthrin, ε-metofluthrin, momfluorothrin, ε- momfluorothrin, κ-tefluthrin, tetra methylfluthrin, and bioethanomethrin;
(b) Sodium channel modulators (DDTs):
DDT, and methoxychlor;

(4) Nicotinic acetylcholine receptor (nAChR) competitive modulators:
acetamiprid, clothianidin, dinotefuran, imidacloprid, nitenpyram, nithiazine, thiacloprid, thiamethoxam, nicotine, sulfoxaflor, flupyradifurone, triflumezopyrim, dichloromezotiaz, and flupyrimin;
(5) Nicotinic acetylcholine receptor (nAChR) allosteric modulators:
spinetoram, and spinosad;
(6) Glutamate-gated chloride ion channel (GluCl) allosteric modulators:
abamectin, emamectin, emamectin-benzoate, lepimectin, milbemectin, ivermectin, selamectin, doramectin, eprinomectin, ivermectin, moxidectin, selamectin, milbemycin, milbemycin oxime, and nemadectin;
(7) Juvenile hormone-like substances:

hydroprene, kinoprene, methoprene, fenoxycarb, pyriproxyfen, diofenolan, epofenonane, and triprene;
(8) Other non-specific (multi-site) inhibitors:
methyl bromide, alkyl halides, chloropicrin, sodium aluminum fluoride, sulfuryl fluoride, borax, boric acid, disodium octaborate, sodium borate, sodium metaborate, tartar emetic, dazomet, metam, metam potassium salt, and metam sodium salt;
(9) Chordotonal organ TRPV channel modulators:
flonicamid, pymetrozine, pyrifluquinazon, and afidopiropen;
(10) Acarian growth inhibitors:
clofentezine, diflovidazin, hexythiazox, and etoxazole.
(11) Insect midgut inner membrane disrupting agent derived from microorganisms:
bacillus thuringiensis subsp. Israelensis, bacillus sphaericus, bacillus thuringiensis subsp. aizawai, bacillus thuringiensis subsp. kurstaki, bacillus thuringiensis subsp. tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1 / Cry35Ab1; and bacillus sphaericus.
(12) Mitochondrial ATP biosynthetic enzyme inhibitors:
diafenthiuron, azocyclotin, cyhexatin, fenbutatin oxide, propargite, and tetradifon;
(13) Oxidative phosphorylation uncouplers that disrupt the proton gradient:
chlorfenapyr, DNOC, sulfluramid, binapacryl, dinobuton, and dinocap;
(14) Nicotinic acetylcholine receptor (nAChR) channel blockers:
bensultap, cartap hydrochloride, nereistoxin, thiocyclam, and thiosultap-sodium;
(15) Chitin biosynthesis inhibitors, type 0:
bistrifluron, chlorfluazuron, diflubenzuron, flucycloxuron, flufenoxuron, hexaflumuron, lufenuron, novaluron, noviflumuron, teflubenzuron, triflumuron, buprofezin, and fluazuron;
(16) Chitin biosynthesis inhibitors, type 1:
buprofezin;
(17) Molting inhibitors:
cyromazine;
(18) Molting hormone (ecdysone) receptor agonists:
chromafenozide, halofenozide, methoxyfenozide, and tebufenozide;
(19) Octopamine receptor agonists:
amitraz, demiditraz, and chlordimeform;
(20) Mitochondrial electron transport system complex III inhibitors:
hydramethylnon, acequinocyl, fluacrypyrim, and bifenazate;
(21) Mitochondrial electron transport system complex I inhibitors (METI):
fenazaquin, fenpyroximate, pyridaben, pyrimidifen, tebufenpyrad, tolfenpyrad, and rotenone;
(22) Voltage-dependent sodium channel blockers:
indoxacarb, and metaflumizone;
(23) Acetyl CoA carboxylase inhibitors:
spirodiclofen, spiromesifen, spirotetramat, and spiropidion.
(24) Mitochondrial electron transport system complex IV inhibitors:
aluminum phosphide, calcium phosphide, zinc phosphide, phosphine, cyanide, calcium cyanide, sodium cyanide, and potassium cyanide;
(25) Mitochondrial electron transport system complex II inhibitors:
cyenopyrafen, cyflumetofen, and pyflubumide.
(28) Ryanodine receptor modulators:
chlorantraniliprole, cyantraniliprole, cyclaniliprole, flubendiamide, cyhalodiamide, tetrachlorantraniliprole, and tetraniliprole.
(29) Chordotonal organ modulator target site unspecified:
flonicamid;
(30) GABA-gated chloride ion channel allosteric modulators:
broflanilide, fluxamethamide, isocycloseram, afoxolaner, fluralaner, lotilaner, and sarolaner;
(31) Other insecticides and acaricides:
azadirachtin, benzoximate, bifenazate, bromopropylate, quinomethionate, cryolite, dicofol, lime sulfur, mancozeb, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimine, fluphenazine, gossyplure, japonilure, metoxadiazone, oil, potassium oleate, tetrasul, triarathene, afidopyropen, flufiprole, fluensulfone, meperfluthrin, tetramethylfluthrin, tralopyril, dimefluthrin, methylneodecanamide, fluralaner, afoxolaner, fluxametamide, 5-[5-(3,5-dichlorophenyl)-5-trifluoromethyl-4,5-dihydroisoxazole-3-yl]-2-(1H-1,2,4-triazol-1-yl) benzonitrile (CAS: 943137-49-3), broflanilide, other meta-diamedes, steinernema carpocapsae, steinernema glaseri, pasteuria penetrans, paecilomyces tenuipes, paecilomyces fumosoroseus, beauveria bassiana, beauveria brongniartii, metarhizium anisopliae, verticillium lecanii, acynonapyr, benzpyrimoxan, flometoquin, fluhexafon, oxazosulfyl, and tyclopyrazoflor.

(32) Anthelmintic agents:

(a) Benzimidazole-based: fenbendazole, albendazole, triclabendazole, oxibendazole, mebendazole, oxfendazole, parbendazole, flubendazole, febantel, netobimin, thiophanate, thiabendazole, and cambendazole;
(b) Salicylanilide-based: closantel, oxyclozanide, rafoxanide, and niclosamide;
(c) Substituted phenol-based: nitroxinil, and nitroscanate;
(d) Pyrimidine-based: pyrantel, and morantel;
(e) Imidazothiazole-based: levamisole, and tetramisole;
(f) Tetrahydropyrimidine-based: praziquantel, and epsiprantel;
(g) Other anthelmintic agents: cyclodiene, ryania, clorsulon, metronidazole, demiditraz, piperazine, diethylcarbamazine, dichlorophen, monepantel, tribendimidine, amidantel, thiacetarsamide, melarsomine, and arsenamide.

Specific examples of plant regulatory agents which can be mixed or used with the agricultural and horticultural fungicide according to the present invention as the active ingredient (II) are shown below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinyl glycine (another name: aviglycine), aminooxyacetic acid, silver nitrate, cobalt chloride, IAA, 4-CPA, cloprop, 2,4-D, MCPB, indole-3-butyrate, dichlorprop, phenothiol, 1-naphthyl acetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl)aminobutyric acid, ethephon, chlormequat, mepiquat chloride, benzyl adenine, 5-aminolevulinic acid, and daminozide.

(Preparation formulation)

The agricultural and horticultural fungicide according to the present invention is not particularly limited by the dosage form thereof. Examples of the dosage form include wettable powder, emulsion, powder, granule, water-soluble agent, suspension, granular wettable powder, and tablet. The method for preparing the formulation is not particularly limited, and any conventional preparation method may be adopted depending on the dosage form.

Some preparation examples are shown below. The following preparation formulations are shown as examples, and may be modified to the extent that the modification is consistent with the gist of the present invention, and the present invention is not intended to be limited to the following preparation examples. The term "part" means "part by mass" unless particularly mentioned.

(Preparation Example 1: wettable powder)

40 parts of a five-membered heteroaryl compound according to the present invention, 53 parts of diatomaceous earth, 4 parts of higher alcohol sulfuric acid ester, and 3 parts of alkyl naphthalene sulfonate were mixed uniformly, and then finely pulverized to obtain wettable powders containing 40% by mass of the active ingredient.

(Preparation Example 2: emulsion)

30 parts of a five-membered heteroaryl compound according to the present invention, 33 parts of xylene, 30 parts of dimethylformamide, and 7 parts of polyoxyethylene alkyl allyl ether were mixed and dissolved to obtain an emulsion containing 30% by mass of the active ingredient.

(Preparation Example 3: granule)

5 parts of a five-membered heteroaryl compound according to the present invention, 40 parts of talc, 38 parts of clay, 10 parts of bentonite, and 7 parts of sodium alkyl sulfate were mixed uniformly, and then finely pulverized, followed by granulating to obtain a particle diameter of 0.5 to 1.0 mm, and thus granules containing 5% by mass of the active ingredient were obtained.

(Preparation Example 4: granule)

5 parts of a five-membered heteroaryl compound according to the present invention, 73 parts of clay, 20 parts of bentonite, 1 part of sodium dioctyl sulfosuccinate, and 1 part of potassium phosphate were uniformly mixed and then pulverized, followed by adding water thereto, and then kneading the mixture. Then, the resultant was granulated and dried to obtain granules containing 5% by mass of the active ingredient.

(Preparation Example 5: suspension)

10 parts of a five-membered heteroaryl compound according to the present invention, 4 parts of polyoxyethylene alkyl allyl ether, 2 parts of sodium polycarboxylate, 10 parts of glycerin, 0.2 parts of xanthan gum, and 73.8 parts of water were mixed, and then wet-pulverized until the particle size became 3 μm or less to obtain a suspension containing 10% by mass of the active ingredient.

(Preparation Example 6: granular wettable powder)

40 parts of a five-membered heteroaryl compound according to the present invention, 36 parts of clay, 10 parts of potassium chloride, 1 part of sodium alkylbenzene sulfonate, 8 parts of sodium lignin sulfonate, and 5 parts of formaldehyde condensate of sodium alkylbenzene sulfonate were mixed uniformly and then pulverized finely. Next, an appropriate amount of water was added thereto and the mixture was kneaded until the resultant became clayey. The clayey resultant was granulated and then dried to obtain granular wettable powders containing 40% by mass of the active ingredient.

Next, compound production examples are shown to explain the present invention further specifically. However, the present invention is not intended to be limited by the following examples.

(Production Example 1)

Methyl 1-(2,4-dimethylbenzyl)hydrazine-1-carboxylate (English name: methyl 1-(2,4-dimethylbenzyl)hydrazine-1-carboxylate) was produced by the following steps.

Step 1: Conversion to methyl (2,4-dimethylbenzyl)(1,3-dioxoisoindolin2-yl)carbamate (English name: methyl (2,4-dimethylbenzyl)(1,3-dioxoisoindolin-2-yl)carbamate)

[Chemical Formula 37]

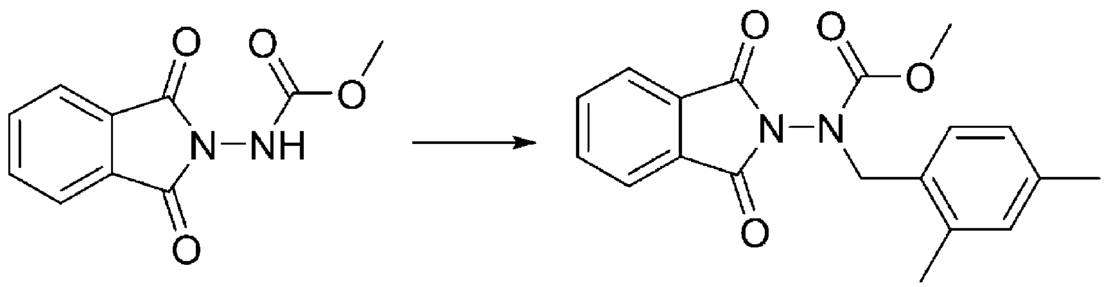

12 13

A compound 12 (0.5 g) was dissolved in acetonitrile (3 mL). 1-(Bromomethyl)-2,4-dimethylbenzene (0.54 g), potassium carbonate (0.3 g) and benzyltriethylammonium chloride (0.07 g) were added thereto at room temperature, and then stirred at 80°C for two hours. The resultant liquid was poured into ice water. The resultant was extracted with ethyl acetate, and dried over anhydrous magnesium sulfate. The solvent was distilled off under reduced pressure, the obtained residue was purified by silica gel column chromatography (developing solvent: n-hexane / ethyl acetate) to obtain a compound 13 (0.4 g, at a yield of 51%).

Step 2: Conversion to methyl 1-(2,4-dimethylbenzyl)hydrazine-1-carboxylate (English name: methyl 1-(2,4-dimethylbenzyl)hydrazine-1-carboxylate)

[Chemical Formula 38]

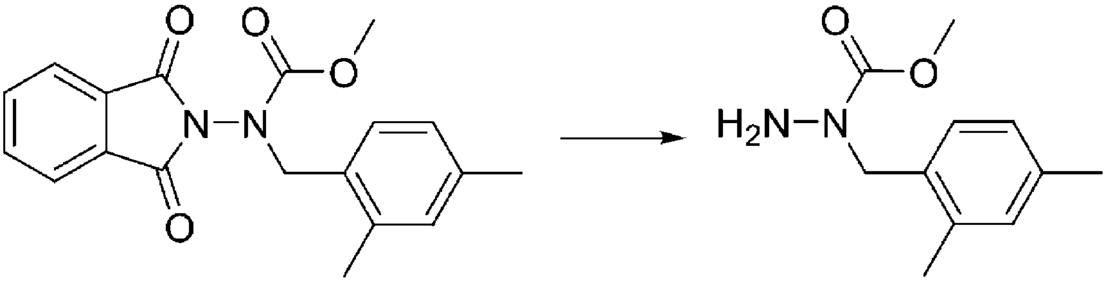

13 14

The compound 13 (0.3 g) was dissolved in ethanol (3 mL). Hydrazine monohydrate (0.22 mL) was added thereto at room temperature, and then stirred at 50°C for one hour. A solution from which a solid was filtered out was poured into ice water, extracted with ethyl acetate, and then dried over anhydrous magnesium sulfate. The solvent was distilled off under reduced pressure to obtain a compound 14 (0.17 g , at a yield of 98%).

(Production Example 2)

Methyl 1-(2,4-dimethylbenzyl)-2-(1-methyl-4-(3-(trifluoromethyl)phenoxy)-1H-1,2,3-triazole-5-carbonyl)hydrazine-1-carboxylate (English name: methyl 1-(2,4-dimethylbenzyl)-2-(1-methyl-4-(3-(trifluoromethyl)phenoxy)-1H-1,2,3-triazole-5-carbonyl)hydrazine-1-carboxylate (compound c-6)) was produced by the following steps.

Step 1: Conversion to isopropyl 4-bromo-1-methyl-1H-1,2,3-triazole-5-carboxylate (English name: isopropyl 4-bromo-1-methyl-1H-1,2,3-triazole-5-carboxylate)

[Chemical Formula 39]

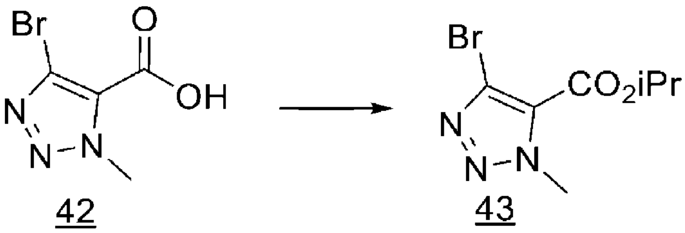

42 43

A compound 42 (0.62 g) was dissolved in N,N-dimethylformamide (6 mL). Isopropyl iodide (0.61 g) and potassium carbonate (0.54 g) were added thereto at room temperature, and then stirred at 60°C for one hour. Then, the resultant was purified by silica gel column chromatography (developing solvent: n-hexane / ethyl acetate) to obtain a compound 43 (0.58 g , at a yield of 78%).

Step 2: Conversion to isopropyl 1-methyl-4-(3-(trifluoromethyl)phenoxy)-1H-1,2,3-triazole-5-carboxylate (English name: isopropyl 1-methyl-4-(3-(trifluoromethyl)phenoxy)-1H-1,2,3-triazole-5-carboxylate)

[Chemical Formula 40]

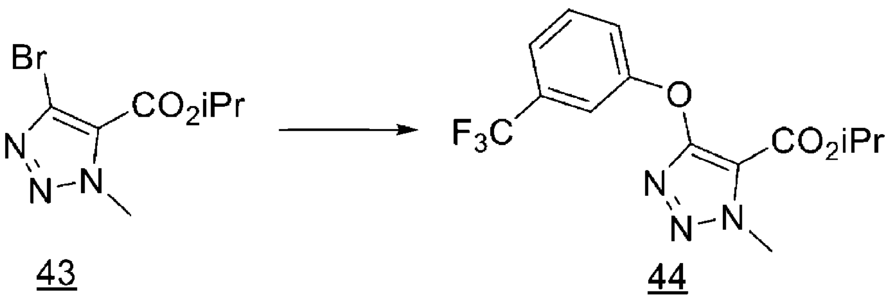

43 44

The compound 43 (0.25 g) was dissolved in toluene (4.5 mL). 3-(Trifluoromethyl)phenol (0.49 g), cesium carbonate (0.65 g), copper iodide (19 mg), and dimethylglycine (21 mg) were added thereto. The mixture was stirred with a microwave reactor at 165°C for one hour to allow the reaction to proceed. Then, the resultant was purified by silica gel column chromatography (developing solvent: n-hexane / ethyl acetate) to obtain a compound 44 (0.14 g , at a yield of 43%).

Step 3: Conversion to 1-methyl-4-(3-(trifluoromethyl)phenoxy)-1H-1,2,3-triazole-5-carboxylic acid (English name: 1-methyl-4-(3-(trifluoromethyl)phenoxy)-1H-1,2,3-triazole-5-carboxylic acid)

[Chemical Formula 41]

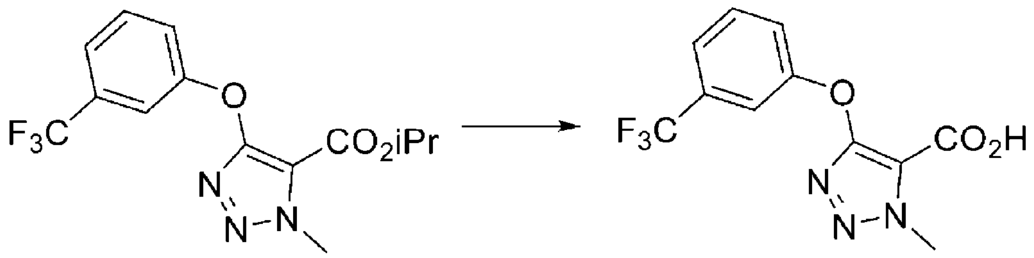

44 45

The compound 44(0.14 g) was dissolved in tetrahydrofuran (3 mL) and water (1 mL). Lithium hydroxide monohydrate (0.09 g) was added thereto at room temperature, and then stirred at room temperature for 18 hours. Dilute hydrochloric acid was added to the resultant liquid, and the mixture was extracted with ethyl acetate, followed by washing the extracted phase with saturated saline. Then, the resultant was dried over anhydrous magnesium sulfate, and the solvent was distilled off under reduced pressure to obtain a compound 45 (0.12 g , at a yield of 97%).

Step 4: Conversion to methyl 1-(2,4-dimethylbenzyl)-2-(1-methyl-4-(3-(trifluoromethyl)phenoxy)-1H-1,2,3-triazole-5-carbonyl)hydrazine-1-carboxylate (English name: methyl 1-(2,4-dimethylbenzyl)-2-(1-methyl-4-(3-(trifluoromethyl)phenoxy)-1H-1,2,3-triazole-5-carbonyl)hydrazine-1-carboxylate)

[Chemical Formula 42]

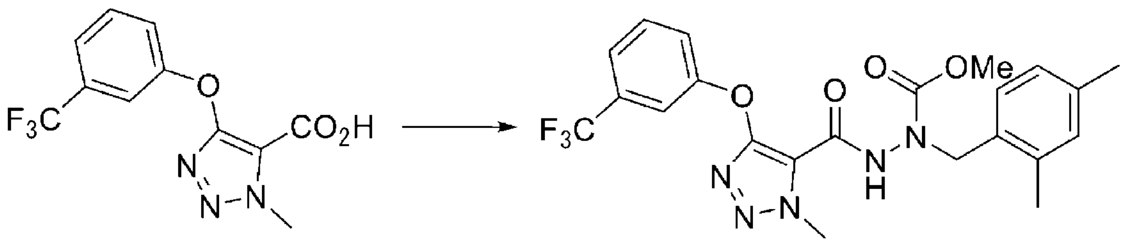

45 c-6

The compound 45 (0.03 g) was dissolved in dichloromethane (1 mL). The compound 14 (23 mg), 1-ethyl-3-(3-dimethylaminopropyl)carbodiimide hydrochloride (29 mg), 1-hydroxybenzotriazole (1 mg) and triethylamine (0.034 mL) were added thereto, and then stirred at room temperature for 18 hours. The resultant liquid was poured into ice water, extracted with ethyl acetate, and then dried over anhydrous magnesium sulfate. The solvent was distilled off under reduced pressure. The obtained residue was purified by silica gel column chromatography (developing solvent: n-hexane / ethyl acetate) and preparative HPLC (developing solvent: water /acetonitrile) to obtain a compound c-6 (6mg, at a yield of 13%).

(Production Example 3)

Methyl 1-(2,4-dimethylbenzyl)-2-(1-methyl-4-((4-(trifluoromethyl)pyridin-2-yl)oxy)-1H-pyrazole-5-carbonyl)hydrazine-1-carboxylate (English name: methyl 1-(2,4-dimethylbenzyl)-2-(1 -methyl-4-((4-(trifluoromethyl)pyridin-2-yl)oxy)-1H-pyrazole-5-carbonyl)hydrazine-1-carboxylate (compound c-12)) was produced by the following steps.

Step 1: Conversion to tert-butyl 4-iodo-1-methyl-1H-pyrazole-5-carboxylate (English name: tert-butyl 4-iodo-1-methyl-1H-pyrazole-5-carboxylate)

[Chemical Formula 43]

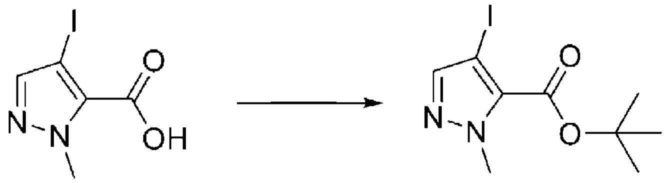

46 47

A compound 46 (20 g) was dissolved in tetrahydrofuran (265 mL). 4-Dimethylaminopyridine (2.9 g) and di-tert-butyl dicarbonate (26 g) were added thereto, and then stirred at 40°C for seven hours. Dilute hydrochloric acid was added to the resultant liquid, and then the mixture was extracted with ethyl acetate. The extracted phase was washed with saline solution. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 47 (22.05 g , at a yield of 90%).

Step 2: Conversion to tert-butyl 1-methyl-4-(4,4,5,5-tetramethyl-1,3,2-dioxaborolan-2-yl)-1H-pyrazole-5-carboxylate (English name: tert-butyl 1-methyl-4-(4,4,5,5-tetramethyl-1,3,2-dioxaborolan-2-yl)-1H-pyrazole-5-carboxylate)

[Chemical Formula 44]

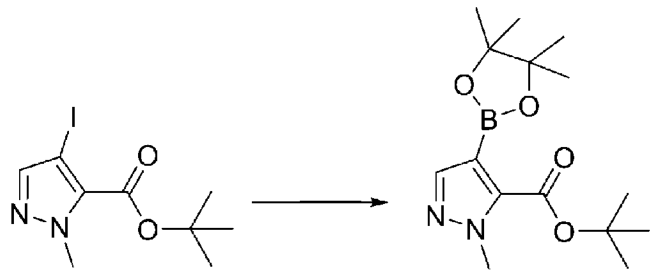

47

48

The compound 47 (21.57 g) was dissolved in dimethyl sulfoxide (350 mL). Bis(pinacolato)diboron (35.6 g), [1,1'-bis(diphenylphosphino)ferrocene] dichloropalladium(II) dichloromethane complex (2.9 g), and potassium acetate (20.6 g) were added thereto, and then stirred at 70°C for seven hours under a nitrogen atmosphere. Water was added to the resultant liquid, and the mixture was extracted with ethyl acetate. The extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 48 (18.99 g , at a yield of 88%).

Step 3: Conversion to tert-butyl 4-hydroxyl-methyl-1H-pyrazole-5-carboxylate (English name: tert-butyl 4-hydroxy-1-methyl-1H-pyrazole-5-carboxylate)

[Chemical Formula 45]

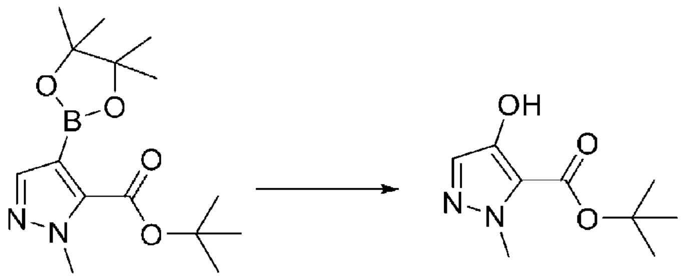

48

49

The compound 48 (18.99 g) was dissolved in a mixture solvent composed of tetrahydrofuran (123 mL) and water (123 mL). Sodium hydroxide (4.9 g) was added thereto, and then stirred while cooling with ice. 30% Hydrogen peroxide (14 g) was added thereto, and then stirred at room temperature for one hour. Dilute hydrochloric acid was added to the resultant liquid, and the mixture was extracted with ethyl acetate. The extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 49 (9.43 g , at a yield of 77%).

Step 4: Conversion to tert-butyl 1-methyl-4-((4-(trifluoromethyl)pyridin-2-yl)oxy))-1H-pyrazole-5-carboxylate (English name: tert-butyl 1-methyl-4-((4-(trifluoromethyl)pyridin-2-yl)oxy)-1H-pyrazole-5-carboxylate)

[Chemical Formula 46]

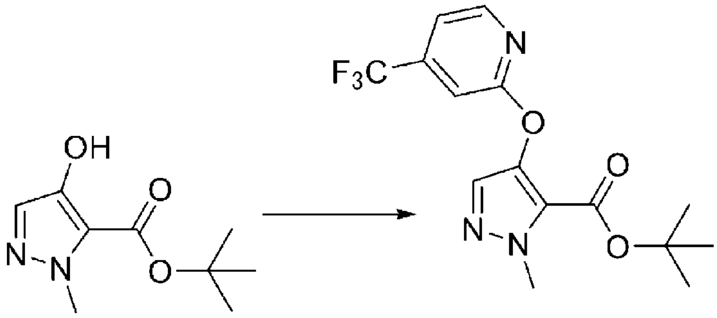

49 50

The compound 49 (0.396 g) was dissolved in N,N-dimethylformamide (10 mL). 2-Chloro4-(trifluoromethyl) pyridine (0.73 g) and potassium carbonate (0.55 g) were added thereto, and then stirred at 80°C for seven hours. Water was added to the resultant liquid, and the mixture was extracted with ethyl acetate. The extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 50 (0.75g, at a yield of 100%) quantitatively.

Step 5: Conversion to 1-methyl-4-((4-(trifluoromethyl)pyridin-2-yl)oxy)-1H-pyrazole-5-carboxylic acid (English name: 1-methyl-4-((4-(trifluoromethyl)pyridin-2-yl)oxy)-1H-pyrazole-5-carboxylic acid)

[Chemical Formula 47]

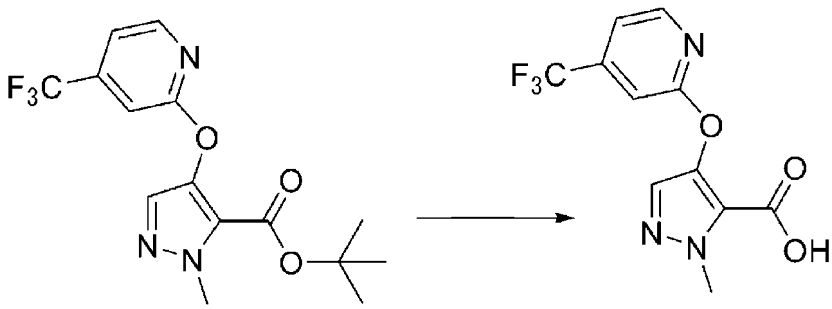

50 51

The compound 50 (0.75 g) was dissolved in dichloromethane (10 mL). Trifluoroacetic acid (2 mL) was added thereto, and the mixture was stirred at room temperature overnight. The resultant liquid was concentrated to obtain a compound 51 (0.74 g, at a yield of 100%).

Step 6: Conversion to methyl 1-(2,4-dimethylbenzyl)-2-(1-methyl-4-((4-(trifluoromethyl)pyridin-2-yl)oxy)-1H-pyrazole-5-carbonyl)hydrazine-1-carboxylate (English name: methyl 1-(2,4-dimethylbenzyl)-2-(1-methyl-4-((4-(trifluoromethyl)pyridin-2-yl)oxy)-1H-pyrazole-5-carbonyl)hydrazine-1-carboxylate)

[Chemical Formula 48]

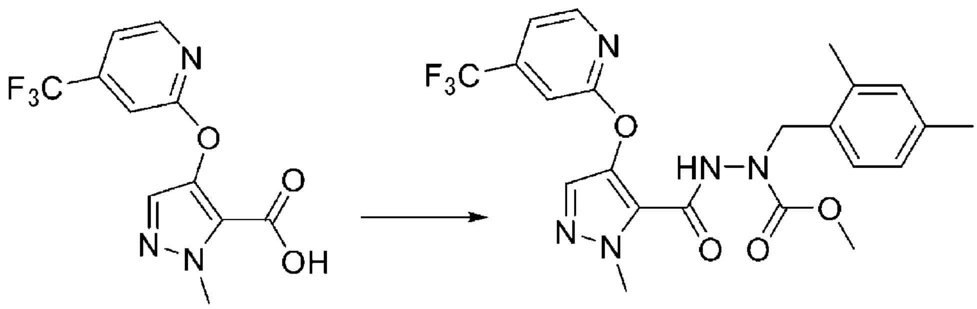

51 c-12

The compound 51 (0.1 g) was dissolved in N,N-dimethylformamide (1 mL). The compound 14 (0.1 g), 1-[bis(dimethylamino)methylene]-1H-1,2,3-triazolo[4,5-b]pyridinium 3-oxide hexafluorophosphate (HATU) (0.26 g) and N,N-diisopropylethylamine (0.09 g) were added thereto, and stirred at room temperature for one hour. The resultant liquid was purified with preparative HPLC (developing solvent: water / acetonitrile) to obtain a compound c-12 (0.093g, at a yield of 55%).

(Production Example 4)

Methyl 2-(5-(3-bromo-2-fluorophenoxy)-1-methyl-1H-pyrazole-4-carbonyl)-1-(2,4-dimethylbenzyl)hydrazine-1-carboxylate (English name: methyl 2-(5-(3-bromo-2-fluorophenoxy)-1-methyl-1H-pyrazole-4-carbonyl)-1-(2,4-dimethylbenzyl)hydrazine-1-carboxylate (compound c-19)) was produced by the following steps.

Step 1: Conversion to ethyl 5-(3-bromo-2-fluorophenoxy)-1-methyl-1H-pyrazole-4-carboxylate (English name: ethyl 5-(3-bromo-2-fluorophenoxy)-1-methyl-1H-pyrazole-4-carboxylate)

[Chemical Formula 49]

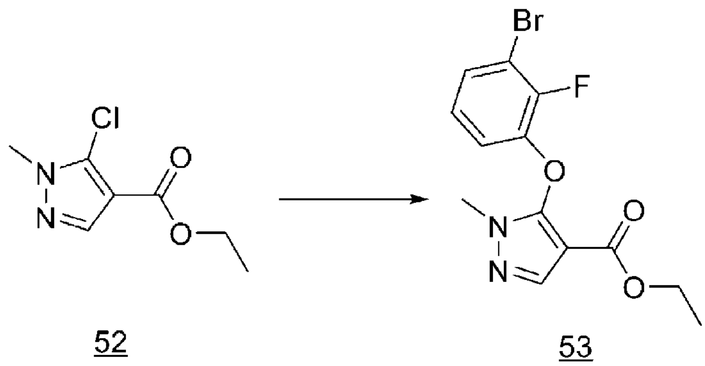

52 53

A compound 52 (0.17 g) was dissolved in N,N-dimethylformamide (3 mL). 3-Bromo 2-fluorophenol (0.181 g) and cesium carbonate (0.62 g) were added thereto, and stirred at 130°C overnight. Water was added to the resultant liquid, and the mixture was extracted with ethyl acetate. The extracted phase was washed with saline solution. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 53 (0.11 g , at a yield of 51%).

Step 2: Conversion to 5-(3-bromo-2-fluorophenoxy)-1-methyl-1H-pyrazole-4-carboxylic acid (English name: 5-(3-bromo-2-fluorophenoxy)-1-methyl-1H-pyrazole-4-carboxylic acid)

[Chemical Formula 50]

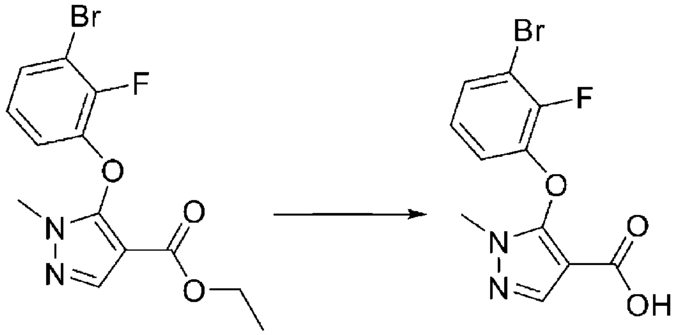

53 54

The compound 53 (0.11 g) was dissolved in a mixture solvent composed of THF (3 mL), methanol (1 mL) and water (1 mL). Lithium hydroxide monohydrate (0.054 g) was added thereto, and stirred at room temperature overnight. Dilute hydrochloric acid was added to the resultant liquid, and the mixture was extracted with ethyl acetate. The extracted phase was washed with saturated saline. Then, the solvent was distilled off under reduced pressure to obtain a compound 54 (0.1 g, at a yield of 100%).

Step 3: Conversion to methyl 2-(5-(3-bromo-2-fluorophenoxy)-1-methyl-1H-pyrazole-4-carbonyl)-1-(2,4-dimethylbenzyl)hydrazine-1-carboxylate (English name: methyl 2-(5-(3-bromo-2-fluorophenoxy)-1-methyl-1H-pyrazole-4-carbonyl)-1-(2,4-dimethylbenzyl)hydrazine-1-carboxylate)

[Chemical Formula 51]

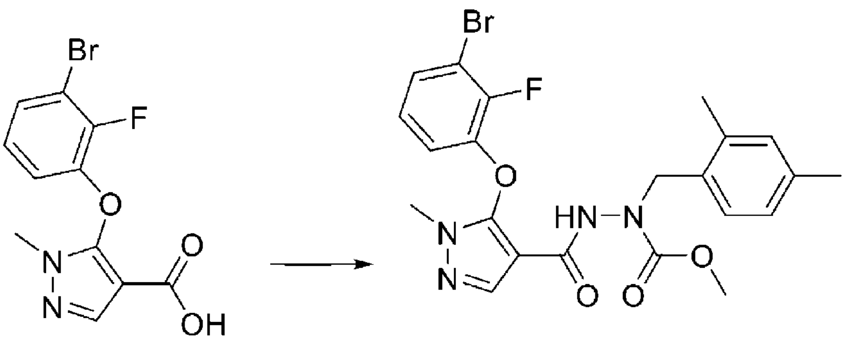

54 c-19

The compound 54 (0.1 g) was dissolved in N,N-dimethylformamide (1 mL). The compound 14 (0.1 g), 1-ethyl-3-(3-dimethylaminopropyl)carbodiimide hydrochloride (0.09 g), 1-hydroxybenzotriazole (0.07 g) and triethylamine (0.06 g) were added thereto, and stirred at room temperature overnight. The resultant liquid was purified by preparative HPLC (developing solvent: water /acetonitrile) to obtain a compound c-19 (0.061g, at a yield of 40%).

(Production Example 5)

Methyl 2-(4-(3-bromo-2-fluorophenoxy)-1-methyl-1H-pyrazole-5-carbonyl)-1-(2,4-dimethylbenzyl)hydrazine-1-carboxylate (English name: methyl 2-(4-(3-bromo-2-fluorophenoxy)-1-methyl-1H-pyrazole-5-carbonyl)-1-(2,4-dimethylbenzyl)hydrazine-1-carboxylate (compound c-13)) was produced by the following steps.

Step 1: Conversion to 3-(3-bromo-2-fluorophenoxy)-4-(dimethylamino)but-3-en-2-one (English name: 3-(3-bromo-2-fluorophenoxy)-4-(dimethylamino)but-3-en-2-one)

[Chemical Formula 52]

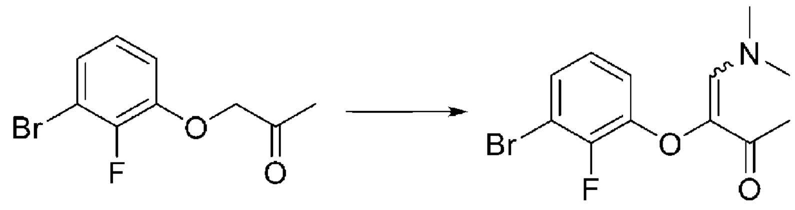

55 56

A compound 55 (10.93 g) was dissolved in N,N-dimethylformamide dimethyl acetal (12 mL), and stirred at 100°C for two hours. The resultant liquid was distilled off under reduced pressure, and the resultant was purified by silica gel column chromatography (developing solvent: n-hexane / ethyl acetate) to obtain a compound 56 (7.38 g , at a yield of 58%).

Step 2: Conversion to 4-(3-bromo-2-fluorophenoxy)-5-methyl-1H-pyrazole (English name: 4-(3-bromo-2-fluorophenoxy)-5-methyl-1H-pyrazole)

[Chemical Formula 53]

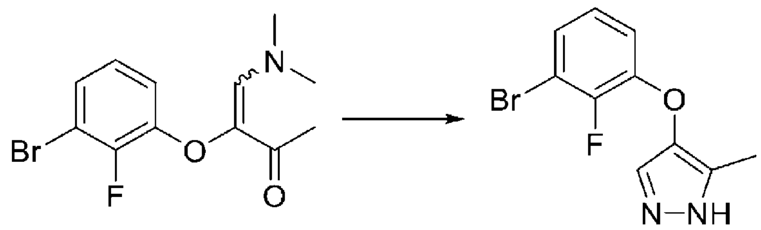

56 57

The compound 56 (7.78 g) was dissolved in methanol (30 mL). Hydrazine hydrate (3 mL) was added thereto, and stirred at 80°C for five hours. A saturated sodium bicarbonate solution was added to the resultant liquid, and the mixture was extracted with ethyl acetate. The extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain a compound 57 (2.34 g , at a yield of 33%).

Step 3: Conversion to 4-(3-bromo-2-fluorophenoxy)-1-methyl-1H-pyrazole-5-carboxylic acid (English name: 4-(3-bromo-2-fluorophenoxy)-1-methyl-1H-pyrazole-5-carboxylic acid)

[Chemical Formula 54]

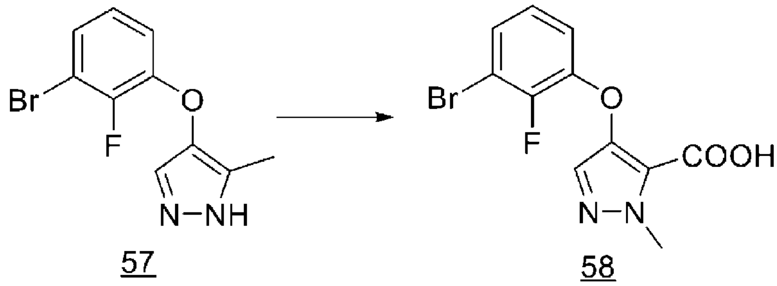

57 58

The compound 57 (2.07 g) was dissolved in N,N-dimethylformamide (23 mL). Potassium carbonate (2.1 g) and methyl iodide (1.4 g) were added thereto, and stirred at room temperature overnight. Water was added to the resultant liquid, and the mixture was extracted with ethyl acetate. The extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain a crude product.

The obtained crude product was dissolved in a mixture solvent composed of tert-butanol (17 mL) and water (8 mL). Potassium hydroxide (1.2 g) and potassium permanganate (2.2 g) were added to the resultant while stirring the resultant at room temperature, and then the resultant was heated to reflux overnight. The resultant liquid was filtered through Celite (registered trademark), and dilute hydrochloric acid was added to the filtrate, followed by subjecting the resultant to an extraction with ethyl acetate. The extracted phase was washed with saturated saline. Then, the resultant was dried over anhydrous magnesium sulfate, the solvent was distilled off under reduced pressure, and the resultant was purified by preparative HPLC(developing solvent: water / acetonitrile) to obtain a compound 58 (0.47 g , at a yield of 20%).

Step 4: Conversion to methyl 2-(4-(3-bromo-2-fluorophenoxy)-1-methyl-1H-pyrazole-5-carbonyl)-1-(2,4-dimethylbenzyl)hydrazine-1-carboxylate (English name: methyl 2-(4-(3-bromo-2-fluorophenoxy)-1-methyl-1H-pyrazole-5-carbonyl)-1-(2,4-dimethylbenzyl)hydrazine-1-carboxylate)

[Chemical Formula 55]

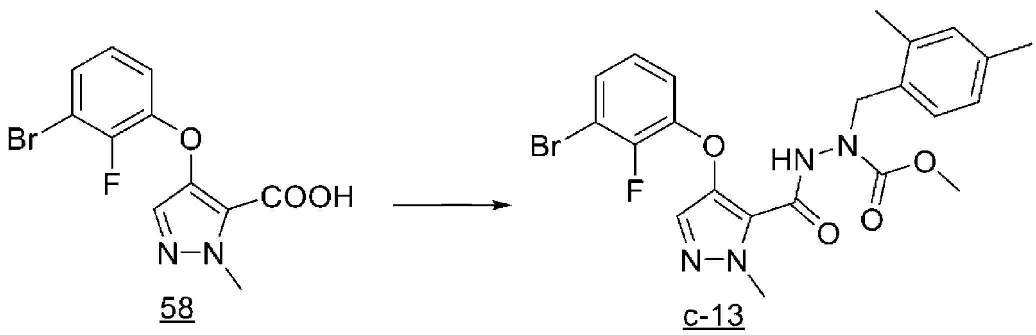

58 c-13

The compound 58 (0.03 g) was dissolved in N,N-dimethylformamide (1 mL). The compound 14 (0.03 g), 1-[bis(dimethylamino)methylene]-1H-1,2,3-triazolo[4,5-b]pyridinium-3-oxide hexafluorophosphate (HATU) (0.055 g) and N,N-diisopropylethylamine (0.025 g) were added thereto, and stirred at room temperature overnight. The resultant liquid was purified by preparative HPLC (developing solvent: water / acetonitrile) to obtain a compound c-13 (0.011g, at a yield of 22%).

(Production Example 6)

Methyl 2-(4-(3-bromophenoxy)-1-methyl-1H-pyrazole-5-carbonyl)-1-(2,4-dimethylbenzyl)hydrazine-1-carboxylate (English name: methyl 2-(4-(3-bromophenoxy)-1-methyl-1H-pyrazole-5-carbonyl)-1-(2,4-dimethylbenzyl)hydrazine-1-carboxylate (compound c-1)) was produced by the following steps.

Step 1: Conversion to methyl 4-(3-bromophenoxy)-1-methyl-1H-pyrazole-5-carboxylate (English name: methyl 4-(3-bromophenoxy)-1-methyl-1H-pyrazole-5-carboxylate)

[Chemical Formula 56]

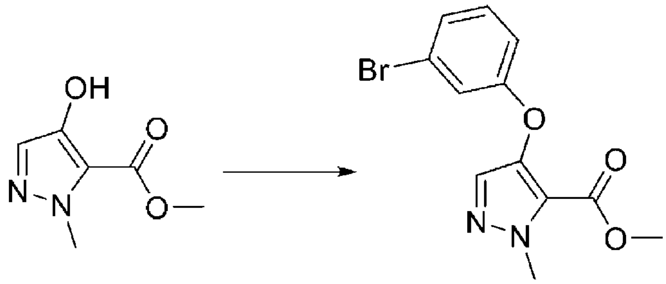

59 60

A compound 59 (0.154 g) was dissolved in dichloromethane (5 mL). 3-Bromophenylboronic acid (0.29 g), copper acetate monohydrate (0.29 g) and pyridine (2.0 g) were added thereto, and stirred at room temperature for two days. The solvent was distilled off from the resultant liquid under reduced pressure. The obtained residue was purified by silica gel column chromatography (developing solvent: n-hexane / ethyl acetate) to obtain a compound 60 (0.198 g , at a yield of 65%).

Step 2: Conversion to 4-(3-bromophenoxy)-1-methyl-1H-pyrazole-5-carboxylic acid (English name:4-(3-bromophenoxy)-1-methyl-1H-pyrazole-5-carboxylic acid)

[Chemical Formula 57]

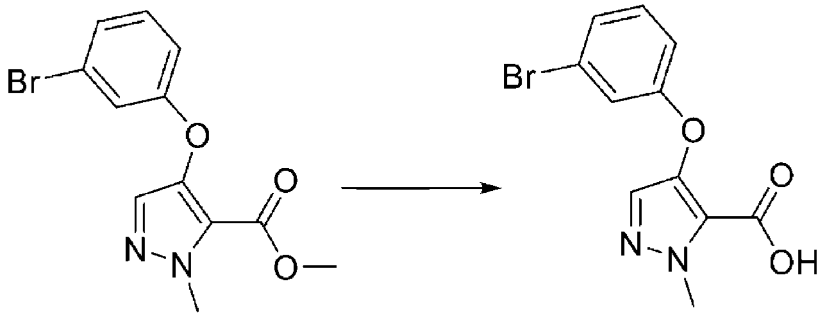

60 61

The compound 60 (0.198 g) was dissolved in a mixture solvent composed of tetrahydrofuran (2 mL), methanol (0.6 mL) and water (0.6 mL). Lithium hydroxide monohydrate (0.14 g) was added thereto, and stirred at room temperature overnight. Dilute hydrochloric acid was added to the resultant liquid, and the mixture was extracted with ethyl acetate. The extracted phase was washed with saturated saline. Then, the resultant was dried over anhydrous magnesium sulfate, and the solvent was distilled off under reduced pressure to obtain a compound 61 (0.185 g , at a yield of 98%).

Step 3: Conversion to methyl 2-(4-(3-bromophenoxy)-1-methyl-1H-pyrazole-5-carbonyl)-1-(2,4-dimethylbenzyl)hydrazine-1-carboxylate (English name: methyl 2-(4-(3-bromophenoxy)-1-methyl-1H-pyrazole-5-carbonyl)-1-(2,4-dimethylbenzyl)hydrazine-1-carboxylate)

[Chemical Formula 58]

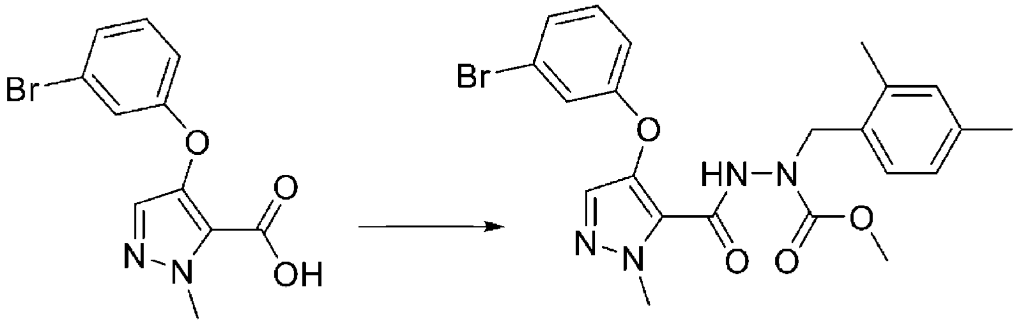

61 c-1

The compound 61(0.05 g) was dissolved in N,N-dimethylformamide (3 mL). The compound 14 (0.052 g), 1-[bis(dimethylamino)methylene]-1H-1,2,3-triazolo[4,5-b]pyridinium-3-oxide hexafluorophosphate (HATU) (0.138 g) and N,N-diisopropylethylamine (0.043 g) were added thereto, and stirred at room temperature overnight. The resultant liquid was purified by preparative HPLC (developing solvent: water /acetonitrile) to obtain a compound c-1 (0.033g, at a yield of 43%).

(Production Example 7)

Methyl 2-(4-(3-bromophenoxy)-3-chlorol -methyl-1H-pyrazole-5-carbonyl)-1-(2,4-dimethylbenzyl)hydrazine-1-carboxylate (English name: methyl 2-(4-(3-bromophenoxy)-3-chloro-1-methyl-1H-pyrazole-5-carbonyl)-1-(2,4-dimethylbenzyl)hydrazine-1-carboxylate) (compound c-35) was produced by the following steps.

Step 1: Conversion to tert-butyl 3-chloro4-hydroxyl-methyl-1H-pyrazole-5-carboxylate (English name: tert-butyl 3-chloro-4-hydroxy-1-methyl-1H-pyrazole-5-carboxylate)

[Chemical Formula 59]

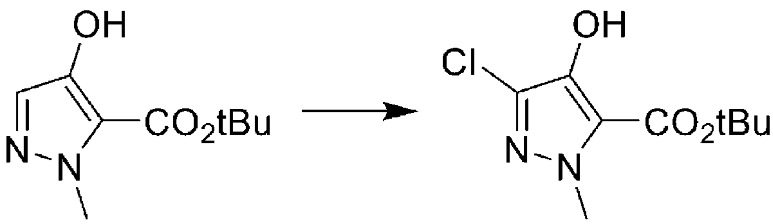

Tert-butyl-4-hydroxyl-methyl-1H-pyrazole-5-carboxylate (0.50 g) was dissolved in N,N-dimethylformamide (8.4 mL). N-chlorosuccinimide (0.40 g) was added thereto and stirred at room temperature for 16 hours.

Then, the reaction liquid was directly purified by silica gel column chromatography (developing solvent: n-hexane / ethyl acetate) to obtain 0.43 g of tert-butyl 3-chloro-4-hydroxyl-methyl-1H-pyrazole-5-carboxylate (at a yield of 73%).

Step 2: Conversion to 3-chloro-4-hydroxy-1-methyl-1H-pyrazole-5-carboxylic acid (English name: 3-chloro-4-hydroxy-1-methyl-1H-pyrazole-5-carboxylic acid)

[Chemical Formula 60]

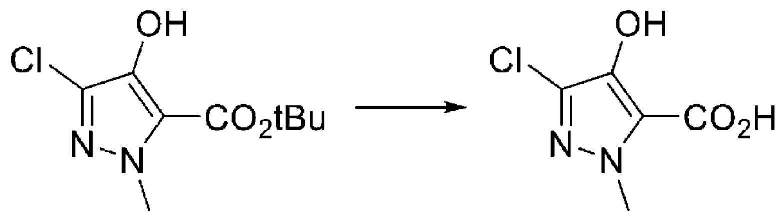

Tert-butyl 3-chloro-4-hydroxy-1-methyl-1H-pyrazole-5-carboxylate (0.43 g) was dissolved in dichloromethane (6.2 mL). Trifluoroacetic acid (2 mL) were added thereto, and stirred at room temperature for 16 hours.

The reaction liquid was concentrated with an evaporator to obtain 0.33g of 3-chloro-4-hydroxy-1-methyl-1H-pyrazole-5-carboxylic acid (at a yield of 100%).

Step 3: Conversion to methyl 2-(3-chloro-4-hydroxy-1-methyl-1H-pyrazole-5-carbonyl)-1-(2,4-dimethylbenzyl)hydrazine-1-carboxylate (English name: methyl 2-(3-chloro-4-hydroxy-1-methyl-1H-pyrazole-5-carbonyl)-1-(2,4-dimethylbenzyl)hydrazine-1-carboxylate)

[Chemical Formula 61]

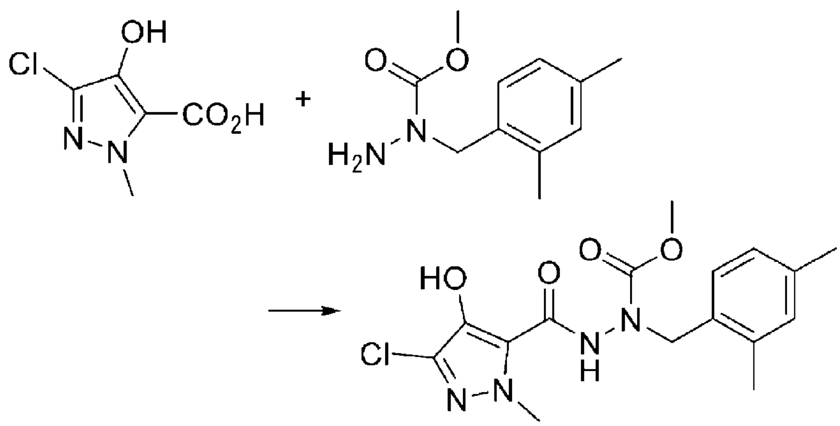

3-Chloro-4-hydroxy-1-methyl-1H-pyrazole-5-carboxylic acid (0.33 g) was dissolved in N,N-dimethylformamide (6.2 mL). Methyl 1-(2,4-dimethylbenzyl)hydrazine-1-carboxylate (0.85 g), 1-[bis(dimethylamino)methylene]-1H-1,2,3-triazolo[4,5-b]pyridinium-3-oxide hexafluorophosphate (HATU) (1.7 g), and triethylamine (0.94 g) were added thereto, and stirred at 60°C for four hours.

After filtration, the resultant was purified by preparative liquid chromatography (developing solvent: water / acetonitrile) to obtain 0.31g of methyl 2-(3-chloro-4-hydroxy-1-methyl-1H-pyrazole-5-carbonyl)-1-(2,4-dimethylbenzyl) hydrazine-1-carboxylate (at a yield of 45%).

Step 4: Conversion to methyl 2-(4-(3-bromophenoxy)-3-chlorol-methyl-1H-pyrazole-5-carbonyl)-1-(2,4-dimethylbenzyl)hydrazine-1-carboxylate (English name: methyl 2-(4-(3-bromophenoxy)-3-chloro-1-methyl-1H-pyrazole-5-carbonyl)-1-(2,4-dimethylbenzyl)hydrazine-1-carboxylate)

[Chemical Formula 62]

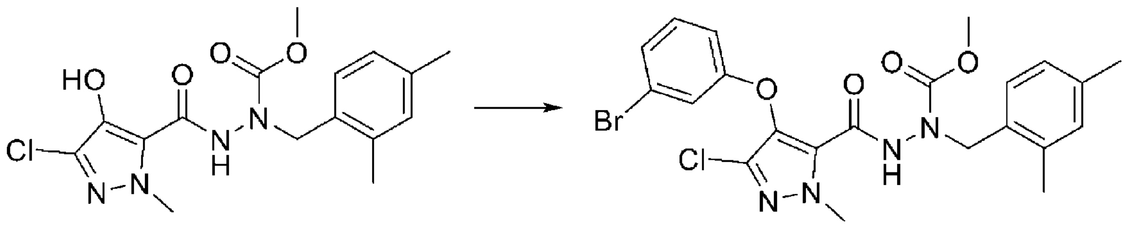

Methyl 2-(3-chloro-4-hydroxy-1-methyl-1H-pyrazole-5-carbonyl)-1-(2,4-dimethylbenzyl)hydrazine-1-carboxylate (0.10 g) was dissolved in dichloromethane (0.91 mL). Copper acetate (0.074 g), pyridine (0.043 g), and (3-bromophenyl)boronic acid (0.22 g) were added thereto.

After the mixture was stirred at room temperature for 16 hours, the reaction solvent was concentrated with an evaporator. The resultant was purified by silica gel column chromatography (developing solvent: n-hexane / ethyl acetate) to obtain 0.007 g of methyl 2-(4-(3-bromophenoxy)-3-chlorol-methyl-1H-pyrazole-5-carbonyl)-1-(2,4-dimethylbenzyl) hydrazine-1-carboxylate (at a yield of 5%).

(Production Example 8)

Methyl 2-(4-(3-bromophenoxy)-1-(tert-butyl)-1H-pyrazole-5-carbonyl)-1-(2,4-dimethylbenzyl)hydrazine-1-carboxylate (English name: methyl 2-(4-(3-bromophenoxy)-1-(tert-butyl)-1H-pyrazole-5-carbonyl)-1-(2,4-dimethylbenzyl) hydrazine-1-carboxylate) (compound c-114)was produced by the following steps.

Step 1: Conversion to 3-(3-bromophenoxy)-4-(dimethylamino)but-3-en-2-one (English name: 3-(3-bromophenoxy)-4-(dimethylamino)but-3-en-2-one)

[Chemical Formula 63]

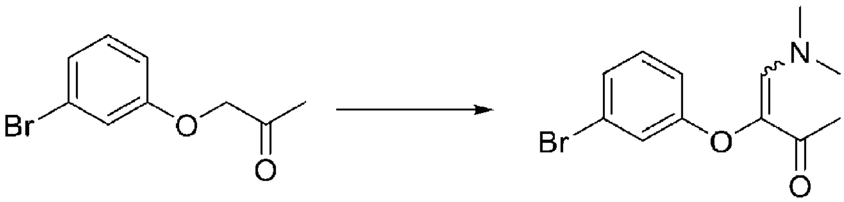

1-(3-Bromophenoxy)propan-2-one (38.35 g) was dissolved in N,N-dimethylformamide dimethyl acetal (33.8 mL), and stirred at 80°C overnight.

The reaction liquid was distilled off under reduced pressure, and then the resultant was purified by silica gel column chromatography (developing solvent: n-hexane / ethyl acetate), followed by washing the obtained purified product with diethyl ether and hexane to obtain 28 g of the target compound (at a yield of 58%).

Step 2: Conversion to 4-(3-bromophenoxy)-1-(tert-butyl)-5-methyl-1H-pyrazole (English name: 4-(3-bromophenoxy)-1-(tert-butyl)-5-methyl-1H-pyrazol)

[Chemical Formula 64]

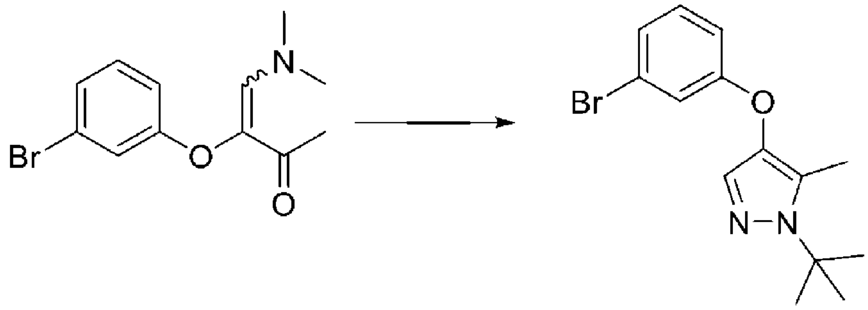

3-(3-Bromophenoxy)-4-(dimethylamino)but-3-en-2-one (17 g) was dissolved in 1,4-dioxane (300 mL). Tert-butylhydrazine hydrochloride (8.9 g) and concentrated hydrochloric acid (5.5 mL) were added thereto, and stirred at 60°C for three hours.

A saturated sodium bicarbonate solution was added to the reaction liquid, the mixture was extracted with ethyl acetate, and the extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain 14.61 g of the target compound (at a yield of 79%).

Step 3: Conversion to 4-(3-bromophenoxy)-1-(tert-butyl)-1H-pyrazole-5-carboxylic acid (English name: 4-(3-bromophenoxy)-1-(tert-butyl)-1H-pyrazole-5-carboxylic acid)

[Chemical Formula 65]

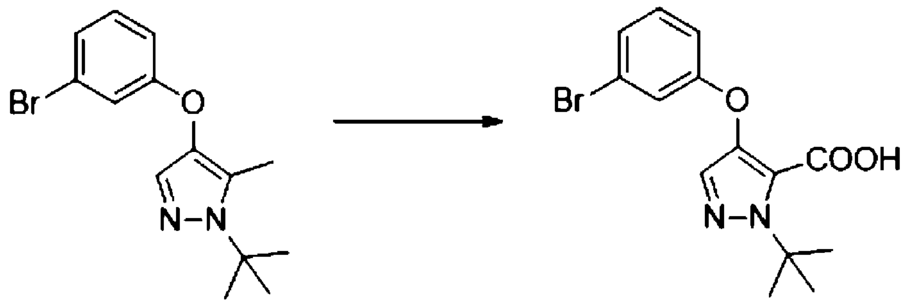

4-(3-Bromophenoxy)-1-(tert-butyl)-5-methyl-1H-pyrazole (14.61 g) was dissolved in a mixture solvent composed of tert-butanol (105 mL) and water (52.5 mL). Potassium hydroxide (5.3 g) and potassium permanganate (22.4 g) were added thereto while carrying out stirring at room temperature, and then the mixture was stirred at 100°C for five hours.

The reaction liquid was filtered through Celite (registered trademark), dilute hydrochloric acid was added thereto, and the mixture was extracted with dichloromethane, followed by washing the extracted phase with saturated saline. Then, the resultant was dried over anhydrous magnesium sulfate, and the solvent was distilled off under reduced pressure to obtain 9.56 g of the target compound (at a yield of 60%).

Step 5: Conversion to methyl 2-(4-(3-bromophenoxy)-1-(tert-butyl)-1H-pyrazole-5-carbonyl)-1-(2,4-dimethylbenzyl)hydrazine-1-carboxylate (English name: methyl 2-(4-(3-bromophenoxy)-1-(tert-butyl)-1H-pyrazole-5-carbonyl)-1-(2,4-dimethylbenzyl)hydrazine-1-carboxylate)

[Chemical Formula 66]

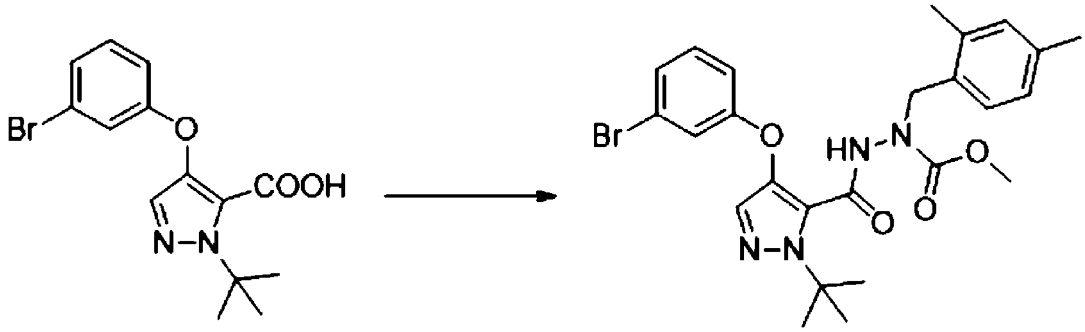

4-(3-Bromophenoxy)-1-(tert-butyl)-1H-pyrazole-5-carboxylic acid (0.05 g) was dissolved in tetrahydrofuran (1 mL). Methyl 1-(2,4-dimethylbenzyl)hydrazine-1-carboxylate (0.046 g), 1-[bis(dimethylamino)methylene]-1H-1,2,3-triazolo[4,5-b]pyridinium-3-oxide hexafluorophosphate (HATU) (0.11 g) and triethylamine (0.05 g) were added thereto, and stirred at room temperature overnight.

The reaction liquid was distilled off under reduced pressure, and then the resultant was purified by silica gel column chromatography (developing solvent: n-hexane / ethyl acetate) to obtain 0.06 g of the target product (at a yield of 80%).

(Production Example 9)

Methyl 2-(1-(tert-butyl)-4-(3-(trifluoromethyl)phenoxy)-1H-1,2,3-triazole-5-carbonyl)-1-(2,4-dimethylbenzyl)hydrazine-1-carboxylate (English name: methyl 2-(1-(tert-butyl)-4-(3-(trifluoromethyl)phenoxy)-1H-1,2,3-triazole-5-carbonyl)-1-(2,4-dimethylbenzyl)hydrazine-1-carboxylate) (compound c-97) was produced by the following steps.

Step 1: Conversion to 4,5-dibromo-1-(tert-butyl)-1H-1,2,3-triazole (English name: 4,5-dibromo-1-(tert-butyl)-1H-1,2,3-triazole)

[Chemical Formula 67]

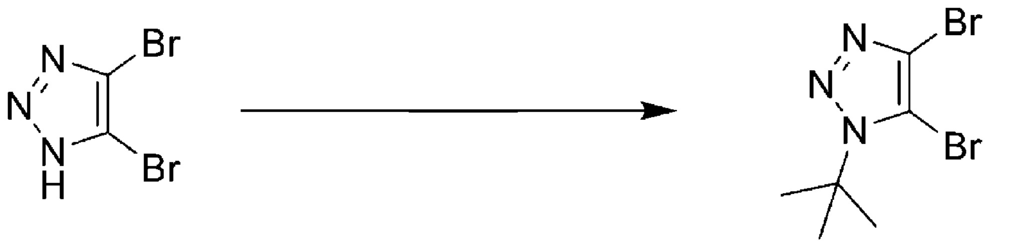

4,5-Dibromo-1H-1,2,3-triazole (3.4 g), tert-butanol (2.2 g), and p-toluenesulfonic acid monohydrate (0.57 g) were added to toluene (50 mL), and stirred at 70°C for three hours.

The solvent was distilled off, and then the resultant was purified by silica gel column chromatography (developing solvent: n-hexane / ethyl acetate) to obtain 4,5-dibromo-1-(tert-butyl)-1H-1,2,3-triazole (1.8 g).

Step 2: Conversion to 4-bromo-1-(tert-butyl)-1H-1,2,3-triazole-5-carboxylic acid (English name: 4-bromo-1-(tert-butyl)-1H-1,2,3-triazole-5-carboxylic acid)

[Chemical Formula 68]

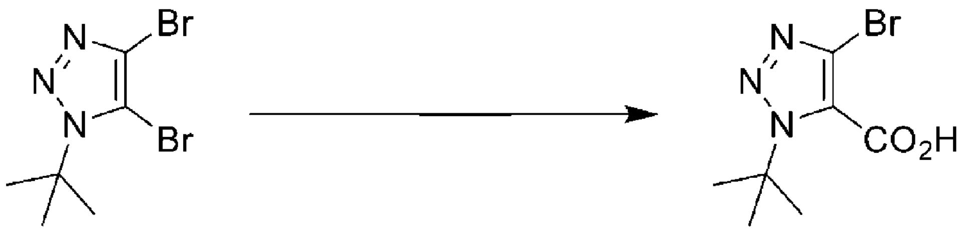

4,5-Dibromo-1-(tert-butyl)-1H-1,2,3-triazole (1.7 g) was dissolved in tetrahydrofuran (15 mL) at 0°C, and a complex of isopropylmagnesium chloride and lithium chloride (1.4 g) was added thereto, and then stirred for two hours. Next, carbon dioxide (10 g) was bubbled.

After the reaction was quenched with 1M HCl, the resultant was extracted with ethyl acetat, and the solvent was distilled off to obtain 4-bromo-1-(tert-butyl)-1H-1,2,3-triazole-5-carboxylic acid (1.5 g).

Step 3: Conversion to isopropyl 4-bromo-1-(tert-butyl)-1H-1,2,3-triazole-5-carboxylate (English name: isopropyl 4-bromo-1-(tert-butyl)-1H-1,2,3-triazole-5-carboxylate)

[Chemical Formula 69]

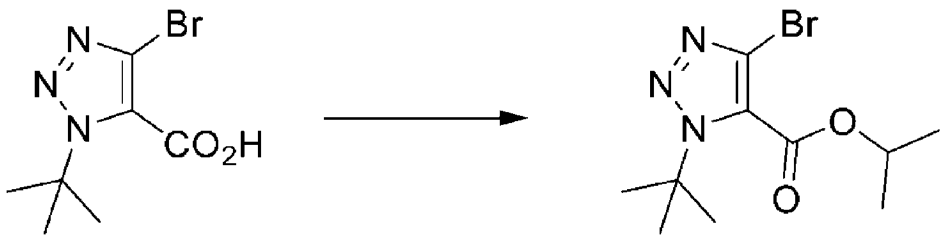

4-Bromo-1-(tert-butyl)-1H-1,2,3-triazole-5-carboxylic acid (1.5 g), isopropyl iodide (3.1 g), and potassium carbonate (2.5 g) were added to N,N-dimethylformamide(20 mL), and stirred at 45°C for three hours.

Water was added thereto, the mixture was extracted with ethyl acetate, and the solvent was distilled off. The resultant was purified by silica gel column chromatography (developing solvent: n-hexane / ethyl acetate) to obtain isopropyl 4-bromo-1-(tert-butyl)-1H-1,2,3-triazole-5-carboxylate (1.4 g, at a yield of 80%).

Step 4: Conversion to isopropyl 1-(tert-butyl)-4-(3-(trifluoromethyl)phenoxy)-1H-1,2,3-triazole-5-carboxylate (English name: isopropyl 1-(tert-butyl)-4-(3-(trifluoromethyl)phenoxy)-1H-1,2,3-triazole-5-carboxylate)

[Chemical Formula 70]

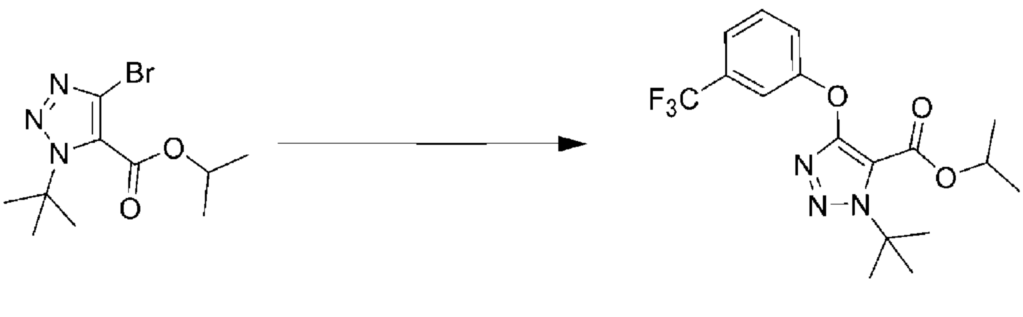

α,α,α-Trifluoro-m-cresol (0.32 g), isopropyl 4-bromo-1-(tert-butyl)-1H-1,2,3-triazole-5-carboxylate (0.29 g),

N,N-dimethylglycine (21 mg), and copper iodide (19 mg) were added to toluene (2.5 mL). The resultant was irradiated with microwaves at 150°C for 1.5 hours.

The resultant was purified by silica gel column chromatography (developing solvent: n-hexane / ethyl acetate) to obtain 0.20 g of isopropyl 1-(tert-butyl)-4-(3-(trifluoromethyl)phenoxy)-1H-1,2,3-triazole-5-carboxylate.

Step 5: Conversion to 1-(tert-butyl)-4-(3-(trifluoromethyl)phenoxy)-1H-1,2,3-triazole-5-carboxylic acid (English name: 1-(tert-butyl)-4-(3-(trifluoromethyl)phenoxy)-1H-1,2,3-triazole-5-carboxylic acid)

[Chemical Formula 71]

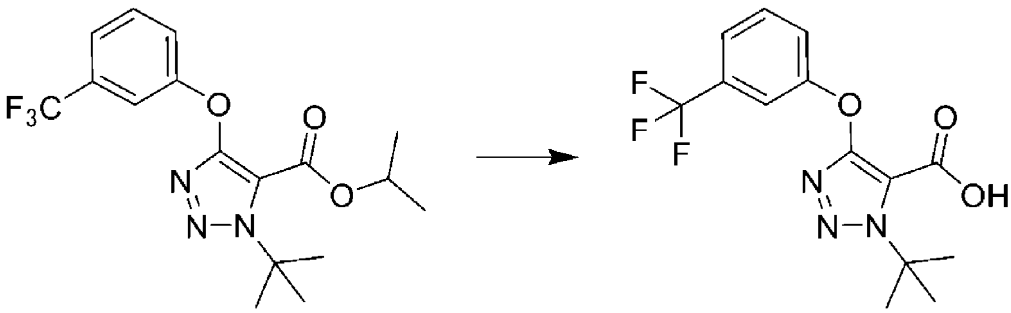

Isopropyl 1-(tert-butyl)-4-(3-(trifluoromethyl)phenoxy)-1H-1,2,3-triazole-5-carboxylate (0.20 g) was dissolved in tetrahydrofuran (0.50 mL), water (0.17 mL), and methanol (0.50 mL). Lithium hydroxide monohydrate (73 mg) was added thereto, and stirred at 25°C for 16 hours.

After the reaction was terminated with 1N hydrochloric acid, the resultant was separated into liquids with ethyl acetate, and dried over magnesium sulfate. The solvent was distilled off to obtain 1-(tert-butyl)-4-(3-(trifluoromethyl) phenoxy)-1H-1,2,3-triazole-5-carboxylic acid (0.12 g).

Step 6: Conversion to methyl 2-(1-(tert-butyl)-4-(3-(trifluoromethyl)phenoxy)-1H-1,2,3-triazole-5-carbonyl)-1-(2,4-dimethylbenzyl)hydrazine-1-carboxylate (English name: methyl 2-(1-(tert-butyl)-4-(3-(trifluoromethyl)phenoxy)-1H-1,2,3-triazole-5-carbonyl)-1-(2,4-dimethylbenzyl)hydrazine-1-carboxylate)

[Chemical Formula 72]

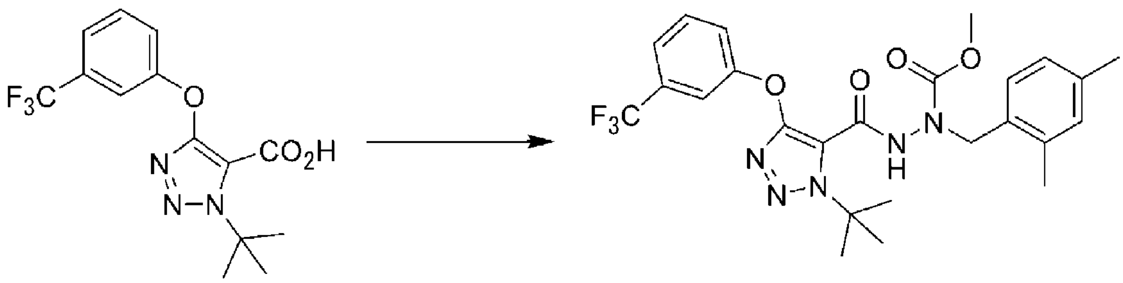

1-(Tert-butyl)-4-(3-(trifluoromethyl)phenoxy)-1H-1,2,3-triazole-5-carboxylic acid (0.060 g), methyl 1-(2,4-dimethylbenzyl)hydrazine-1-carboxylate (30 mg), triethylamine (36 mg), and 1-[bis(dimethylamino)methylene]-1H-1,2,3-triazolo[4,5-b]pyridinium-3-oxide hexafluorophosphate (HATU) (68 mg) were added sequentially to tetrahydrofuran (0.39 mL). The mixture was stirred at 25°C for 18 hours.

The resultant was purified by preparative chromatography to obtain methyl 2-(1-(tert-butyl)-4-(3-(trifluoromethyl) phenoxy)-1H-1,2,3-triazole-5-carbonyl)-1-(2,4-dimethylbenzyl)hydrazine-1-carboxylate (0.062 g).

(Production Example 10)

Methyl 2-(1-(tert-butyl)-4-(3-chlorophenoxy)-1H-1,2,3-triazole-5-carbonyl)-1-(2,4-dimethylbenzyl)hydrazine-1-carboxylate (English name: methyl 2-(1-(tert-butyl)-4-(3-chlorophenoxy)-1H-1,2,3-triazole-5-carbonyl)-1-(2,4-dimethylbenzyl)hydrazine-1-carboxylate) (compound No.: c-131) was produced by the following steps.

Step 1: Conversion to 4-bromo-1-(tert-butyl)-1H-1,2,3-triazole-5-carboxamide (English name: 4-bromo-1-(tert-butyl)-1H-1,2,3-triazole-5-carboxamide)

[Chemical Formula 73]

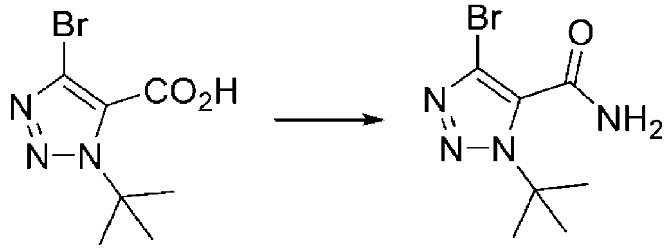

4-Bromo-1-(tert-butyl)-1H-1,2,3-triazole-5-carboxylic acid (0.50 g) was dissolved in N,N-dimethylformamide (6.7 mL). Ammonium chloride (0.21 g), 1-[bis(dimethylamino)methylene]-1H-1,2,3-triazolo[4,5-b]pyridinium-3-oxide hexafluorophosphate (HATU) (1.1 g), and triethylamine (0.61 g) were added thereto.

After water was added to the reaction liquid, the resultant was extracted with ethyl acetate. After the obtained organic phase was concentrated with an evaporator, the resultant was purified by silica gel column chromatography to obtain 4-bromo-1-(tert-butyl)-1H-1,2,3-triazole-5-carboxamide (0.36 g, at a yield of 73%).

Step 2: Conversion to 4-bromo-1-(tert-butyl)-1H-1,2,3-triazole-5-carbonitrile (English name: 4-bromo-1-(tert-butyl)-1H-1,2,3-triazole-5-carbonitrile)

[Chemical Formula 74]

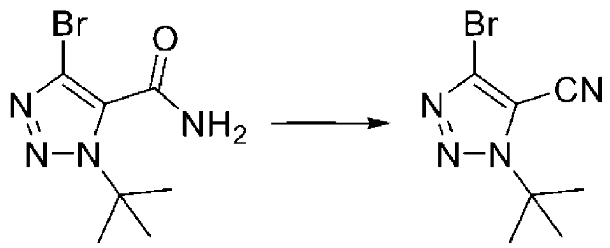

4-Bromo-1-(tert-butyl)-1H-1,2,3-triazole-5-carboxamide (0.050 g) was dissolved in dichloromethane (0.67 mL). Trifluoroacetic anhydride (0.13 g) and triethylamine (72 mg) were added thereto, and stirred at 25°C for one hour.

After an aqueous solution of saturated sodium bicarbonate was added to the reaction liquid to quench the reaction, the resultant was extracted with ethyl acetate. The obtained organic phase was concentrated with an evaporator. After the residue was separated and collected by silica gel column chromatography, the resultant was purified by amino silica gel column chromatography to obtain 4-bromo-1-(tert-butyl)-1H-1,2,3-triazole-5-carbonitrile (0.039 g, at a yield of 84%).

Step 3: Conversion to 1-(tert-butyl)-4-(3-chlorophenoxy)-1H-1,2,3-triazole-5-carbonitrile (English name: 1-(tert-butyl)-4-(3-chlorophenoxy)-1H-1,2,3-triazole-5-carbonitrile)

[Chemical Formula 75]

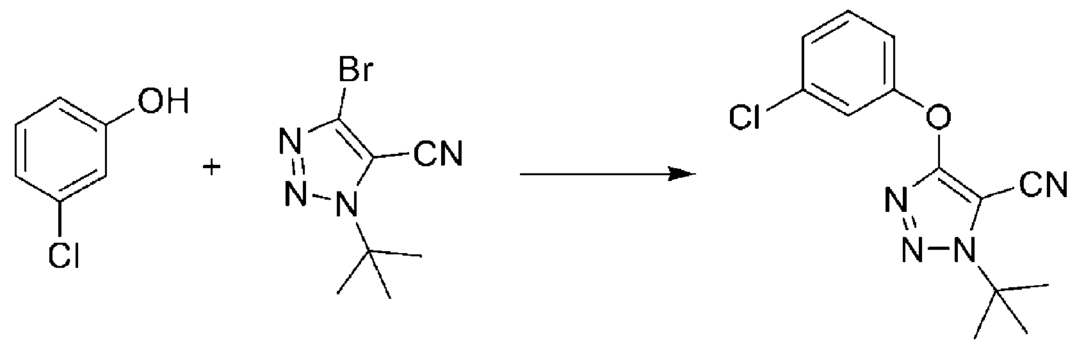

1-Chloro3-hydroxybenzene (73 mg), 4-bromo-1-(tert-butyl)-1H-1,2,3-triazole-5-carbonitrile (0.10 g), and potassium carbonate (90 mg) were added to dimethyl sulfoxide(2.2 mL), and stirred at 120°C for four hours.

Water was added to the reaction liquid, and the resultant was extracted with ethyl acetate. The obtained organic phase was concentrated with an evaporator. The resultant was purified by silica gel chromatography to obtain 1-(tert-butyl)-4-(3-chlorophenoxy)-1H-1,2,3-triazole-5-carbonitrile (0.09 g, at a yield of 70%).

Step 4: Conversion to 1-(tert-butyl)-4-(3-chlorophenoxy)-1H-1,2,3-triazole-5-carboxamide (English name: 1-(tert-butyl)-4-(3-chlorophenoxy)-1H-1,2,3-triazole-5-carboxamide)

[Chemical Formula 76]

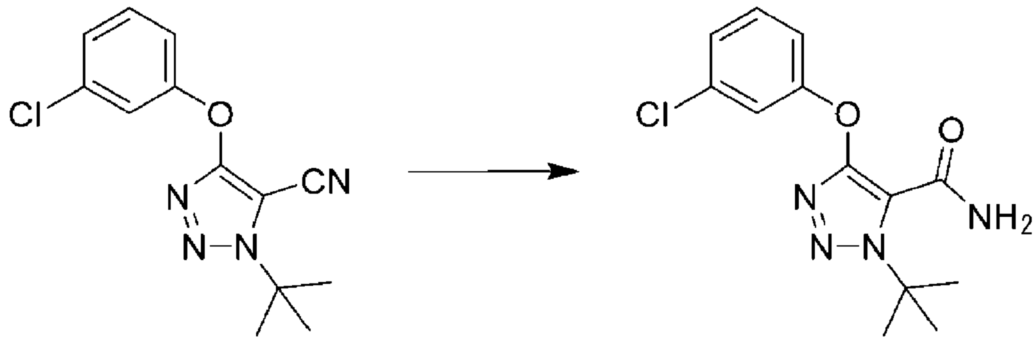

1-(Tert-butyl)-4-(3-chlorophenoxy)-1H-1,2,3-triazole-5-carbonitrile (0.15 g), and sodium hydroxide (4 M, 2.7 mL) were added to ethanol (2.7 mL), and stirred at 100°C for 12 hours.

After the reaction liquid was quenched with 1N hydrochloric acid, the resultant was extracted with ethyl acetate. The obtained organic phase was dried over magnesium sulfate. The solvent was distilled off with an evaporator to obtain 1-(tert-butyl)-4-(3-chlorophenoxy)-1H-1,2,3-triazole-5-carboxamide (0.16 g).

Step 5: Conversion to 1-(tert-butyl)-4-(3-chlorophenoxy)-1H-1,2,3-triazole-5-carboxylic acid (English name: 1-(tert-butyl)-4-(3-chlorophenoxy)-1H-1,2,3-triazole-5-carboxylic acid)

[Chemical Formula 77]

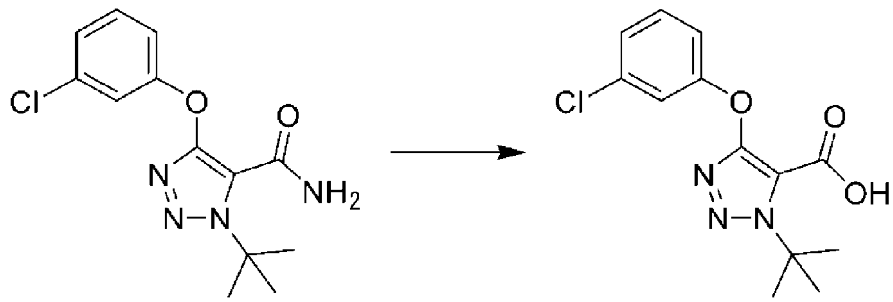

1-(Tert-butyl)-4-(3-chlorophenoxy)-1H-1,2,3-triazole-5-carboxamide (0.16 g), and sodium nitrite (0.11 g) were added to trifluoroacetic acid (1.8 mL) at 0°C, and stirred at 0°C for two hours.

Water was added to the reaction liquid, and then the resultant was extracted with ethyl acetate. The obtained organic phase was dried over magnesium sulfate. The solvent was distilled off with an evaporator to obtain 1-(tert-butyl)-4-(3-chlorophenoxy)-1H-1,2,3-triazole-5-carboxylic acid (0.13 g, at a yield of 81%).

Step 6: Conversion to methyl 2-(1-(tert-butyl)-4-(3-chlorophenoxy)-1H-1,2,3-triazole-5-carbonyl)-1-(2,4-dimethylbenzyl)hydrazine-1-carboxylate (English name: methyl 2-(1-(tert-butyl)-4-(3-chlorophenoxy)-1H-1,2,3-triazole-5-carbonyl)-1-(2,4-dimethylbenzyl)hydrazine-1-carboxylate)

[Chemical Formula 78]

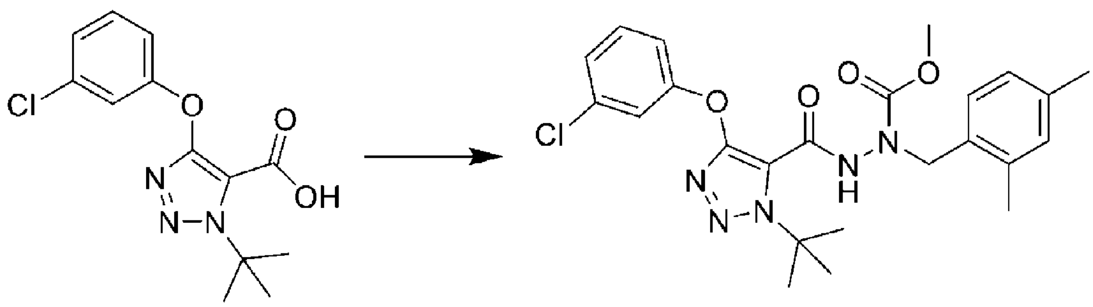

1-(Tert-butyl)-4-(3-chlorophenoxy)-1H-1,2,3-triazole-5-carboxylic acid (41 mg), methyl 1-(2,4-dimethylbenzyl) hydrazine-1-carboxylate (35 mg), 1-[bis(dimethylamino)methylene]-1H-1,2,3-triazolo[4,5-b]pyridinium-3-oxide hexafluorophosphate (HATU) (80 mg), and triethylamine (42 mg) were added to tetrahydrofuran (0.70 mL), and stirred at 25°C for 16 hours.

The reaction liquid was purified by preparative HPLC (developing solvent: water /acetonitrile) to obtain methyl 2-(1-(tert-butyl)-4-(3-chlorophenoxy)-1H-1,2,3-triazole-5-carbonyl)-1-(2,4-dimethylbenzyl)hydrazine-1-carboxylate (0.040 g, at a yield of 59%).

(Production Example 11)

Methyl 2-(3-chloro1-isopropyl4-((4-(trifluoromethyl)thiazol-2-yl)oxy)-1H-pyrazole-5-carbonyl)-1-(2,4-dimethylbenzyl)hydrazine-1-carboxylate (English name: methyl 2-(3-chloro-1-isopropyl-4-((4-(trifluoromethyl)thiazol-2-yl) oxy)-1H-pyrazole-5-carbonyl)-1-(2,4-dimethylbenzyl)hydrazine-1-carboxylate) (compound No.: c-137) was produced by the following steps.

Step 1: Conversion to ethyl 3-chlorol-isopropyl4-((4-(trifluoromethyl)thiazol-2-yl)oxy)-1H-pyrazole-5-carboxylate (English name: ethyl 3-chloro-1-isopropyl-4-((4-(trifluoromethyl)thiazol-2-yl)oxy)-1H-pyrazole-5-carboxylate)

[Chemical Formula 79]

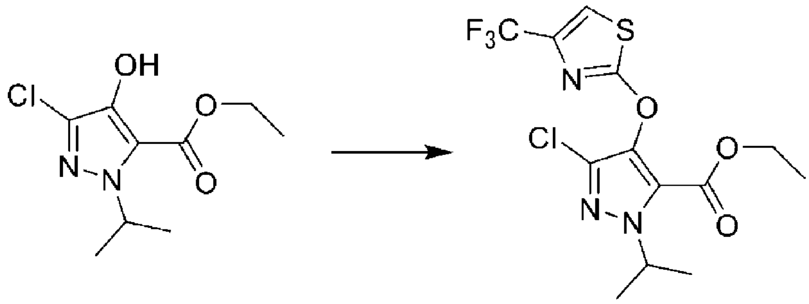

Ethyl 3-chloro-4-hydroxy-1-isopropyl1H-pyrazole-5-carboxylate (0.059 g) was dissolved in N,N-dimethylformamide (2.5 mL). 2-Chloro-4-(trifluoromethyl)thiazole (0.071 g) and potassium carbonate (0.070 g) were added thereto, and stirred at 60°C for 17 hours.

Water was added to the reaction liquid, the resultant was extracted with ethyl acetate, and the extracted phase was washed with saturated saline. Then, the resultant was dried over anhydrous magnesium sulfate, the solvent was distilled off under reduced pressure, and the resultant was purified by silica gel column chromatography (developing solvent: n-hexane / ethyl acetate) to obtain 0.053 g of ethyl 3-chloro-1-isopropyl-4-((4-(trifluoromethyl)thiazol-2-yl)oxy)-1H-pyrazole-5-carboxylate (at a yield of 54%).

Step 2: Conversion to 3-chloro-1-isopropyl-4-((4-(trifluoromethyl)thiazol-2-yl)oxy)-1H-pyrazole-5-carboxylic acid (English name: 3-chloro-1-isopropyl-4-((4-(trifluoromethyl)thiazol-2-yl)oxy)-1H-pyrazole-5-carboxylic acid)

[Chemical Formula 80]

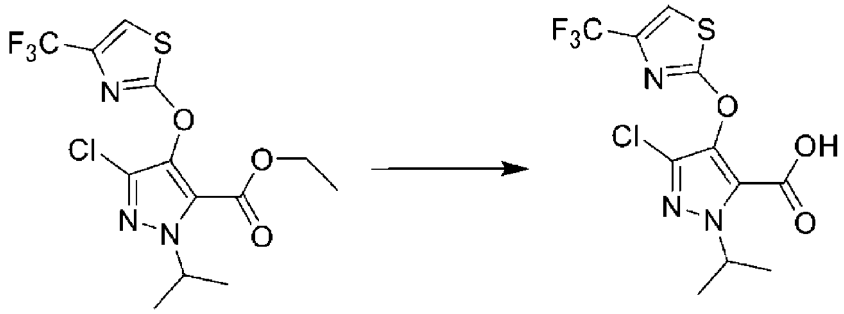

Ethyl 3-chloro-1-isopropyl-4-((4-(trifluoromethyl)thiazol-2-yl)oxy)-1H-pyrazole-5-carboxylate (0.053 g) was dissolved in tetrahydrofuran (0.82 mL), methanol (0.28 mL), and water (0.28 mL). Lithium hydroxide monohydrate (0.023 g) was added thereto, and stirred at room temperature for 1.5 hours.

Dilute hydrochloric acid was added to the reaction liquid, the resultant was extracted with ethyl acetate, and the extracted phase was washed with saturated saline. Then, the resultant was dried over anhydrous magnesium sulfate, and the solvent was distilled off under reduced pressure to obtain 0.047 g of 3-chloro-1-isopropyl-4-((4-(trifluoromethyl)thiazol-2-yl)oxy)-1H-pyrazole-5-carboxylic acid (at a yield of 97%).

Step 3: Conversion to methyl 2-(3-chloro-1-isopropyl-4-((4-(trifluoromethyl)thiazol-2-yl)oxy)-1H-pyrazole-5-carbonyl)-1-(2,4-dimethylbenzyl)hydrazine-1-carboxylate (English name: methyl 2-(3-chloro-1-isopropyl-4-((4-(trifluoromethyl)thiazol-2-yl)oxy)-1H-pyrazole-5-carbonyl)-1-(2,4-dimethylbenzyl)hydrazine-1-carboxylate)

[Chemical Formula 81]

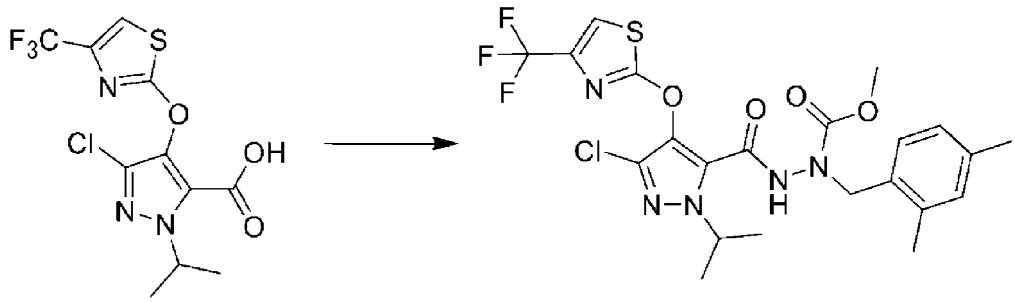

3-Chloro-1-isopropyl-4-((4-(trifluoromethyl)thiazol-2-yl)oxy)-1H-pyrazole-5-carboxylic acid (0.034 g) was dissolved in tetrahydrofuran (1 mL). Methyl 1-(2,4-dimethylbenzyl)hydrazine-1-carboxylate (0.030 g), 1-[bis(dimethylamino) methylene]-1H-1,2,3-triazolo[4,5-b]pyridinium-3-oxide hexafluorophosphate (0.073 g) and triethylamine (0.029 g) were added thereto, and stirred at room temperature for 16 hours.

The solvent was distilled off under reduced pressure, and the resultant was purified by silica gel column chromatography (developing solvent: n-hexane / ethyl acetate) to obtain 0.044 g of methyl 2-(3-chloro-1-isopropyl-4-((4-(trifluoromethyl)thiazol-2-yl)oxy)-1H-pyrazole-5-carbonyl)-1-(2,4-dimethylbenzyl)hydrazine-1-carboxylate (at a yield of 84%).

(Production Example 12)

Methyl 1-(2,4-dimethylbenzyl)-2-(1-(thiazol-2-yl)-4-(3-(trifluoromethyl)phenoxy)-1H-pyrazole-5-carbonyl)hydrazine-1-carboxylate (English name: methyl 1-(2,4-dimethylbenzyl)-2-(1-(thiazol-2-yl)-4-(3-(trifluoromethyl)phenoxy)-1H-pyrazole-5-carbonyl)hydrazine-1-carboxylate) (compound No.: c-120) was produced by the following steps.

Step 1: Conversion to ethyl 1-(thiazol-2-yl)-4-(3-(trifluoromethyl)phenoxy)-1H-pyrazole-5-carboxylate (English name: ethyl 1-(thiazol-2-yl)-4-(3-(trifluoromethyl)phenoxy)-1H-pyrazole-5-carboxylate)

[Chemical Formula 82]

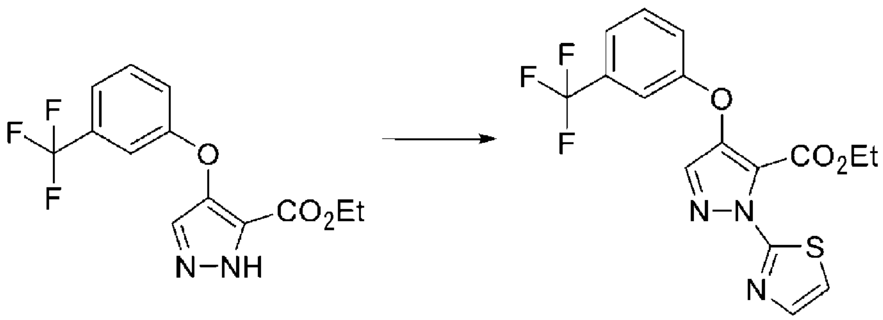

Ethyl 4-(3-(trifluoromethyl)phenoxy)-1H-pyrazole-5-carboxylate (150 mg) was dissolved in N,N-dimethylformamide dimethyl acetal (2 mL), followed by adding 2-bromo-1,3-thiazole (81.9 mg) and cesium carbonate (406.3 mg) thereto, and stirring the resultant at 100°C for three hours.

After the reaction liquid was filtered, water was added thereto, and the resultant was extracted with ethyl acetate. After an organic phase was dried over anhydrous sodium sulfate, and filtered, the organic phase was subjected to a

distillation under reduced pressure. The obtained residue was purified by silica gel column chromatography (developing solvent: n-hexane / ethyl acetate) to obtain 110 mg of the target compound (at a yield of 58%).

Step 2: Conversion to 1-(thiazol-2-yl)-4-(3-(trifluoromethyl)phenoxy)-1H-pyrazole-5-carboxylic acid (English name: 1-(thiazol-2-yl)-4-(3-(trifluoromethyl)phenoxy)-1H-pyrazole-5-carboxylic acid)

[Chemical Formula 83]

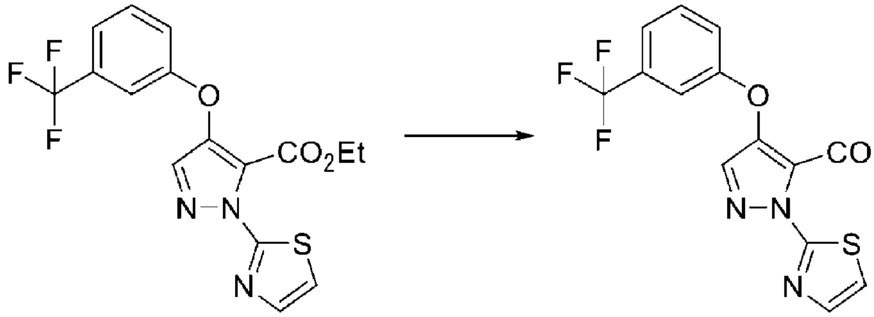

Ethyl 1-(thiazol-2-yl)-4-(3-(trifluoromethyl)phenoxy)-1H-pyrazole-5-carboxylate (110 mg) was dissolved in a mixture solvent composed of methanol (2 mL) and water (1 mL). Lithium hydroxide monohydrate (24mg) was added thereto while carrying out stirring at room temperature, and then the resultant was stirred at room temperature for two hours.

Dilute hydrochloric acid was added to the reaction liquid, and then the reaction liquid was distilled off under reduced pressure. The obtained residue was purified by preparative HPLC (developing solvent: water / acetonitrile) to obtain 71.1 mg of the target compound (at a yield of 100%).

Step 3 : Conversion to methyl 1-(2,4-dimethylbenzyl)-2-(1-(thiazol-2-yl)-4-(3-(trifluoromethyl)phenoxy)-1H-pyrazole-5-carbonyl)hydrazine-1-carboxylate (English name: methyl 1-(2,4-dimethylbenzyl)-2-(1-(thiazol-2-yl)-4-(3-(trifluoromethyl)phenoxy)-1H-pyrazole-5-carbonyl)hydrazine-1-carboxylate)

[Chemical Formula 84]

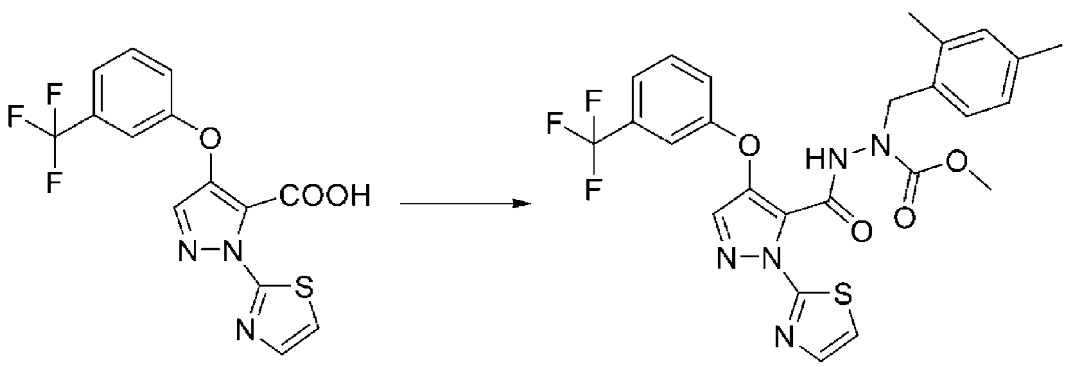

1-(thiazol-2-yl)-4-(3-(trifluoromethyl)phenoxy)-1H-pyrazole-5-carboxylic acid (20mg) was dissolved in tetrahydrofuran (1 mL). Methyl 1-(2,4-dimethylbenzyl)hydrazine-1-carboxylate (17.6mg), 1-[bis(dimethylamino)methylene]-1H-1,2,3-triazolo[4,5-b]pyridinium-3-oxide hexafluorophosphate (42.8mg) and triethylamine (17.1mg) were added thereto, and stirred at room temperature overnight.

After the reaction liquid was distilled off under reduced pressure, the resultant was purified by silica gel column chromatography (developing solvent: n-hexane / ethyl acetate) to obtain 9 mg of the target product (at a yield of 30%).

Compounds shown in Table 1 were produced through steps similar to those in the above-mentioned Production

Examples. The melting point or the external appearance (AMORPHOUS or VISCOUS OIL) is recorded in the property column. The LCMS retention times of compounds, the external appearance of which was AMORPHOUS or VISCOUS OIL, are recorded.

The LCMS (SQD2) retention time was determined by the following method. The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (SQ Detector 2 detector (positive and negative ion electrospray modes and an atmospheric pressure ionization mode), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% by mass of formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

The LCMS (QDa) retention time was determined by the following method. The measurement was carried out with a system using an ultra-high-performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (ACQUITY QDa detector (positive and negative ion electrospray modes), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% by mass of formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

[Table 1]

TABLE 1

| COMPOUND NO. | CHEMICAL FORMULA | PROPERTY | LCMS (SQD2) RETENTION TIME | LCMS (QDa) RETENTION TIME |
|---|---|---|---|---|
| c-1 | 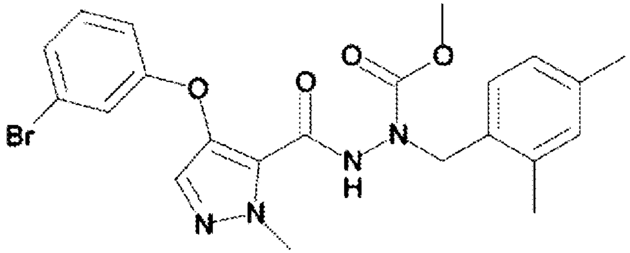 | VISCOUS OIL | 1.86min | |
| c-2 | 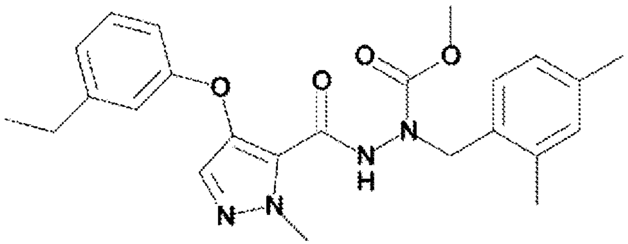 | VISCOUS OIL | 1.88min | |
| c-3 | 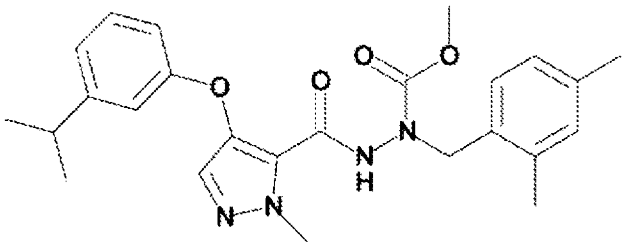 | VISCOUS OIL | 1.99min | |
| c-4 | 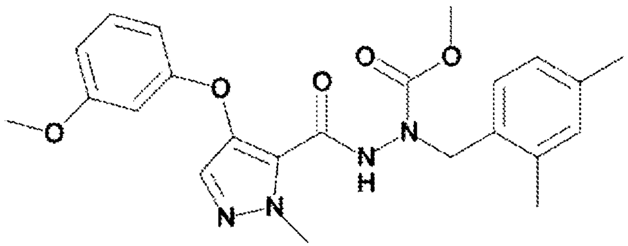 | VISCOUS OIL | | 1.74min |

[Table 2]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-5 | 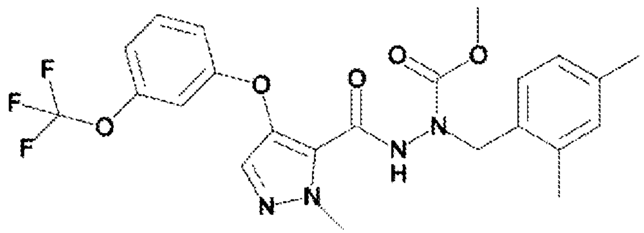 | VISCOUS OIL | | 1.90min |
| c-6 | 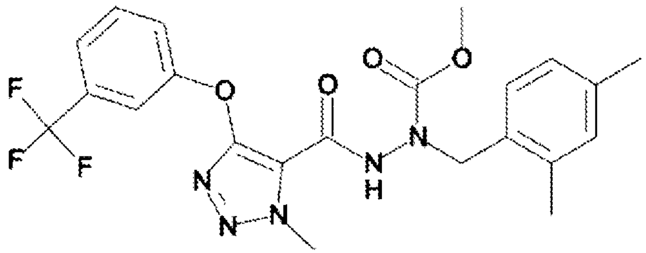 | AMORPHOUS | 1.76min | |
| c-7 | 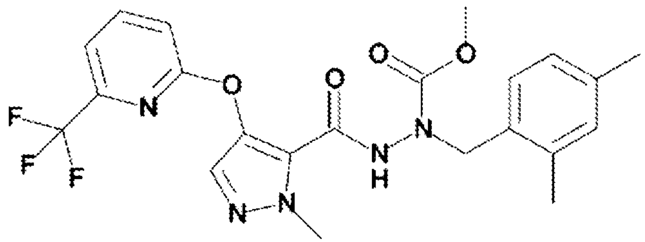 | VISCOUS OIL | | 1.66min |
| c-9 | 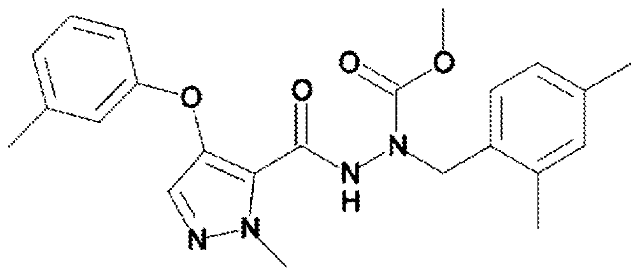 | VISCOUS | 1.79min | |

[Table 3]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-10 | 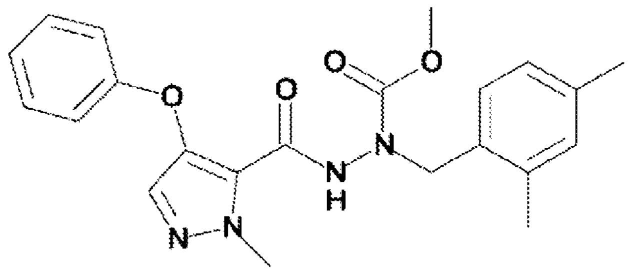 | VISCOUS OIL | 1.70min | |
| c-11 | 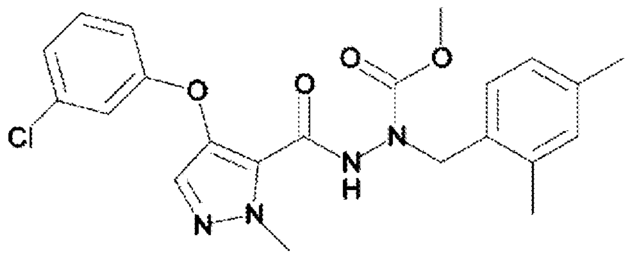 | VISCOUS OIL | 1.80min | |
| c-12 | 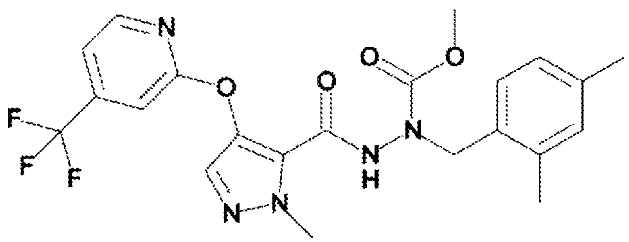 | m.p. 126-128°C | 1.71min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-13 | 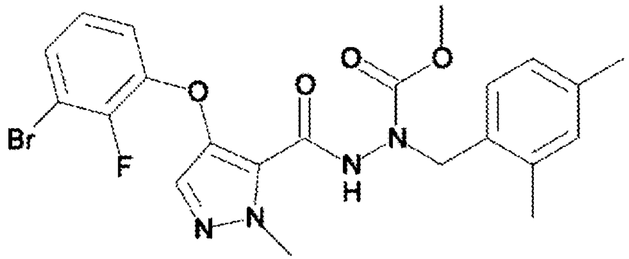 | VISCOUS | 1.81min | |

[Table 4]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-14 | 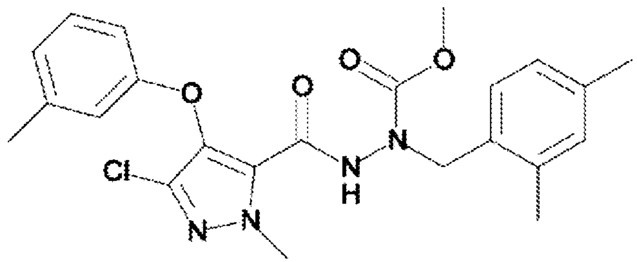 | VISCOUS OIL | 1.91min | |
| c-15 | 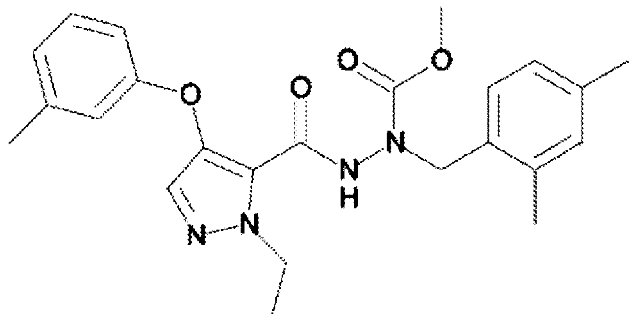 | VISCOUS OIL | 1.88min | |
| c-16 | 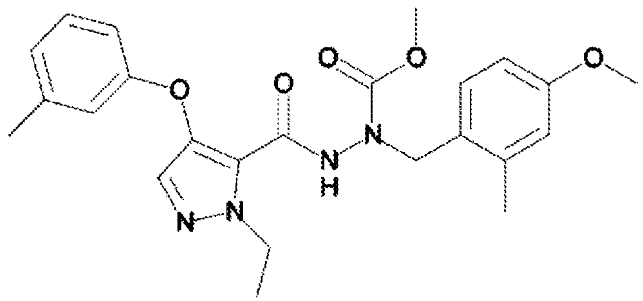 | VISCOUS OIL | 1.77min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-17 | 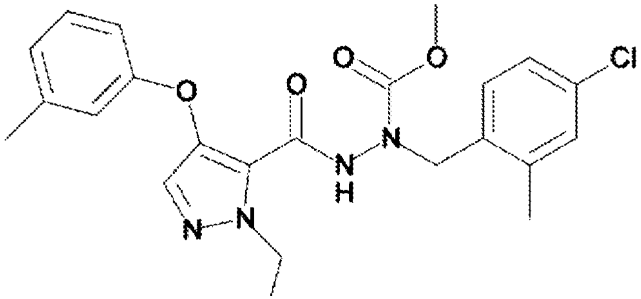 | VISCOUS OIL | 1.92min | |

[Table 5]

| TABLE 1(CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-18 | 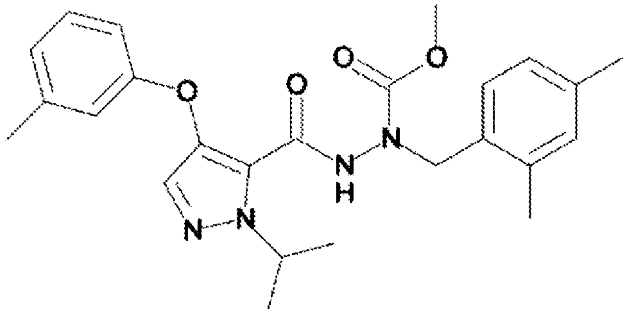 | VISCOUS OIL | 1.98min | |
| c-19 | 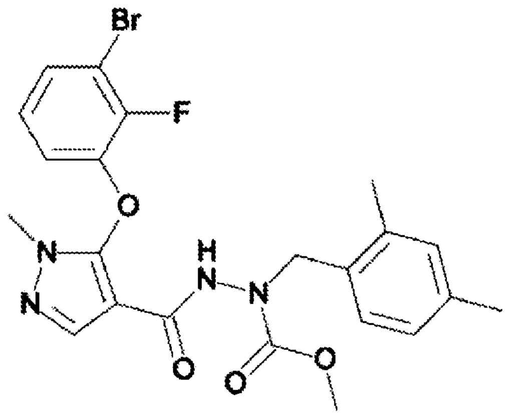 | VISCOUS OIL | | 1.55min |
| c-20 | 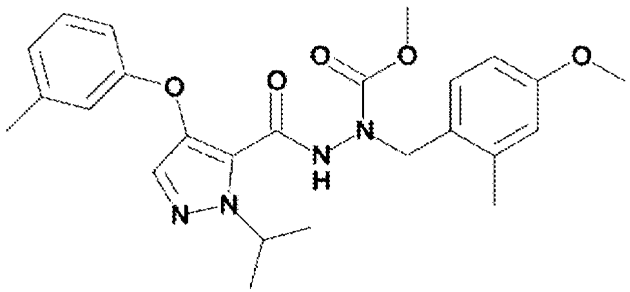 | VISCOUS OIL | 1.85min | |

[Table 6]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-22 | 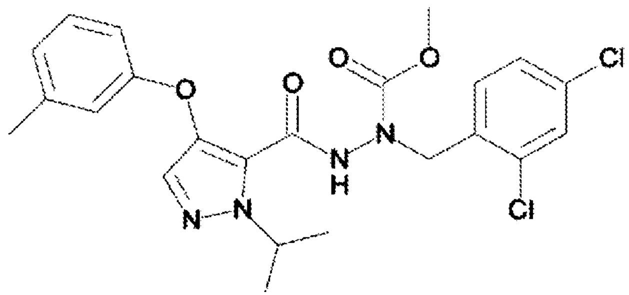 | VISCOUS OIL | 2.03min | |
| c-23 | 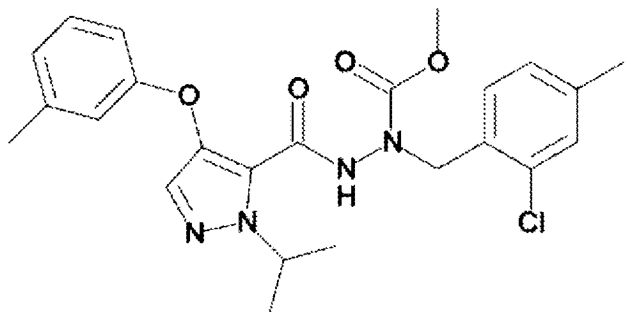 | VISCOUS OIL | 1.99min | |
| c-24 | 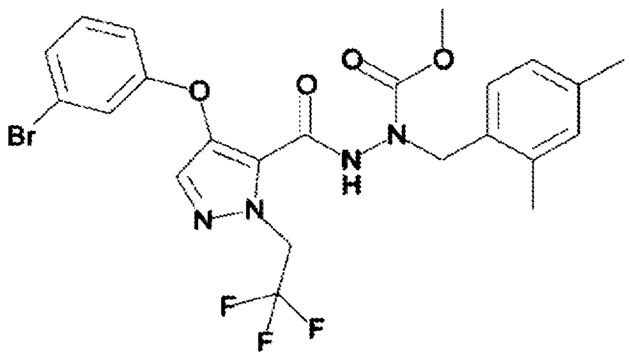 | m.p. 119-122°C | | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-25 | 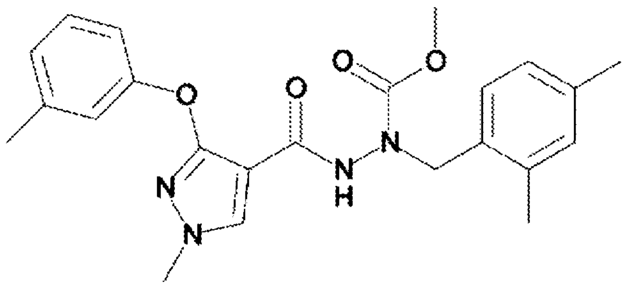 | VISCOUS OIL | 1.63min | |

[Table 7]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-26 | 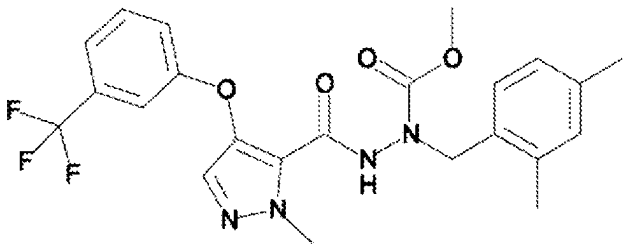 | AMORPHOUS | 1.80min | |
| c-27 | 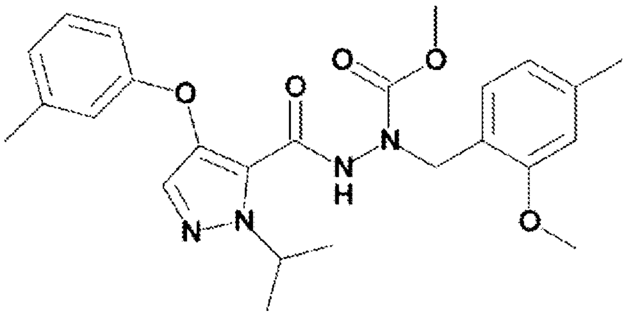 | VISCOUS OIL | 1.89min | |
| c-28 | 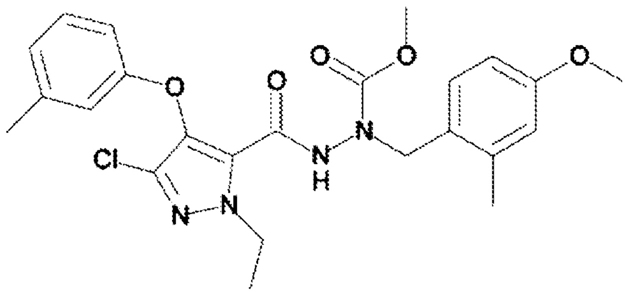 | VISCOUS OIL | 1.88min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-29 | 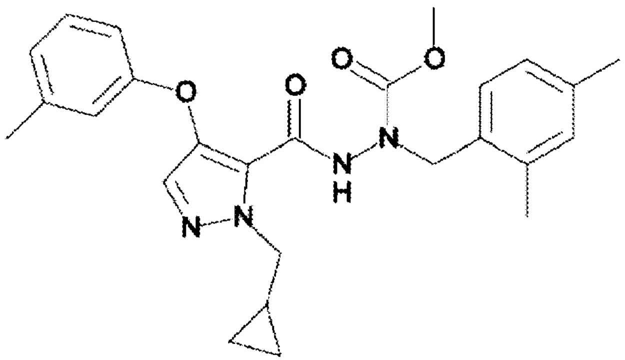 | VISCOUS OIL | 1.99min | |

[Table 8]

TABLE 1 (CONTINUED)

| COMPOUND NO. | CHEMICAL FORMULA | PROPERTY | LCMS (SQD2) RETENTION TIME | LCMS (QDa) RETENTION TIME |
|---|---|---|---|---|
| c-30 | 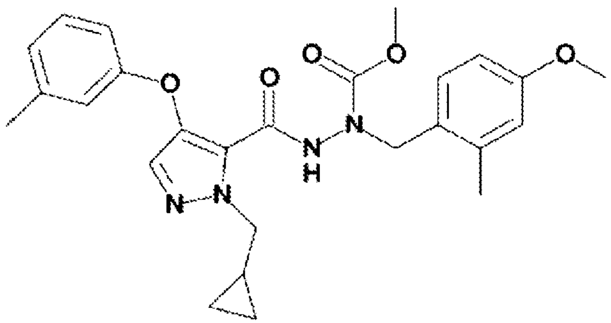 | VISCOUS OIL | 1.86min | |
| c-31 | 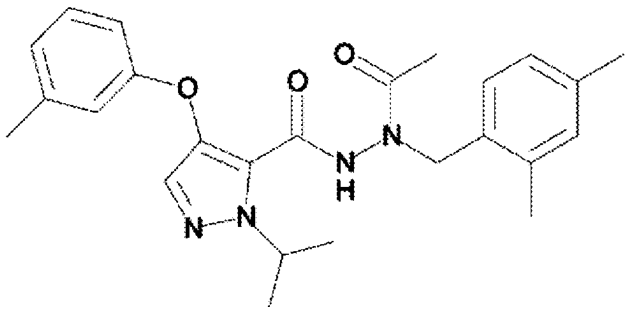 | VISCOUS OIL | 1.92min | |
| c-32 | 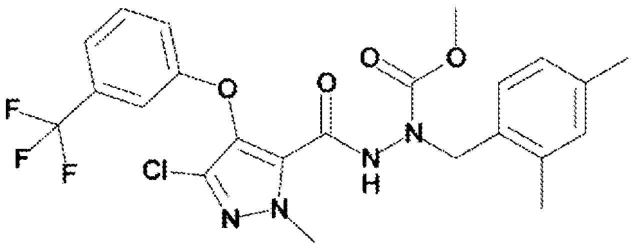 | AMORPHOUS | 1.95min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-33 | 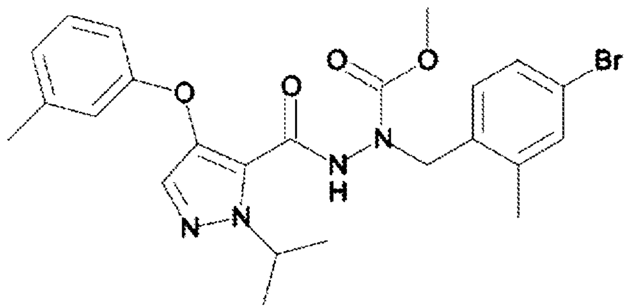 | VISCOUS OIL | 2.03min | |

[Table 9]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-34 | 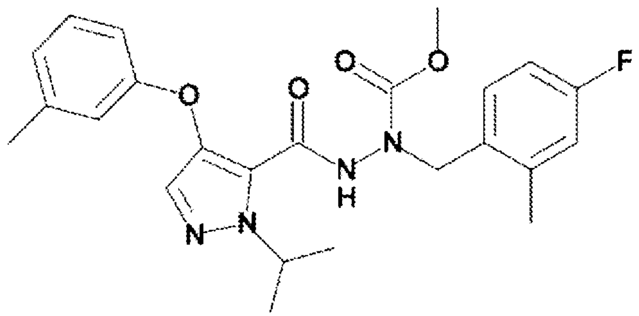 | VISCOUS OIL | 1.89min | |
| c-35 | 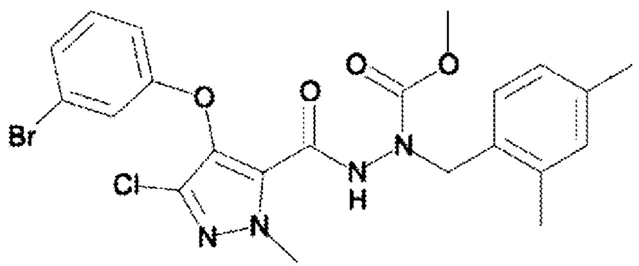 | AMORPHOUS | 1.95min | |
| c-36 | 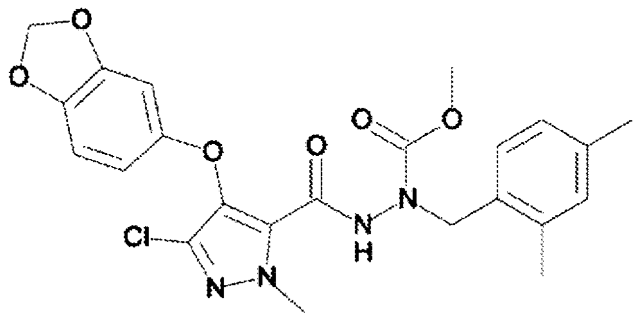 | AMORPHOUS | 1.76min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-37 | 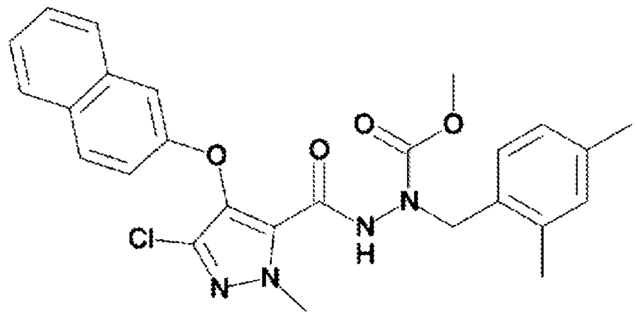 | AMORPHOUS | 1.96min | |

[Table 10]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-38 | 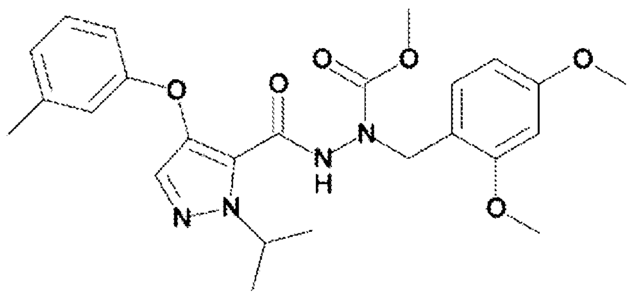 | VISCOUS OIL | 1.79min | |
| c-39 | 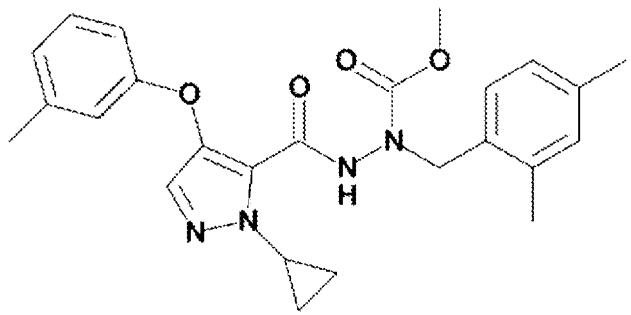 | VISCOUS OIL | 1.86min | |
| c-40 | 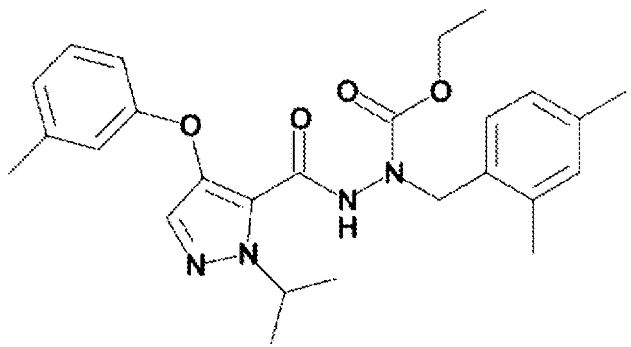 | VISCOUS OIL | 2.03min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-41 | 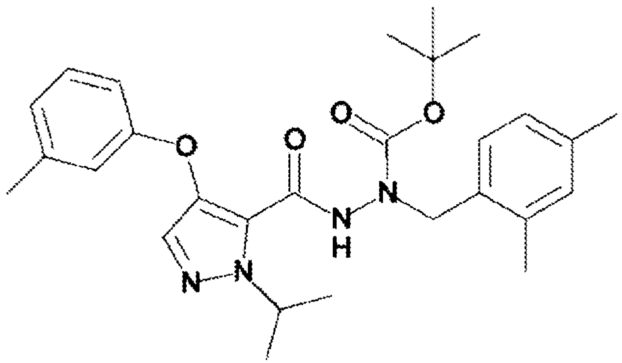 | VISCOUS OIL | 2.19min | |

[Table 11]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-42 | 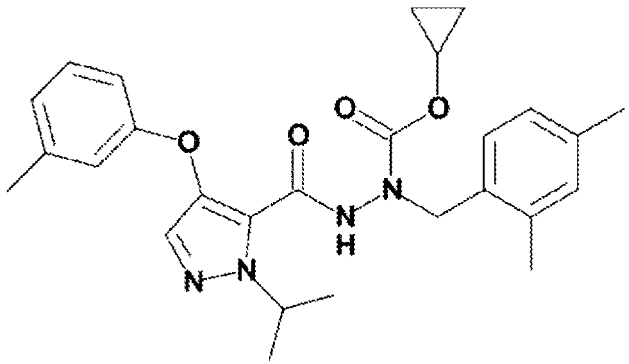 | VISCOUS OIL | 2.04min | |
| c-43 | 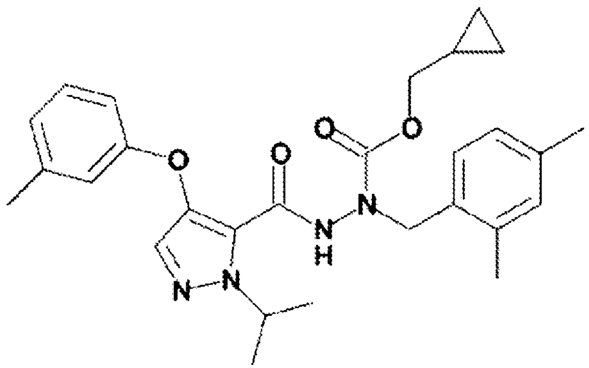 | VISCOUS OIL | 2.11min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-44 | 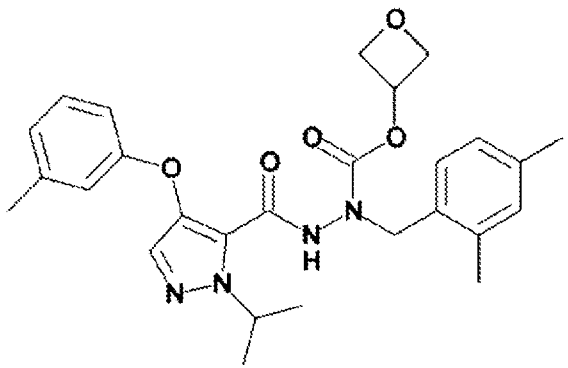 | VISCOUS OIL | 1.89min | |
| c-45 | 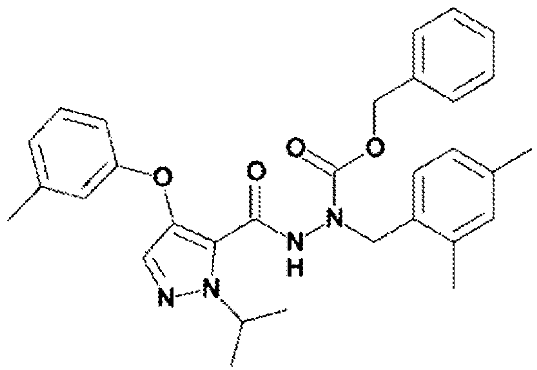 | VISCOUS OIL | 2.15min | |

[Table 12]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-46 | 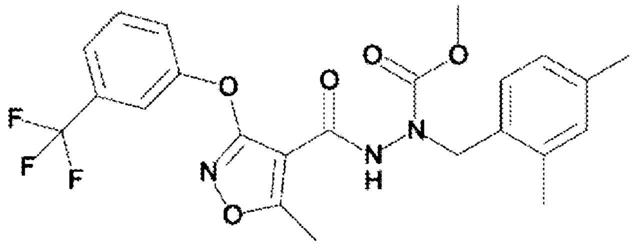 | m.p. 139-142°C | | |
| c-47 | 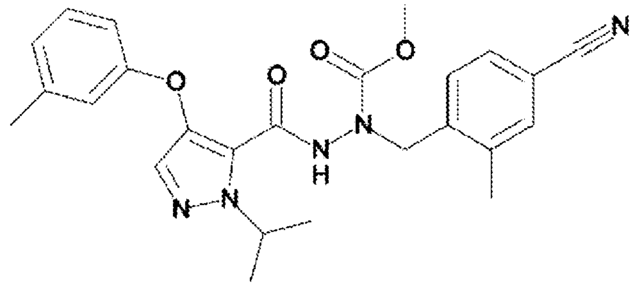 | VISCOUS | 1.76min | |
| c-48 | 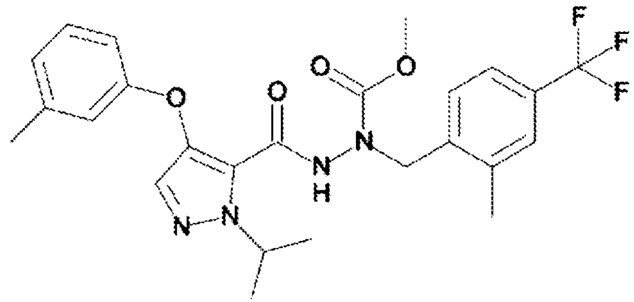 | VISCOUS OIL | 2.00min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-49 | 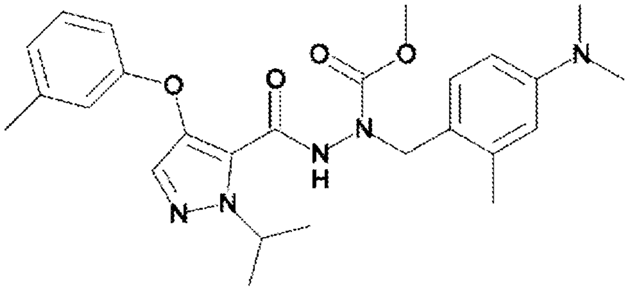 | VISCOUS OIL | 1.71min | |

[Table 13]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-50 | 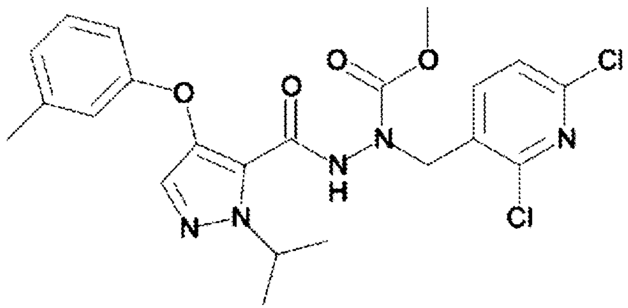 | VISCOUS OIL | 1.84min | |
| c-51 | 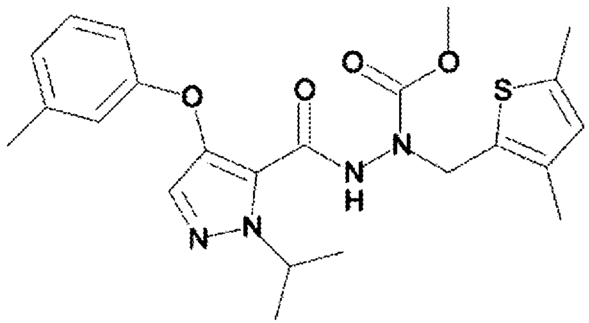 | VISCOUS OIL | 1.97min | |
| c-52 | 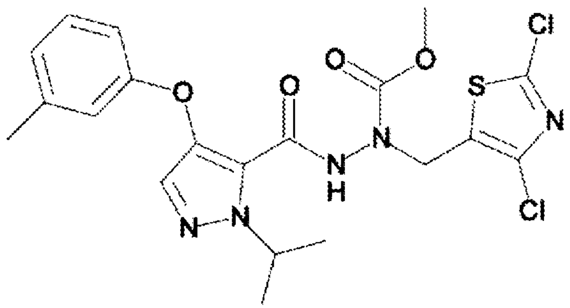 | VISCOUS OIL | 1.89min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-53 | 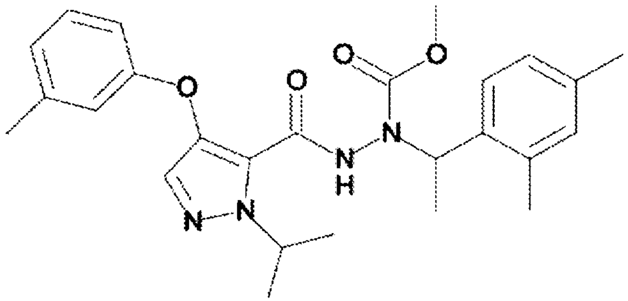 | VISCOUS OIL | 2.04min | |

[Table 14]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-54 | 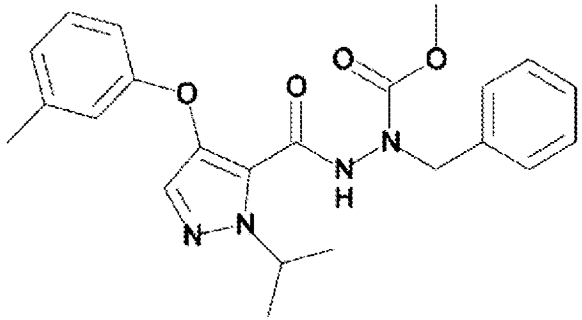 | VISCOUS OIL | 1.82min | |
| c-55 | 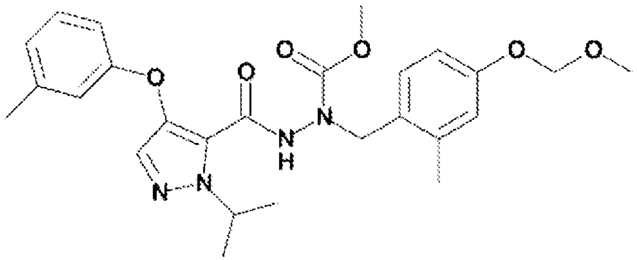 | VISCOUS OIL | 1.84min | |
| c-56 | 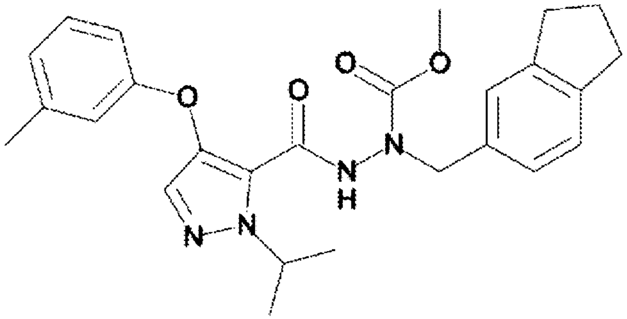 | VISCOUS | 2.04min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-57 | 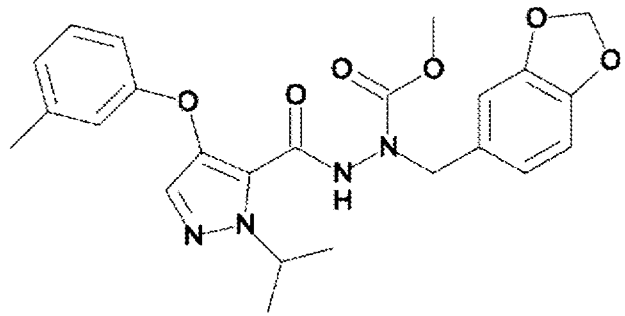 | VISCOUS OIL | 1.76min | |

[Table 15]

TABLE 1 (CONTINUED)

| COMPOUND NO, | CHEMICAL FORMULA | PROPERTY | LCMS (SQD2) RETENTION TIME | LCMS (QDa) RETENTION TIME |
|---|---|---|---|---|
| c-58 | 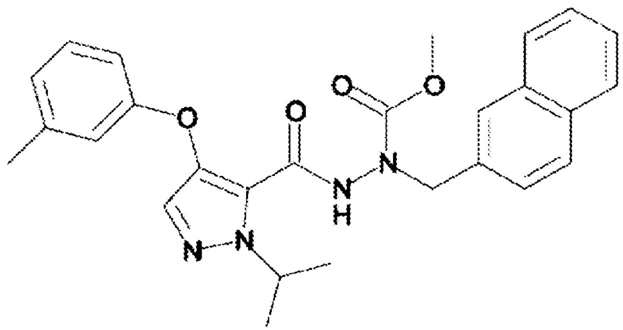 | VISCOUS OIL | 1.97min | |
| c-59 | 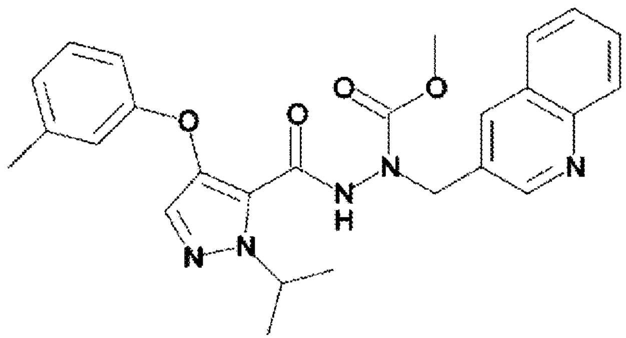 | m.p. 137-139°C | | |
| c-60 | 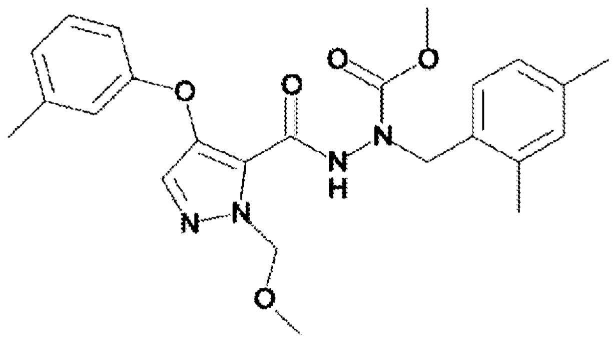 | VISCOUS OIL | 1.76min | |

(continued)

| TABLE 1 (CONTINUED) | | | | |
|---|---|---|---|---|
| COMPOUND NO, | CHEMICAL FORMULA | PROPERTY | LCMS (SQD2) RETENTION TIME | LCMS (QDa) RETENTION TIME |
| c-61 | 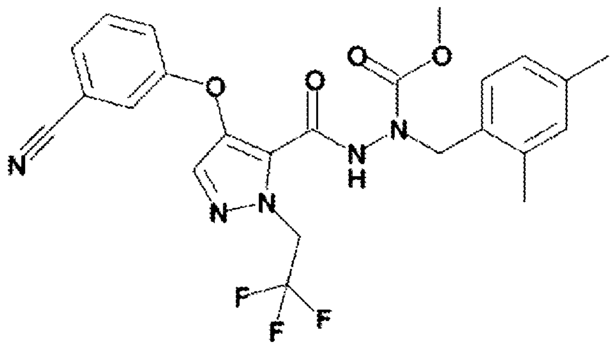 | VISCOUS OIL | 1.72min | |

[Table 16]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-62 | 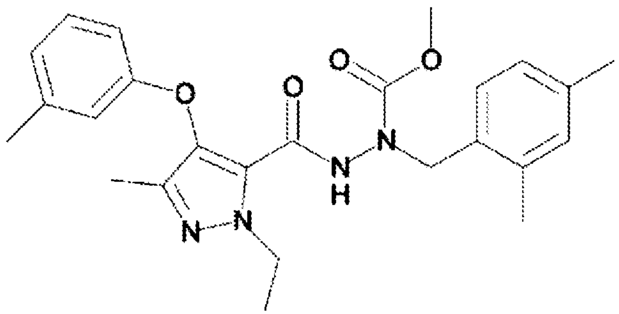 | - | 1.76min | |
| c-63 | 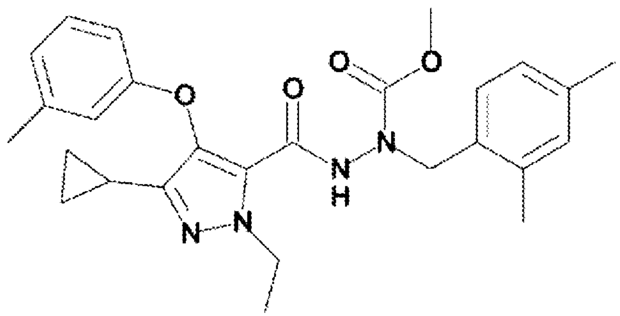 | - | 2.04min | |
| c-64 | 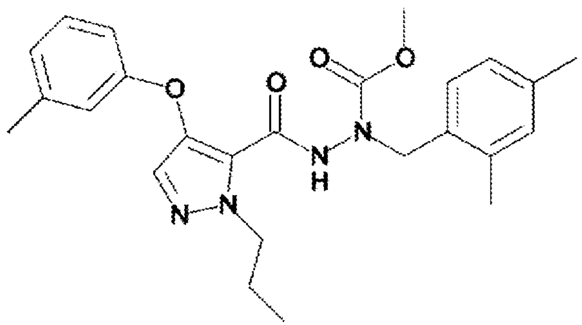 | VISCOUS OIL | 1.96min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-65 | 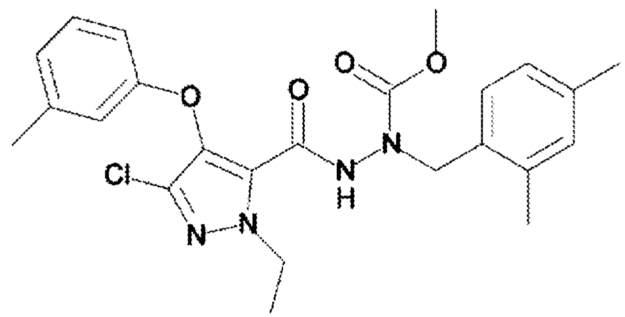 | VISCOUS OIL | 1.99min | |

[Table 17]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-66 | 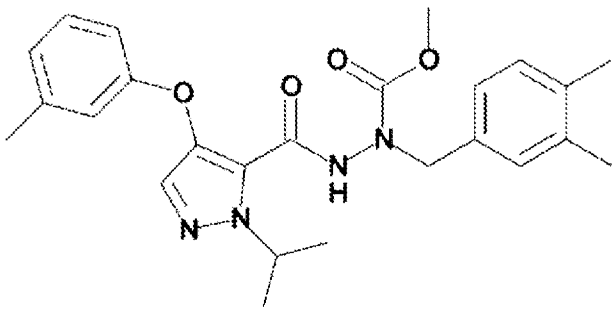 | VISCOUS OIL | 1.98min | |
| c-67 | 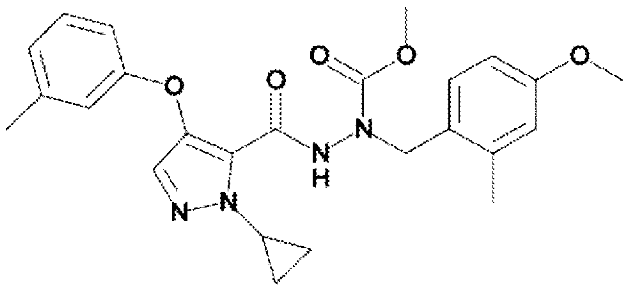 | VISCOUS OIL | 1.75min | |
| c-68 | 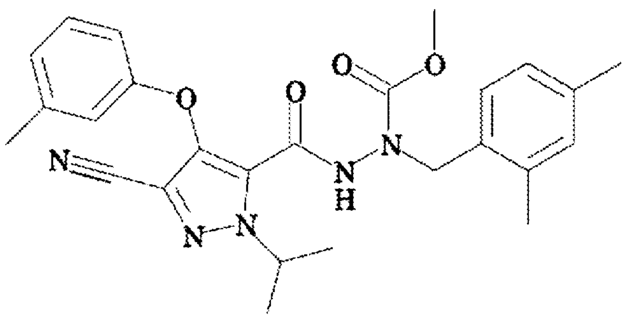 | m. p. 130-132°C | | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-69 | 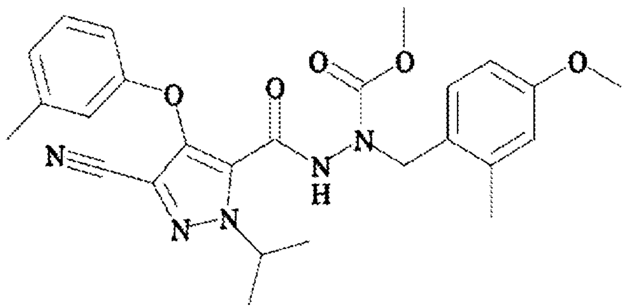 | m.p. 123-125°C | | |

[Table 18]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-70 | 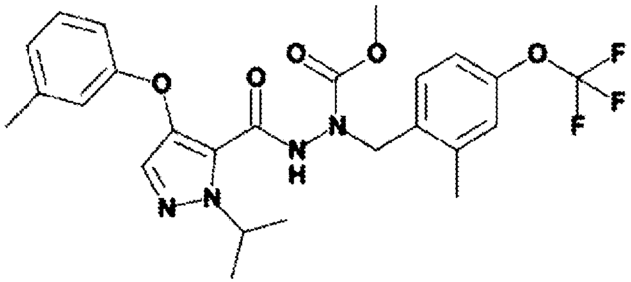 | VISCOUS OIL | 2.00min | |
| c-71 | 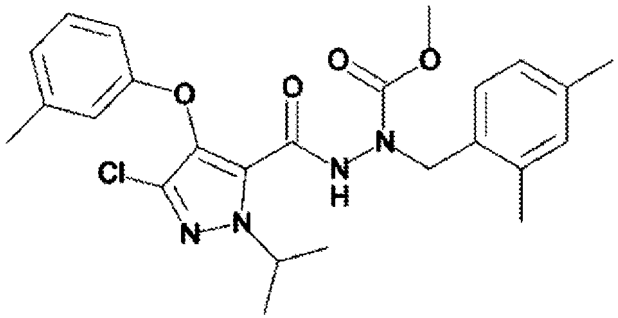 | VISCOUS OIL | 2.08min | |
| c-72 | 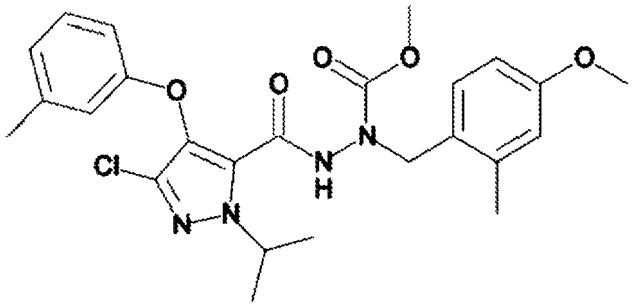 | VISCOUS OIL | 1.96min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-73 | 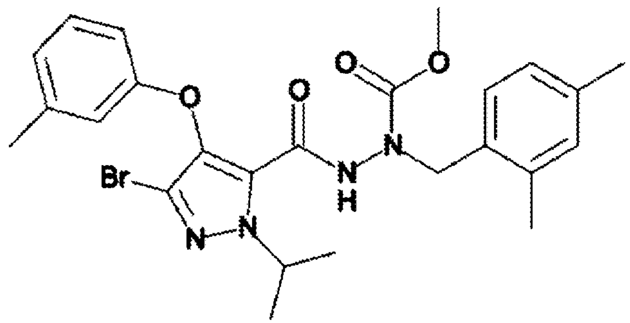 | VISCOUS OIL | 2.08min | |

[Table 19]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-74 | 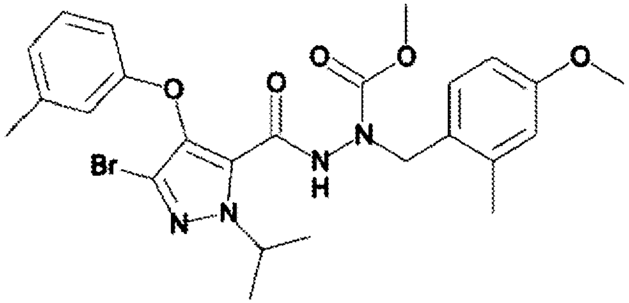 | VISCOUS OIL | 1.96min | |
| c-75 | 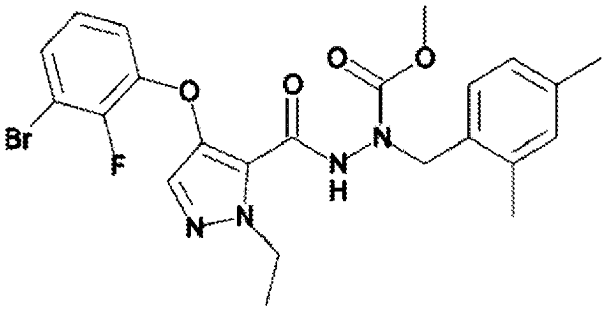 | m. p. 115-118°C | 1.92min | |
| c-76 | 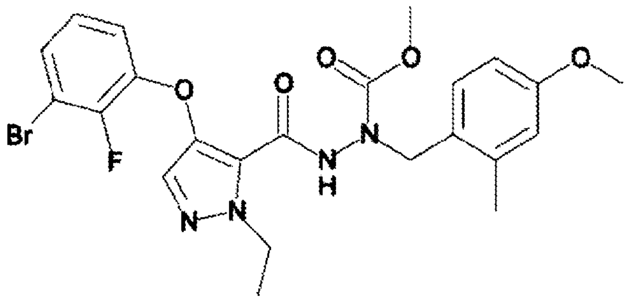 | VISCOUS OIL | 1.78min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-77 | 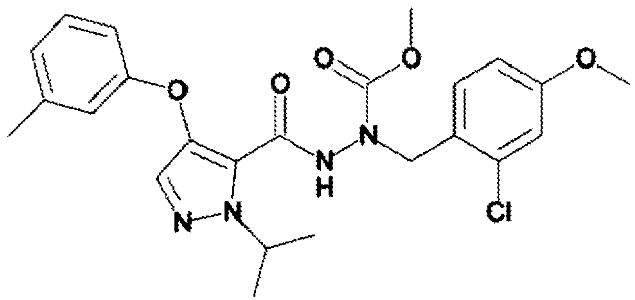 | VISCOUS OIL | 1.83min | |

[Table 20]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-78 | 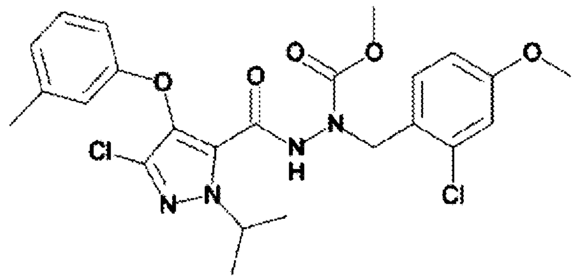 | VISCOUS OIL | 1.95min | |
| c-79 | 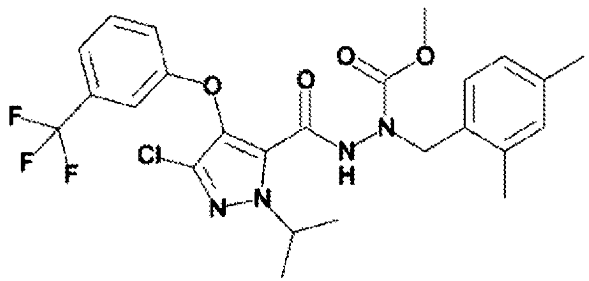 | m. p. 104-106°C | 2.05min | |
| c-80 | 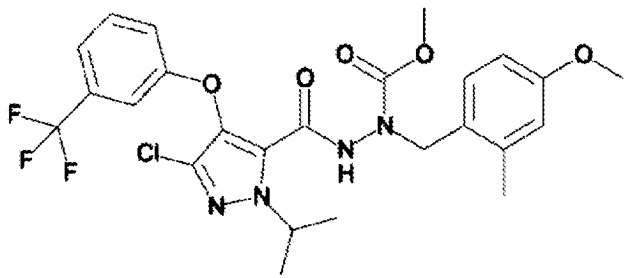 | VISCOUS OIL | 1.93min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-81 | 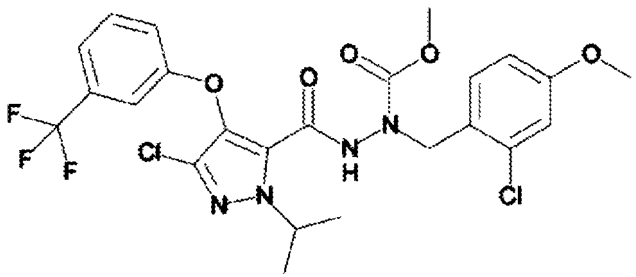 | VISCOUS OIL | 1.97min | |

[Table 21]

| TABLE 1(CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-82 | 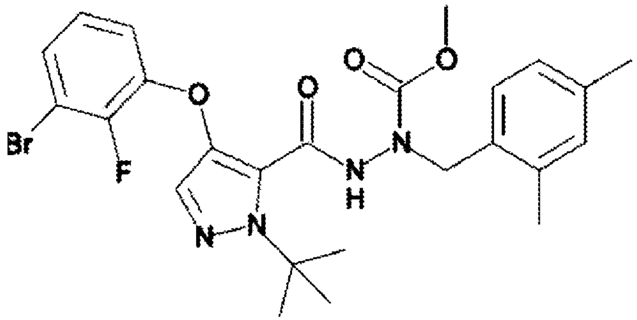 | m. p. 140-141°C | 1.96min | |
| c-83 | 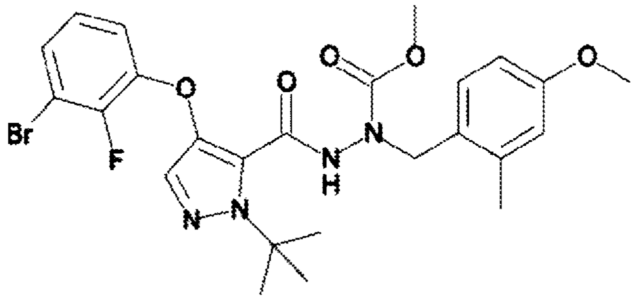 | m. p. 109-110°C | 1.85min | |
| c-84 | 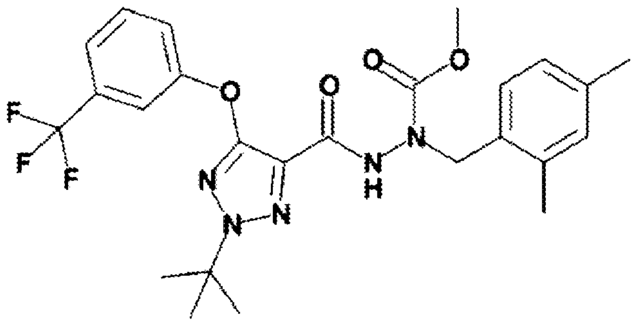 | m.p. 136-138°C | 1.98min | |

(continued)

| TABLE 1(CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-85 | 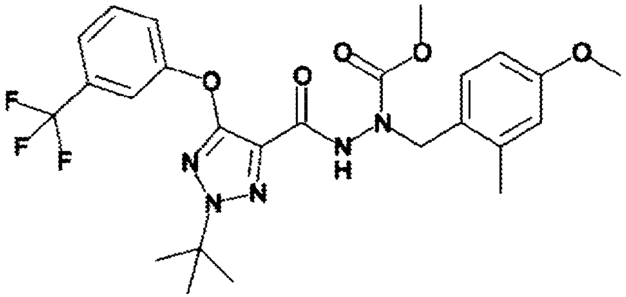 | m.p. 135-137°C | 1.88min | |

[Table 22]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-86 | 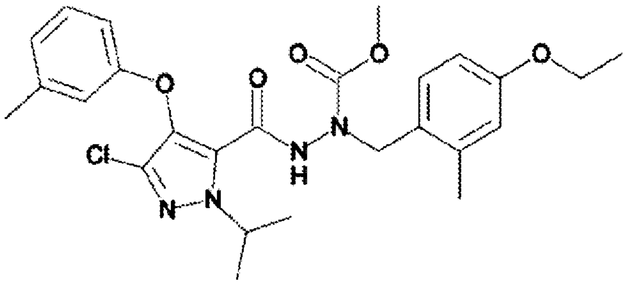 | m. p. 103-104°C | | |
| c-87 | 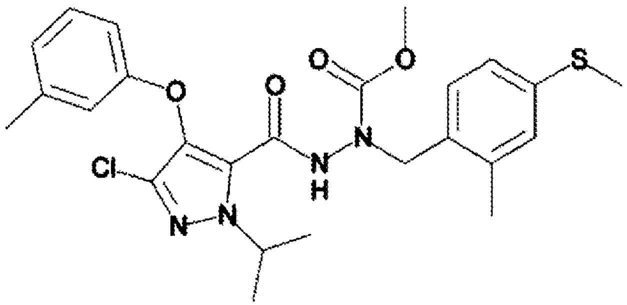 | AMORPHOUS | 2.04min | |
| c-88 | 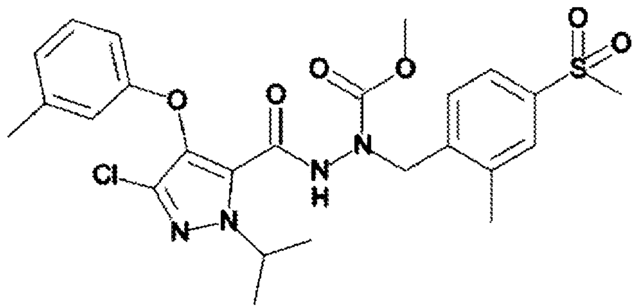 | AMORPHOUS | 1.68min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-89 | 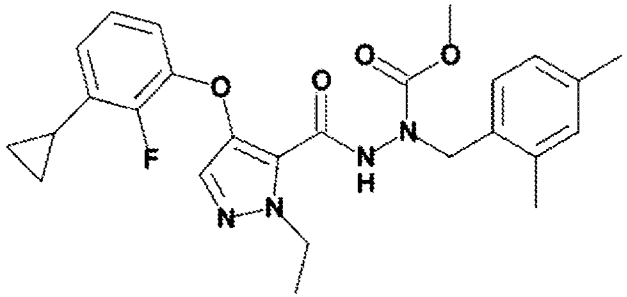 | VISCOUS OIL | 1.93min | |

[Table 23]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-90 | 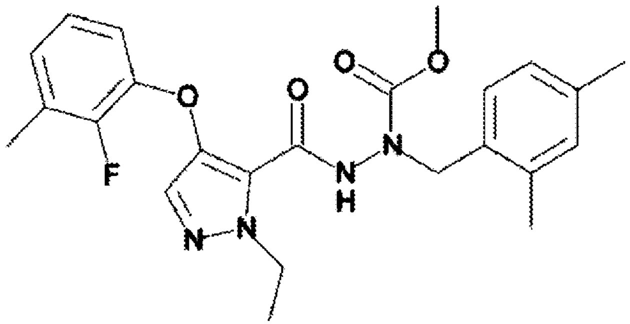 | VISCOUS OIL | 1.85min | |
| c-91 | 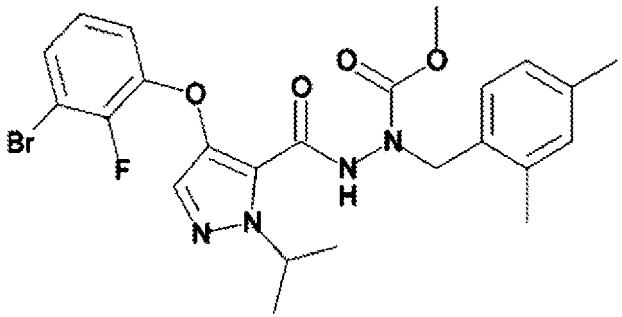 | m. p. 103-105°C | 1.99min | |
| c-92 | 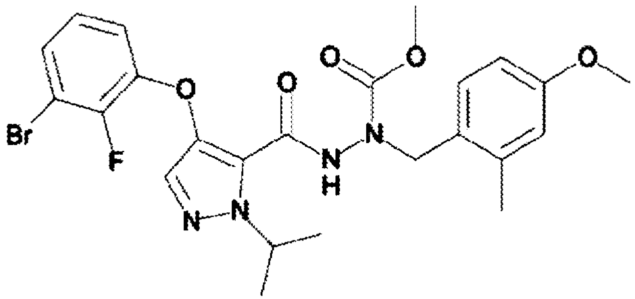 | VISCOUS OIL | 1.86min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-93 | 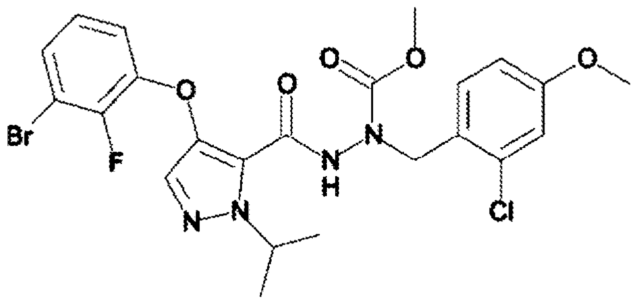 | VISCOUS OIL | 1.90min | |

[Table 24]

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-94 | 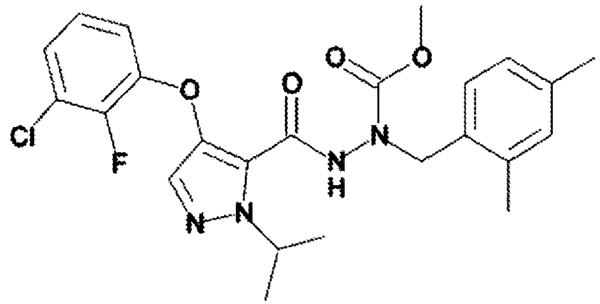 | VISCOUS OIL | 1.96min | |
| c-95 | 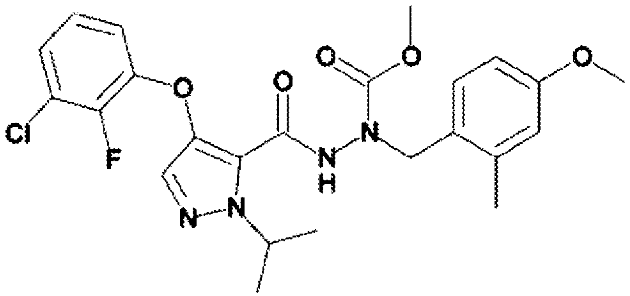 | VISCOUS OIL | 1.83min | |
| c-96 | 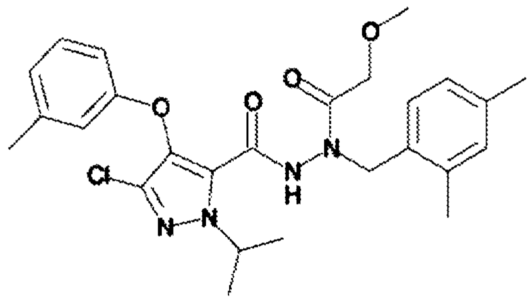 | m.p. 193-124°C | 1.92min | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-97 | 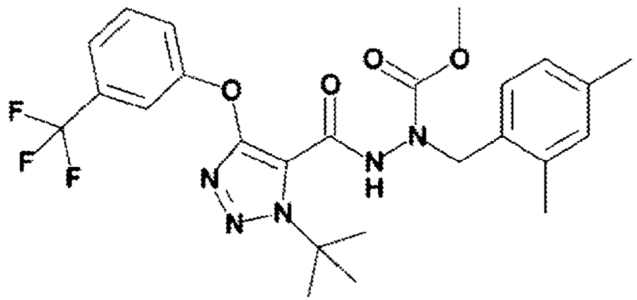 | AMORPHOUS | 1.94min | |

[Table 25]

TABLE 1 (CONTINUED)

| COMPOU-ND NO. | CHEMICAL FORMULA | PROPERTY | LCMS (SQD2) RETENTION TIME | LCMS (QDa) RETENTION TIME |
|---|---|---|---|---|
| c-98 | 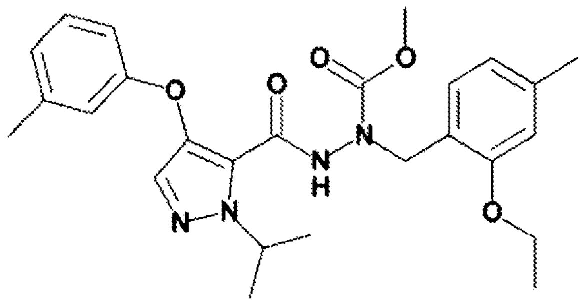 | VISCOUS OIL | 1.98min | |
| c-99 | 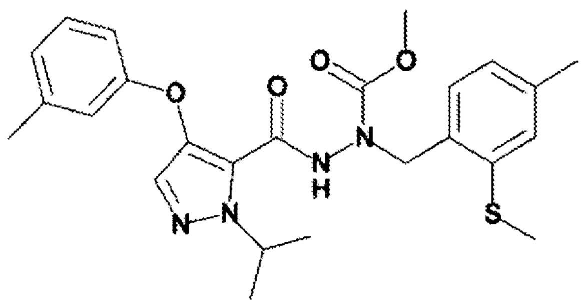 | VISCOUS OIL | 1.96min | |
| c-100 | 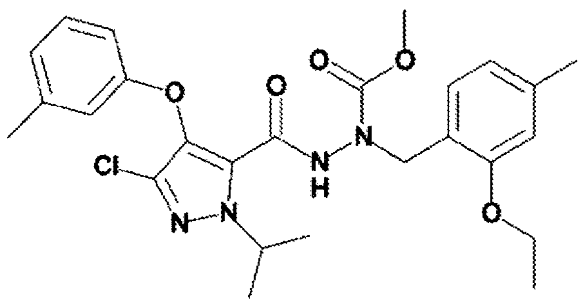 | VISCOUS OIL | 2.07min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-101 | 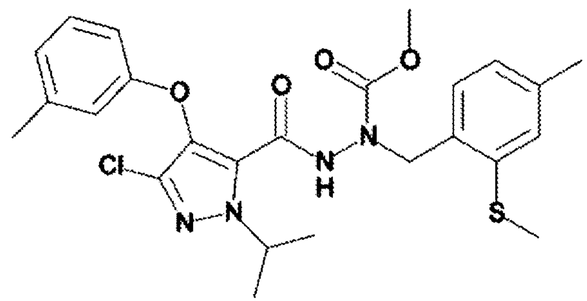 | VISCOUS OIL | 2 .05min | |

[Table 26]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-102 | 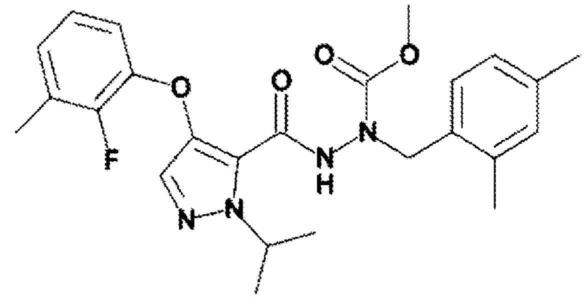 | VISCOUS OIL | | 1.96min |
| c-103 | 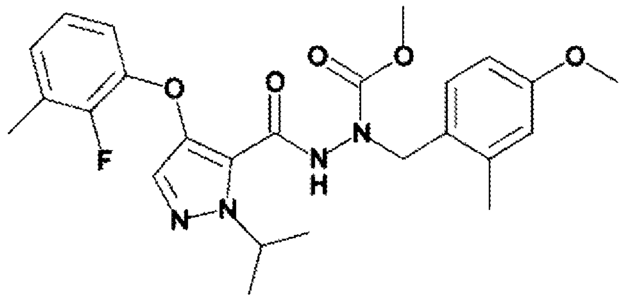 | VISCOUS OIL | | 1.82min |
| c-104 | 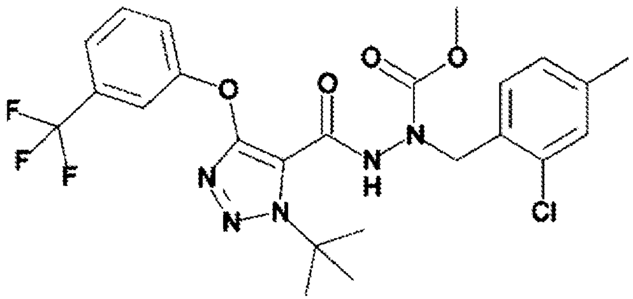 | AMORPHOUS | 1.93min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-105 | 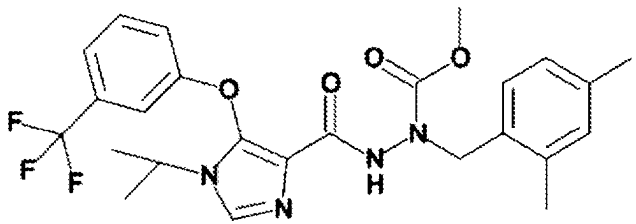 | m.p. 77-80°C | 1.86min | |

[Table 27]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-106 | 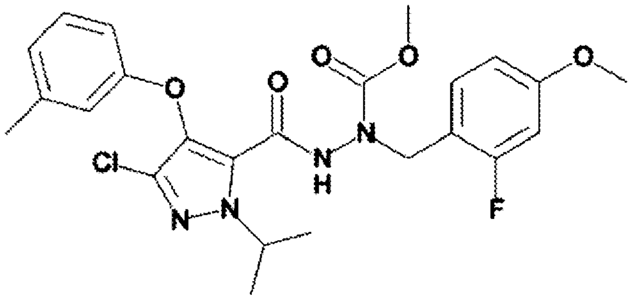 | VISCOUS OIL | 1.95min | |
| c-107 | 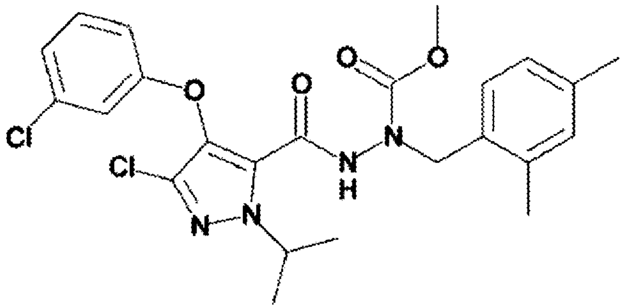 | VISCOUS OIL | 2.13min | |
| c-108 | 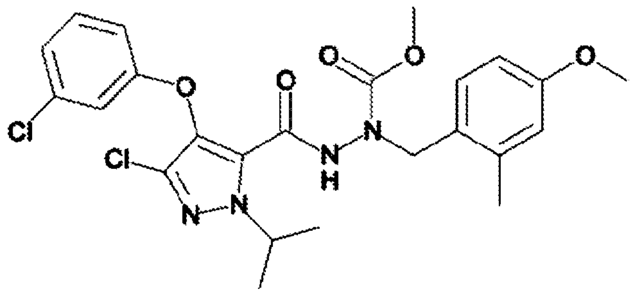 | VISCOUS OIL | 2.00min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-109 | 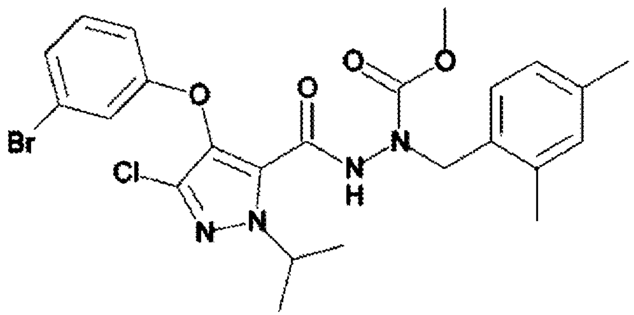 | VISCOUS OIL | 2.15min | |

[Table 28]

| TABLE 1(CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-110 | 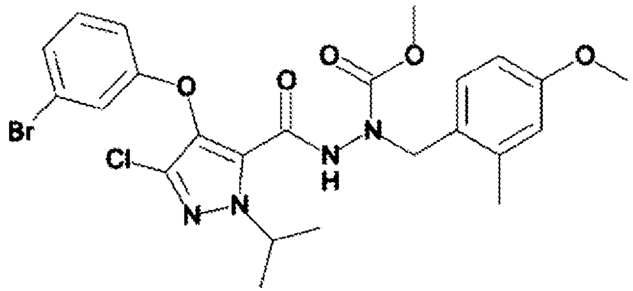 | VISCOUS OIL | 2.02min | |
| c-111 | 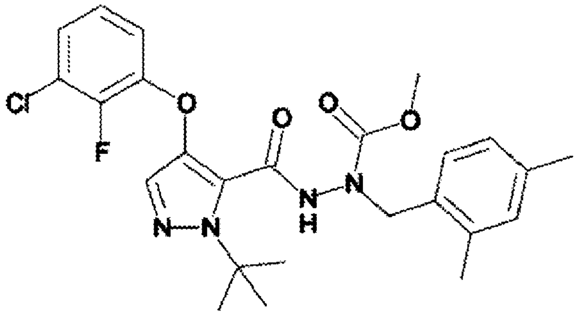 | m.p. 157-160°C | 1.96min | |
| c-112 | 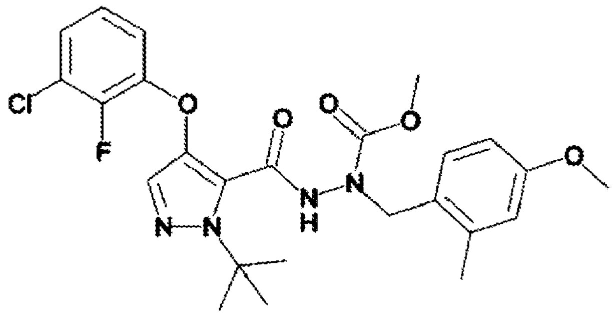 | m.p. 133-135°C | 1.83min | |

(continued)

| TABLE 1(CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-113 | 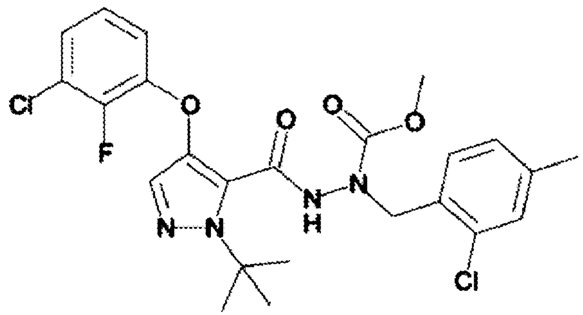 | m.p. 137-140°C | 1.96min | |

[Table 29]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-114 | 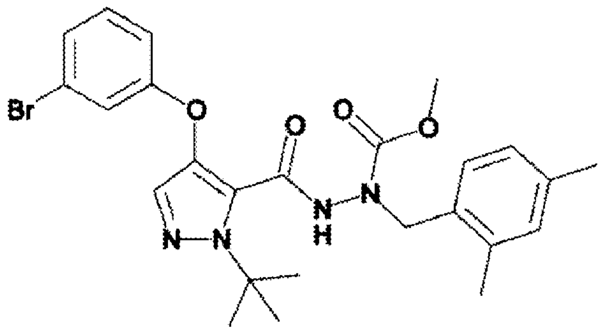 | m.p. 120-124°C | 2.04min | |
| c-115 | 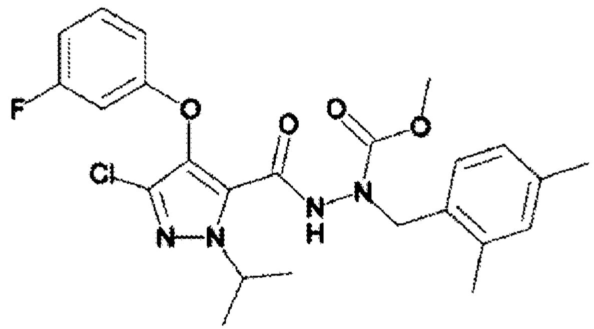 | m.p. 104-106°C | 2.03min | |
| c-116 | 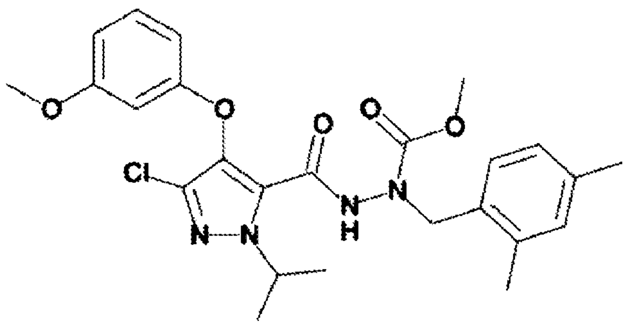 | VISCOUS OIL | 2.03min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-117 | 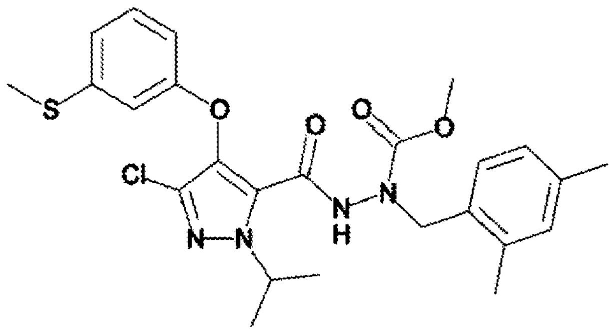 | VISCOUS OIL | 2.05min | |

[Table 30]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-118 | 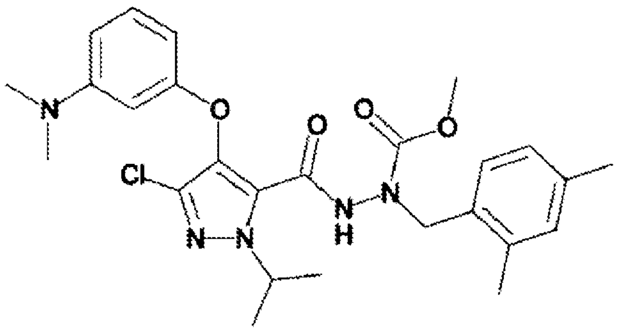 | VISCOUS OIL | 2.04min | |
| c-119 | 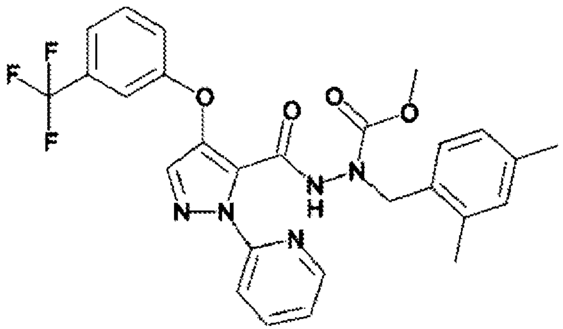 | VISCOUS OIL | | 1.89min |
| c-120 | 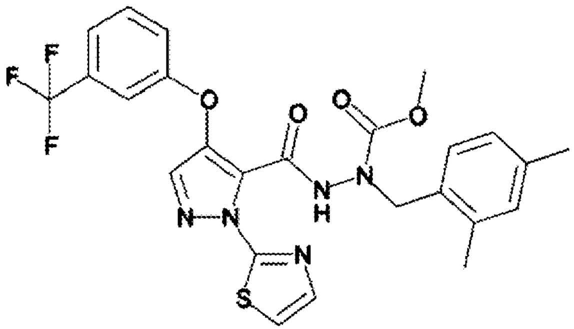 | VISCOUS OIL | | 1.83min |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-121 | 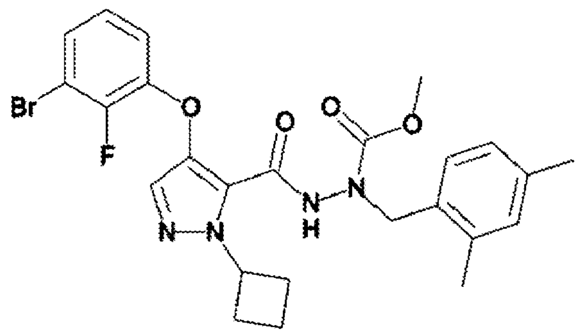 | VISCOUS OIL | | 2.00min |

[Table 31]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-122 | 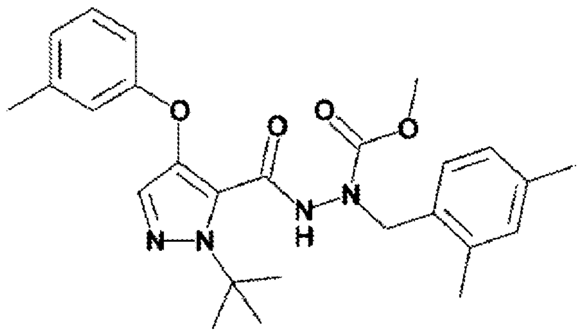 | m.p. 148-150°C | | |
| c-123 | 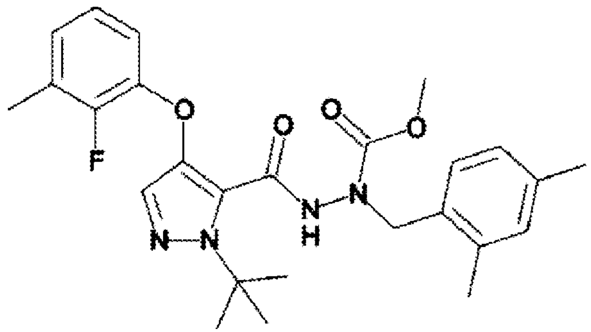 | m.p. 180-163°C | | |
| c-124 | 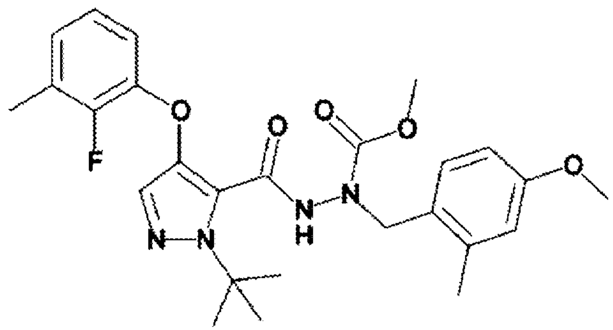 | m.p. 120-123°C | | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-125 | 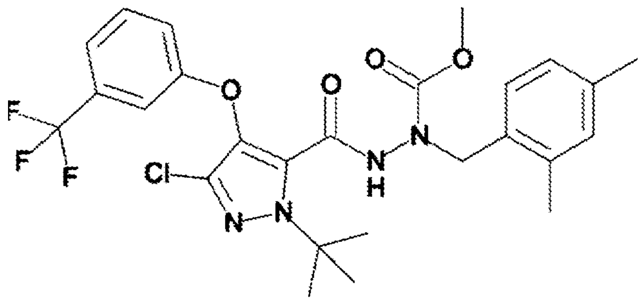 | AMORPHOUS | 2.06min | |

[Table 32]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-126 | 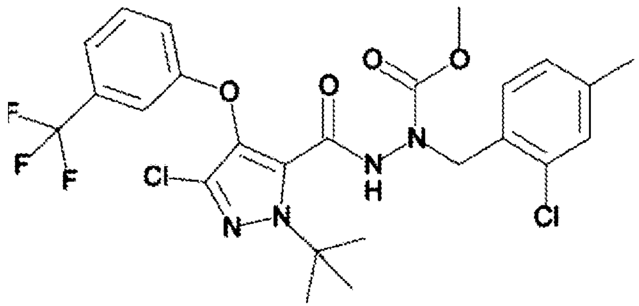 | AMORPHOUS | 2.07min | |
| c-127 | 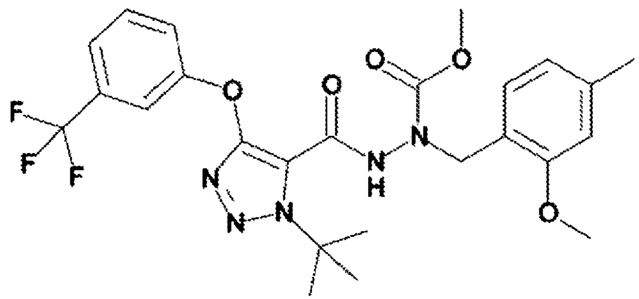 | m.p. 107-109°C | | |
| c-128 | 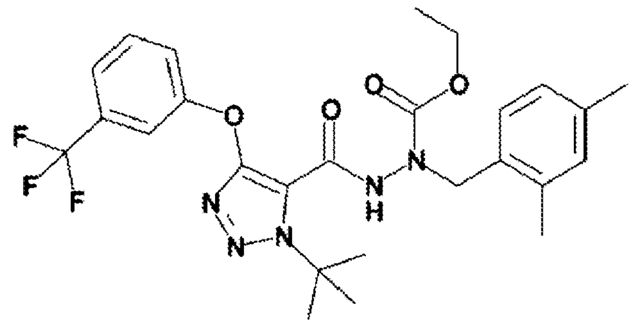 | VISCOUS OIL | 1.98min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-129 | 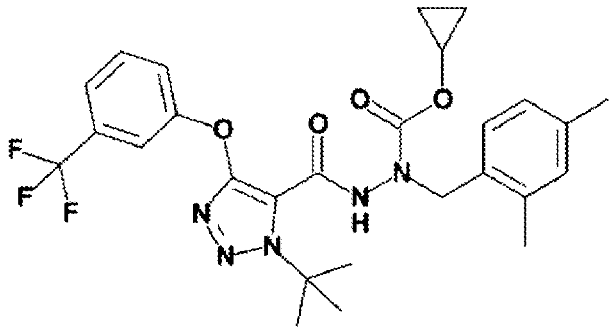 | VISCOUS OIL | 1.99min | |

[Table 33]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-130 | 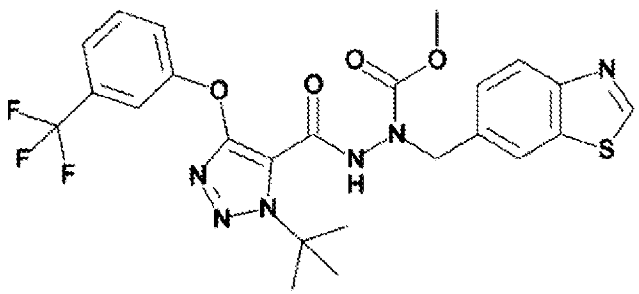 | VISCOUS OIL | 1.63min | |
| c-131 | 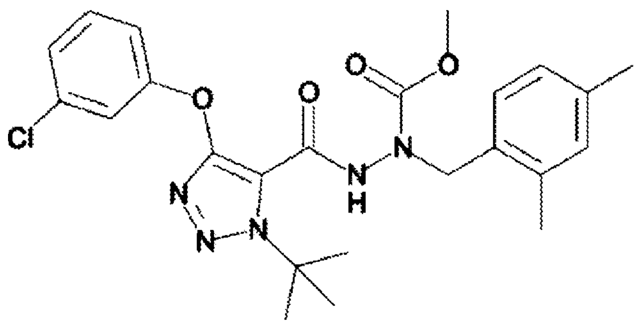 | AMORPHOUS | 1.88min | |
| c-132 | 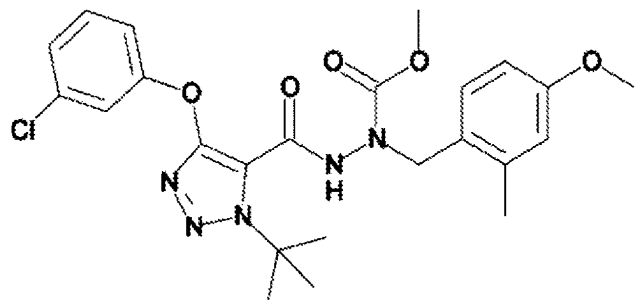 | AMORPHOUS | 1.75min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-133 | 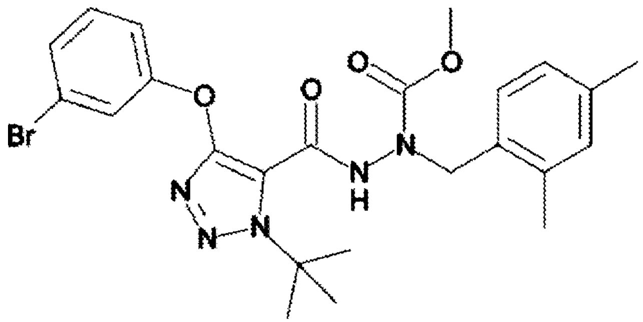 | AMORPHOUS | 1.90min | |

[Table 34]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-134 | 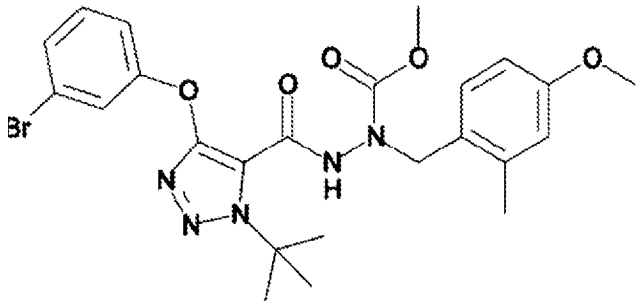 | AMORPHOUS | 1.79min | |
| c-135 | 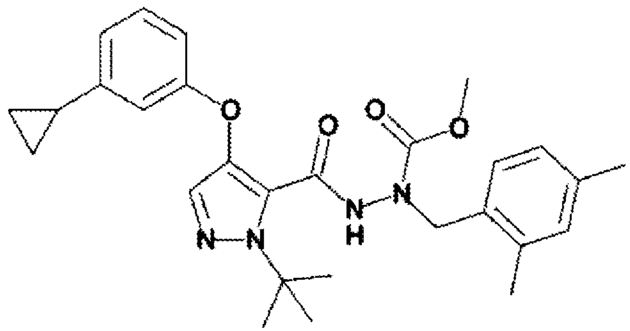 | m.p. 135-138°C | | |
| c-136 | 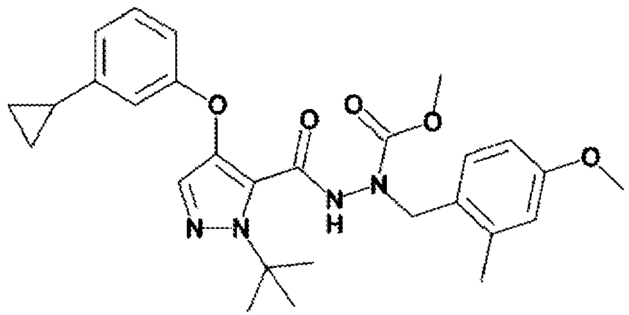 | VISCOUS OIL | 1.90min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-137 | 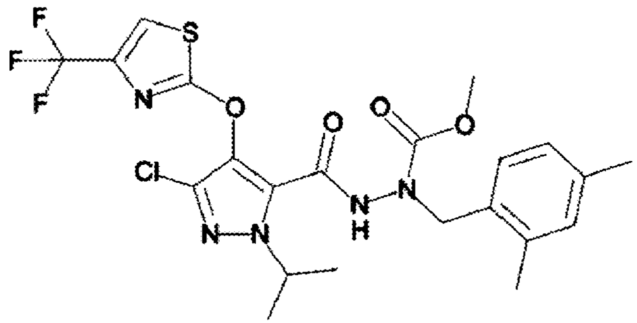 | m.p. 50-56°C | 1.92min | |

[Table 35]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-138 | 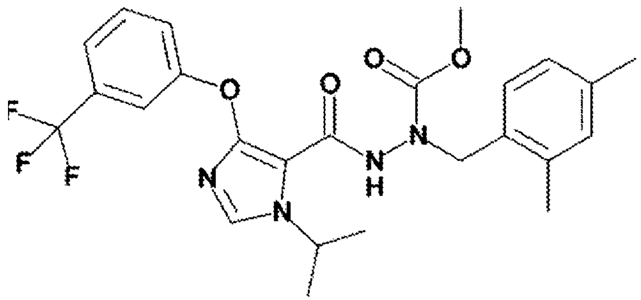 | VISCOUS OIL | 1.77min | |
| c-139 | 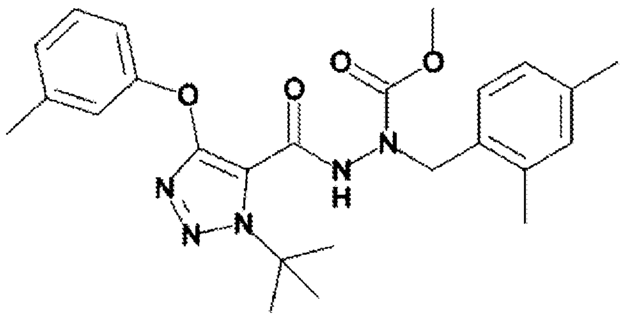 | AMORPHOUS | 1.87min | |
| c-140 | 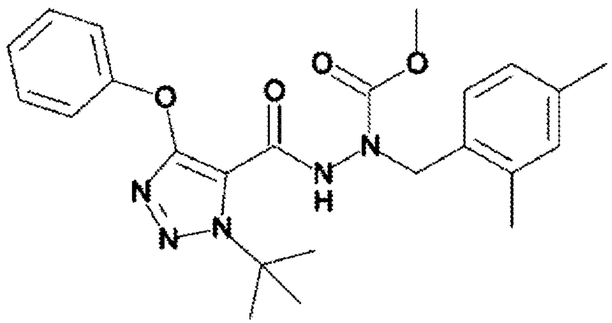 | AMORPHOUS | 1.79min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-141 | 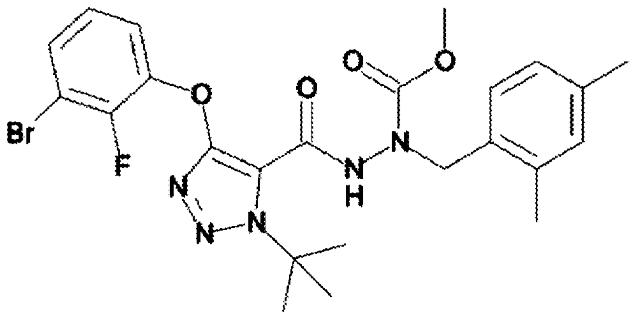 | AMORPHOUS | 1.90min | |

[Table 36]

TABLE 1 (CONTINUED)

| COMPOUND NO. | CHEMICAL FORMULA | PROPERTY | LCMS (SQD2) RETENTION TIME | LCMS (QDa) RETENTION TIME |
|---|---|---|---|---|
| c-142 | 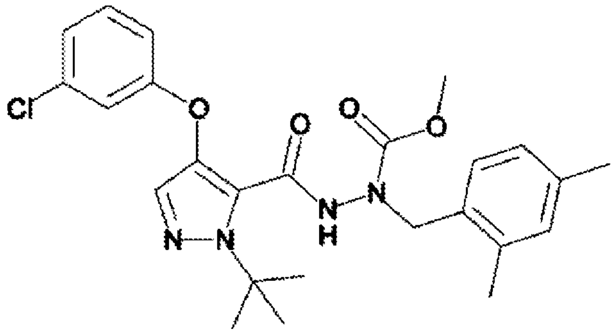 | m.p. 149-151°C | | |
| c-143 | 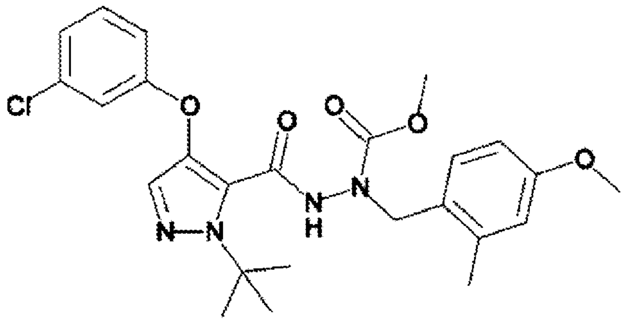 | m.p. 120-123°C | | |
| c-144 | 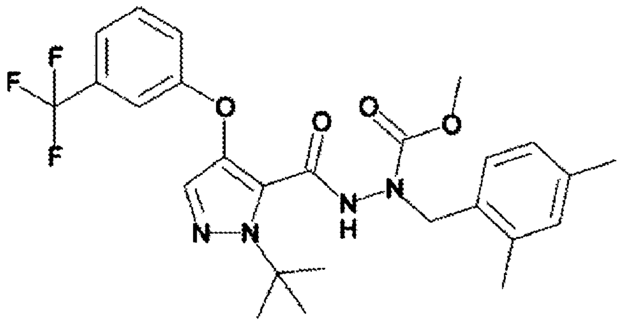 | m. p. 123-125°C | | |

(continued)

| TABLE 1 (CONTINUED) | | | | |
|---|---|---|---|---|
| COMPOUND NO. | CHEMICAL FORMULA | PROPERTY | LCMS (SQD2) RETENTION TIME | LCMS (QDa) RETENTION TIME |
| c-145 | 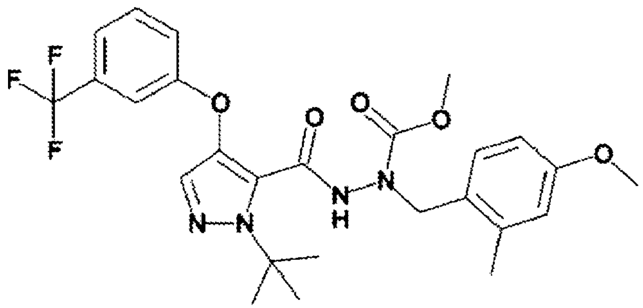 | m.p. 113-116°C | | |

[Table 37]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-146 | 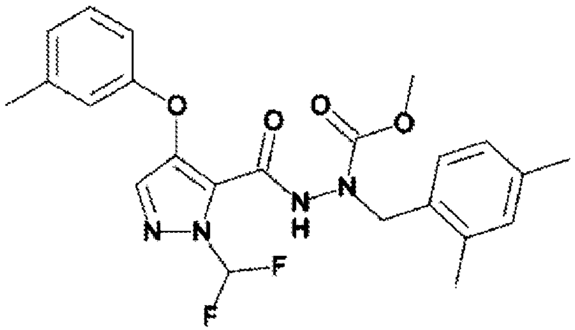 | VISCOUS OIL | 1.85min | |
| c-147 | 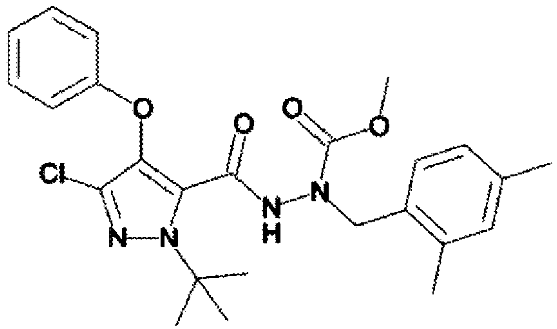 | VISCOUS OIL | 1.97min | |
| c-148 | 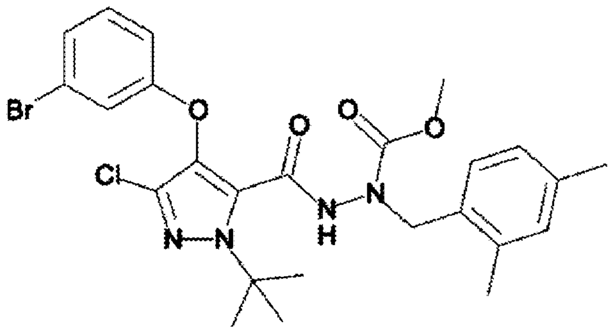 | VISCOUS OIL | 2.09min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-149 | 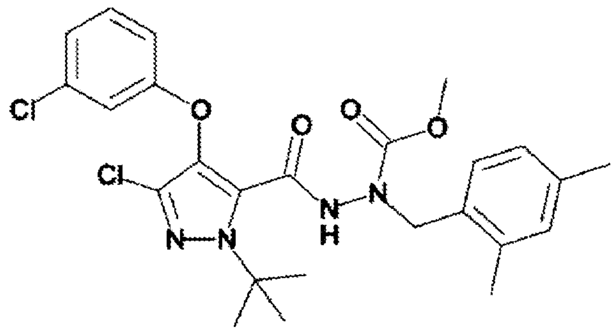 | VISCOUS OIL | 2.07min | |

[Table 38]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-150 | 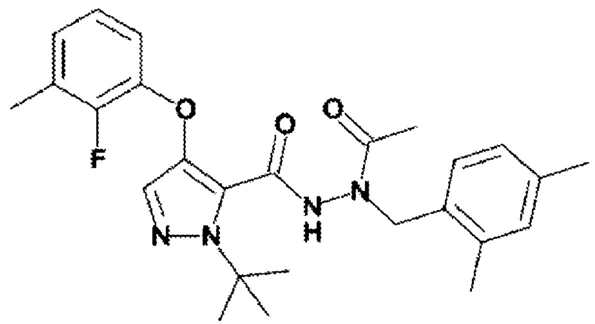 | m.p. 167-169°C | | |
| c-151 | 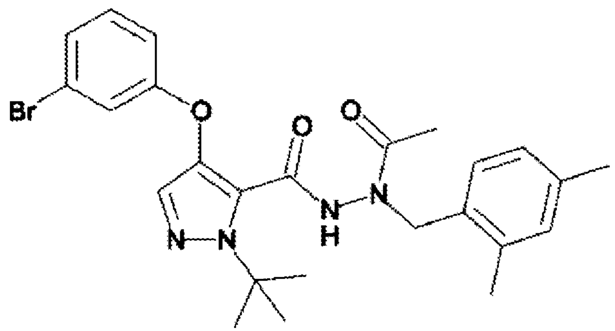 | m.p. 120-123°C | | |
| c-152 | 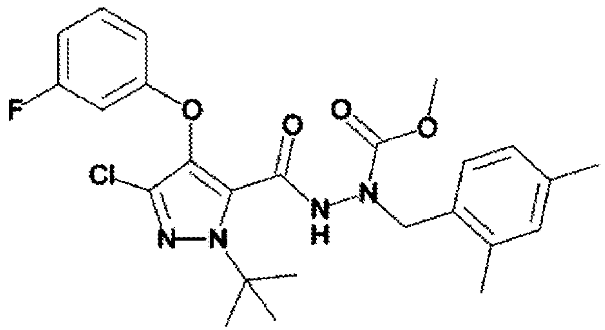 | VISCOUS OIL | 1.98min | |

(continued)

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-153 | 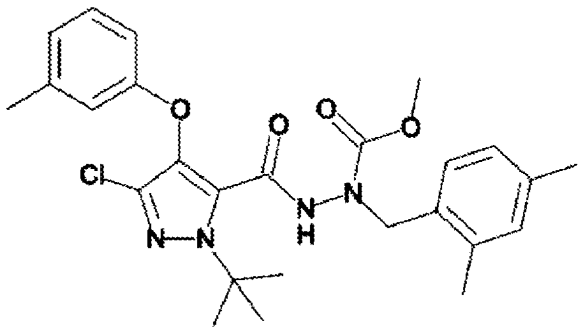 | VISCOUS OIL | 2.04min | |

[Table 39]

| TABLE 1 (CONTINUED) | | | | |
|---|---|---|---|---|
| COMPOU-ND NO. | CHEMICAL FORMULA | PROPERTY | LCMS (SQD2) RETENTION TIME | LCMS (QDa) RETENTION TIME |
| c-154 | 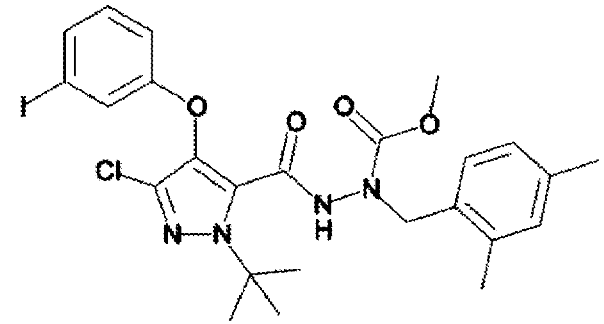 | VISCOUS OIL | 2.12min | |
| c-155 | 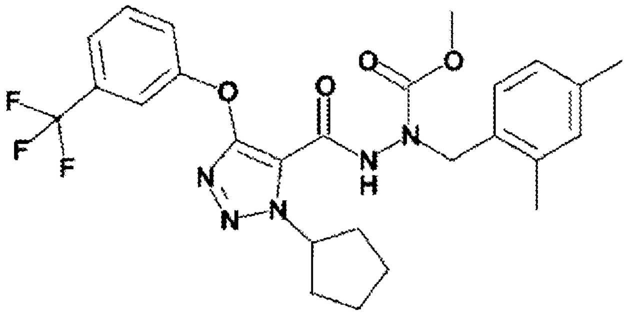 | VISCOUS OIL | 2.02min | |
| c-156 | 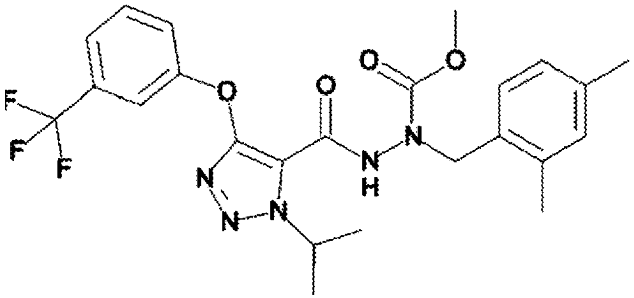 | VISCOUS OIL | 1.91min | |

Compounds that may be intermediate products are shown in the following Tables 3, 4 and 5. The LCMS (SQD2) retention times are indicated with the chemical formulae of the intermediate products.

[Table 40]

| TABLE 3 | | |
|---|---|---|
| COMPOUND NO. | CHEMICAL FORMULA | LCMS(SQD2) RETENTION TIME |
| e-1 | 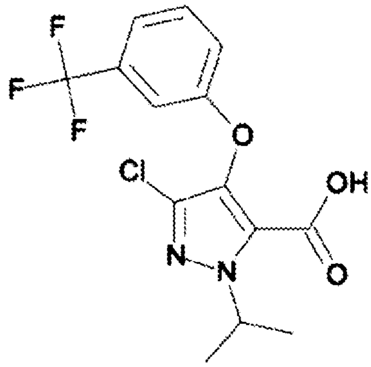 | 1.76min |
| e-2 | 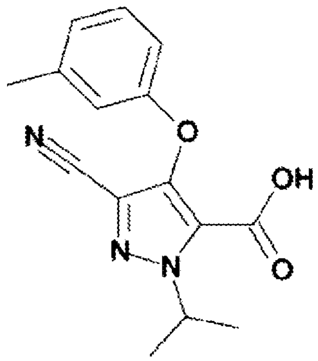 | 1.59min |
| e-3 | 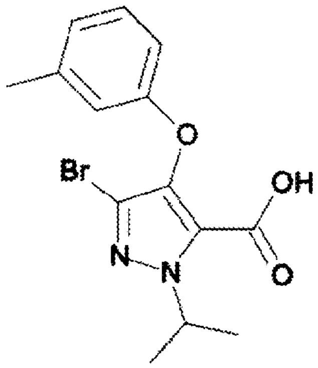 | 1.67min |
| e-4 | 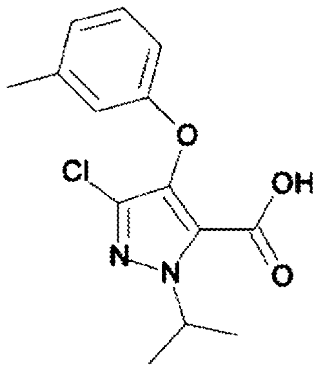 | 2.01min |

[Table 41]

| TABLE 3 (CONT I NUED) | | |
|---|---|---|
| COMPOUND NO. | CHEMICAL FORMULA | LCMS(SQD2) RETENTION TIME |
| e-5 | 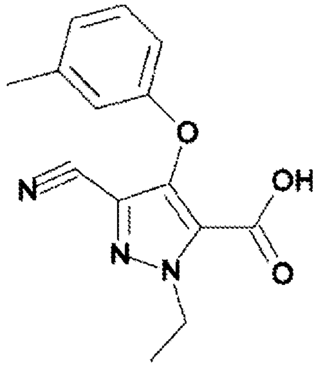 | 1.45min |
| e-6 | 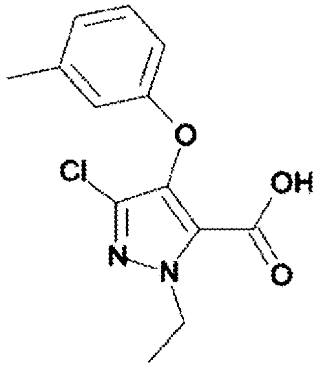 | 1.50min |
| e-7 | 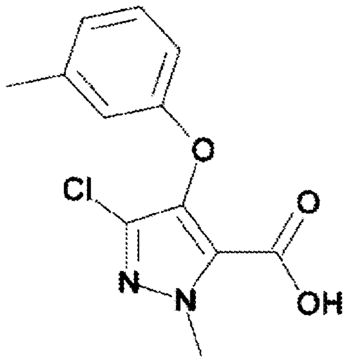 | 1.44min |
| e-8 | 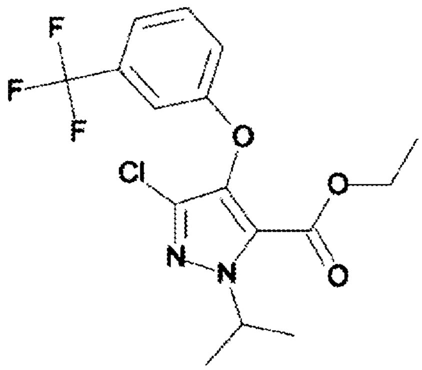 | 2.10min |

[Table 42]

| TABLE 3 (CONT I NUED) | | |
|---|---|---|
| COMPOUND NO. | CHEMICAL FORMULA | LCMS(SQD2) RETENTION TIME |
| e-9 | 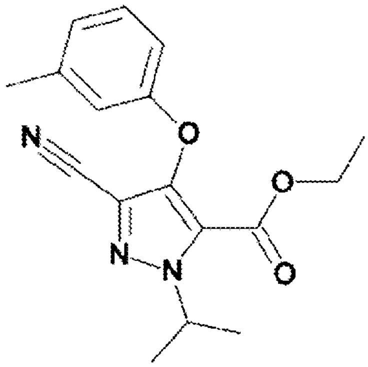 | 1.93min |
| e-10 | 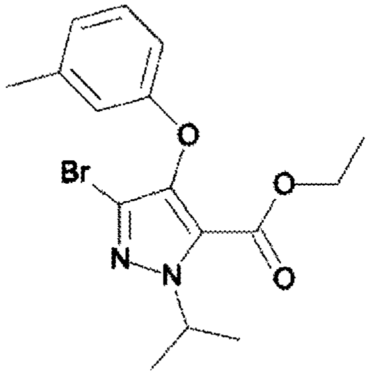 | 2.01min |
| e-11 | 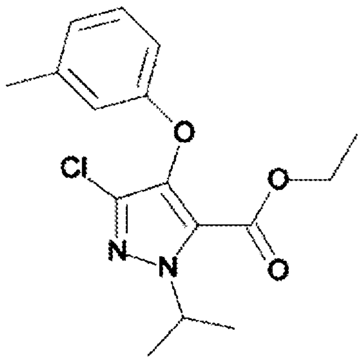 | 2.01min |
| e-12 | 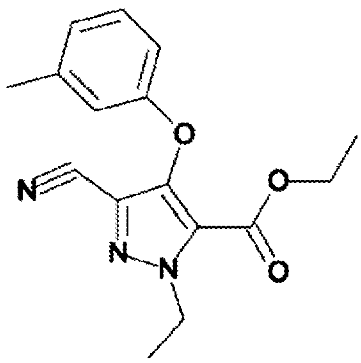 | 1.82min |

[Table 43]

| TABLE 3 (CONTINUED) | | |
|---|---|---|
| COMPOUND NO. | CHEMICAL FORMULA | LCMS (SQD2) RETENTION TIME |
| e-13 | 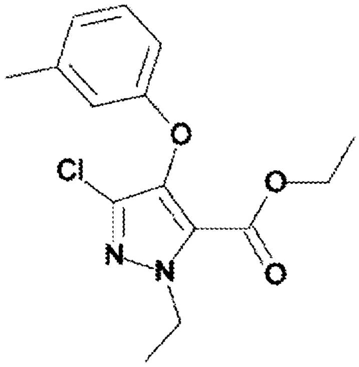 | 1.92min |
| e-14 | 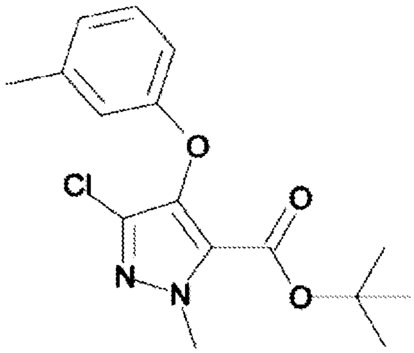 | 2.01min |

[Table 44]

| TABLE 3 (CONT I NUED) | | |
|---|---|---|
| COMPOUND NO. | CHEMICAL FORMULA | LCMS(SQD2) RETENTION TIME |
| e-15 | 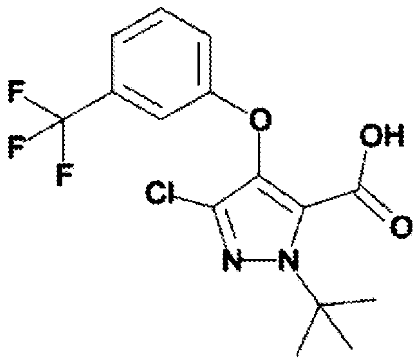 | 1.83min |
| e-16 | 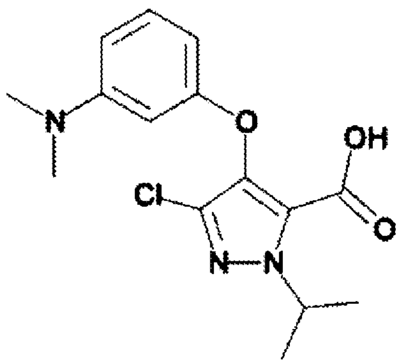 | 1.45min |

(continued)

| TABLE 3 (CONT I NUED) | | |
|---|---|---|
| COMPOUND NO. | CHEMICAL FORMULA | LCMS(SQD2) RETENTION TIME |
| e-17 | 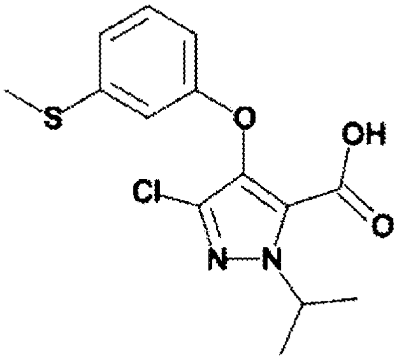 | 1.70min |
| e-18 | 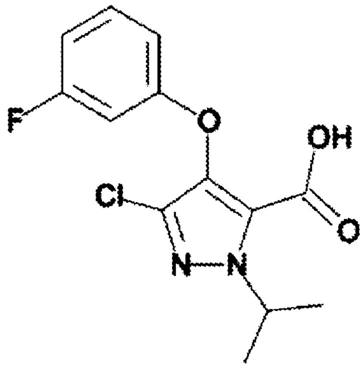 | 1.60min |

[Table 45]

| TABLE 3 (CONT I NUED) | | |
|---|---|---|
| COMPOUND NO. | CHEMICAL FORMULA | LCMS(SQD2) RETENTION TIME |
| e-19 | 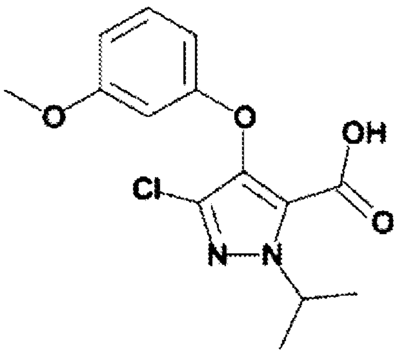 | 1.55min |
| e-20 | 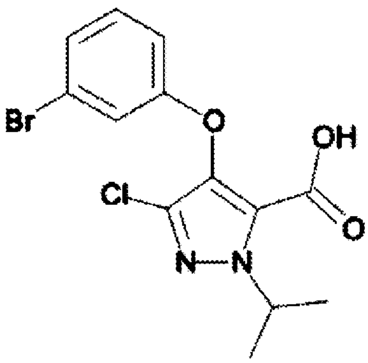 | 1.76min |

(continued)

| TABLE 3 (CONT I NUED) | | |
|---|---|---|
| COMPOUND NO. | CHEMICAL FORMULA | LCMS(SQD2) RETENTION TIME |
| e-21 | 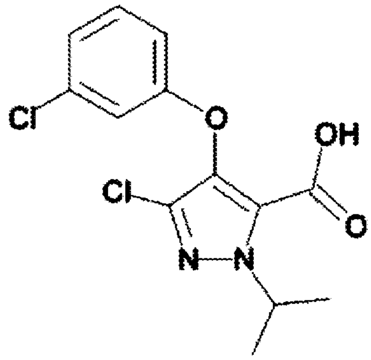 | 1.73min |
| e-22 | 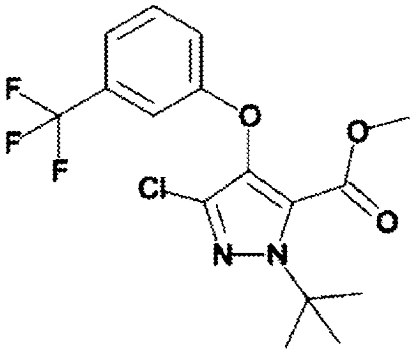 | 2.04min |

[Table 46]

| TABLE 3 (CONTINUED) | | |
|---|---|---|
| COMPOUND NO. | CHEMICAL FORMULA | LCMS(SQD2) RETENTION TIME |
| e-23 | 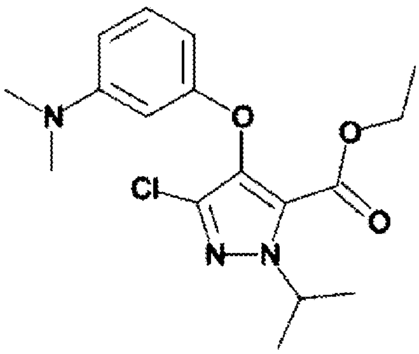 | 1.95min |
| e-24 | 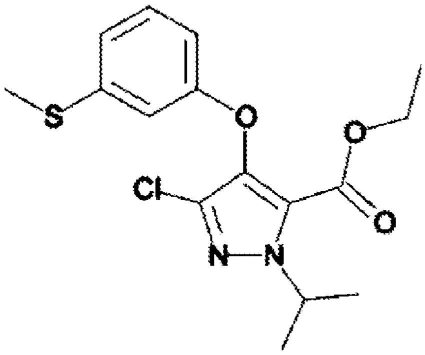 | 2.02min |

(continued)

| TABLE 3 (CONTINUED) | | |
|---|---|---|
| COMPOUND NO. | CHEMICAL FORMULA | LCMS(SQD2) RETENTION TIME |
| e-25 | 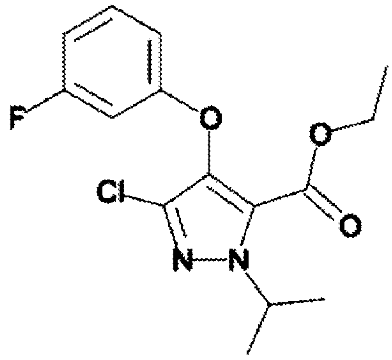 | 1.90min |
| e-26 | 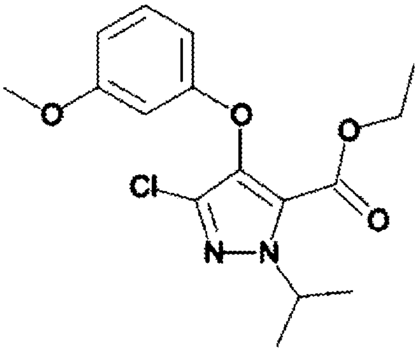 | 1.94min |

[Table 47]

| TABLE 3 (CONTINUED) | | |
|---|---|---|
| COMPOUND NO. | CHEMICAL FORMULA | LCMS(SQD2) RETENTION TIME |
| e-27 | 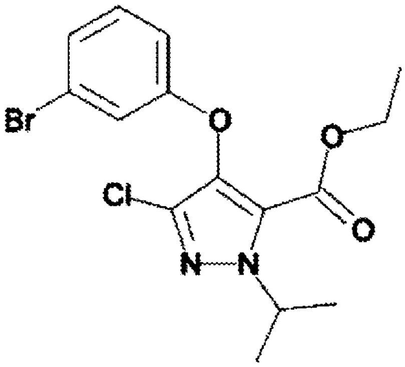 | 2.12min |

(continued)

| TABLE 3 (CONTINUED) | | |
|---|---|---|
| COMPOUND NO. | CHEMICAL FORMULA | LCMS(SQD2) RETENTION TIME |
| e-28 | 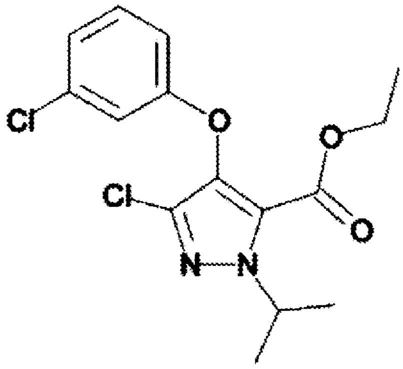 | 2.10min |
| e-29 | 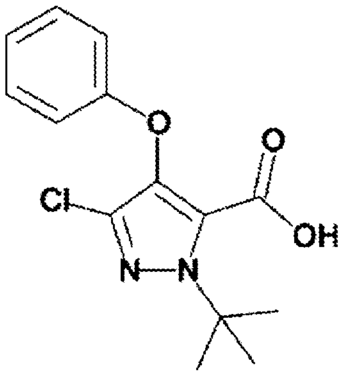 | 1.58min |
| e-30 | 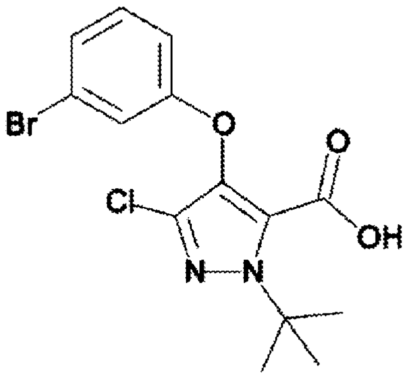 | 1.79min |

[Table 48]

| TABLE 3 (CONTINUED) | | |
|---|---|---|
| COMPOUND NO. | CHEMICAL FORMULA | LCMS(SQD2) RETENTION TIME |
| e-31 | 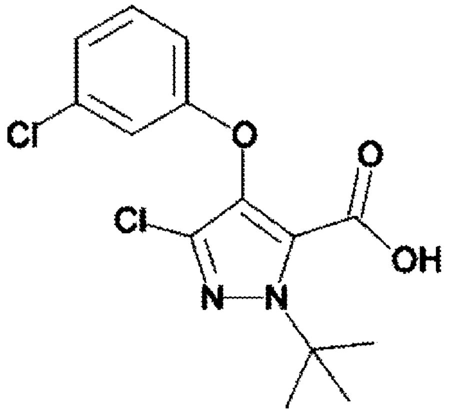 | 1.75min |
| e-32 | 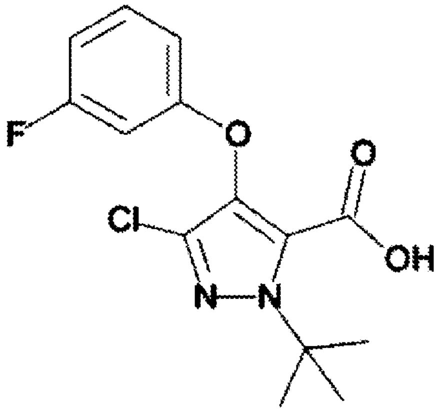 | 1.64min |
| e-33 | 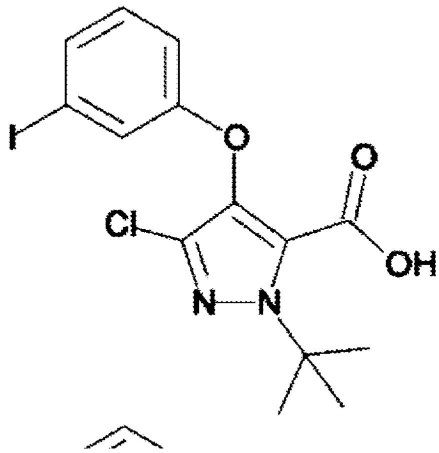 | 1.84min |

(continued)

| TABLE 3 (CONTINUED) | | |
|---|---|---|
| COMPOUND NO. | CHEMICAL FORMULA | LCMS(SQD2) RETENTION TIME |
| e-34 | 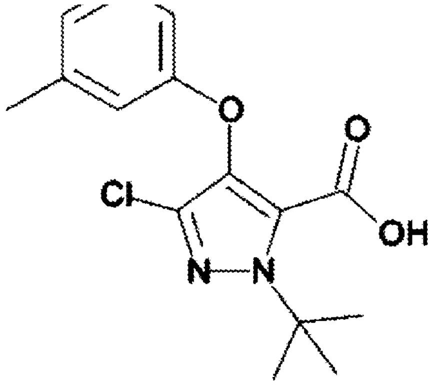 | 1.68min |

[Table 49]

| TABLE 3 (CONTINUED) | | |
|---|---|---|
| COMPOUND NO. | CHEMICAL FORMULA | LCMS (SOD2) RETENTION TIME |
| e-35 | 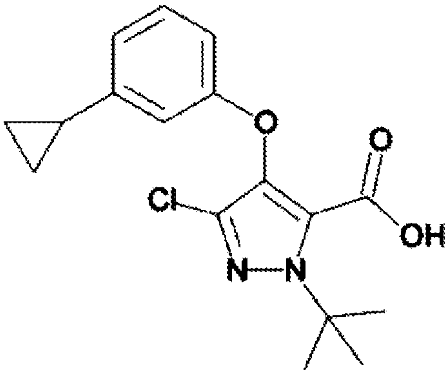 | 1.80min |
| e-36 | 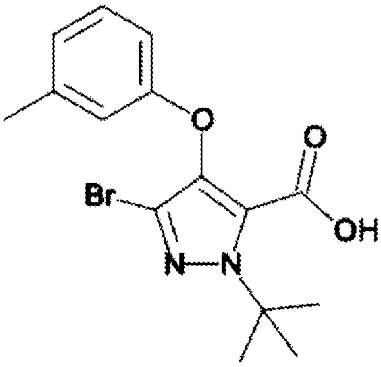 | 1.70min |

(continued)

| TABLE 3 (CONTINUED) | | |
|---|---|---|
| COMPOUND NO. | CHEMICAL FORMULA | LCMS (SOD2) RETENTION TIME |
| e-37 | 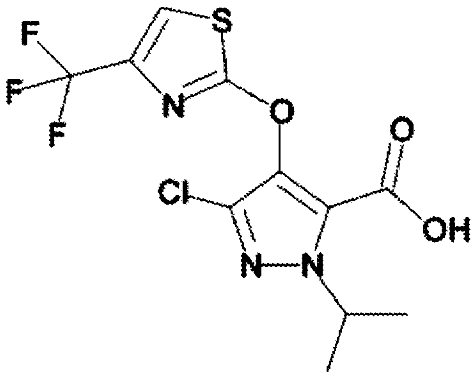 | 1.62min |
| e-38 | 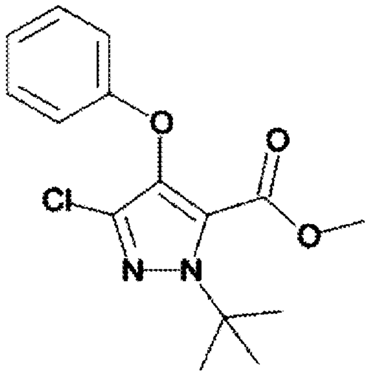 | 1.93min |

[Table 50]

| TABLE 3(CONTINUED) | | |
|---|---|---|
| COMPOUND NO. | CHEMICAL FORMULA | LCMS(SQD2) RETENTION TIME |
| e-39 | 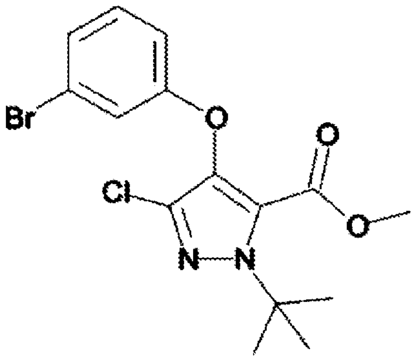 | 2.09min |

(continued)

| TABLE 3(CONTINUED) | | |
|---|---|---|
| COMPOUND NO. | CHEMICAL FORMULA | LCMS(SQD2) RETENTION TIME |
| e-40 | 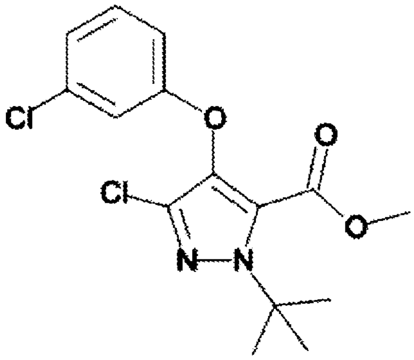 | 2.06min |
| e-41 | 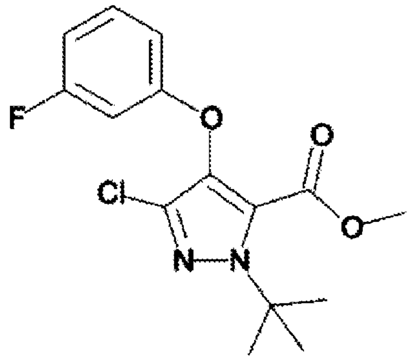 | 1.96min |
| e-42 | 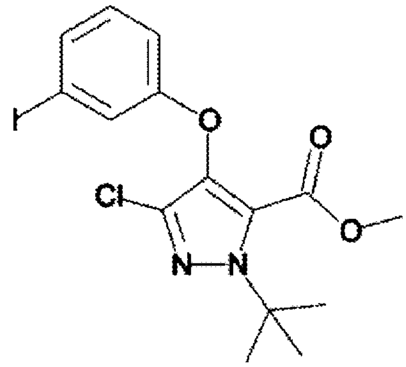 | 2.13min |

[Table 51]

| TABLE 3 (CONTINUED) | | |
|---|---|---|
| COMPOUND NO. | CHEMICAL FORMULA | LCMS (SQD2) RETENTION TIME |
| e-43 | 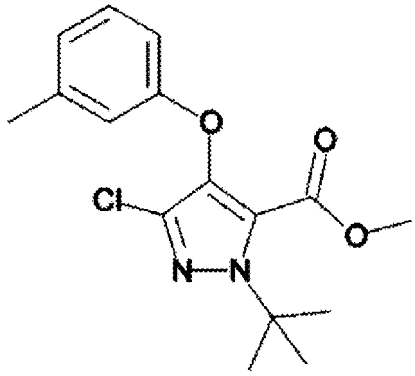 | 2.00min |
| e-44 | 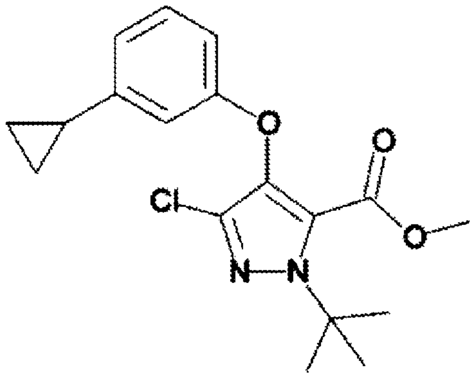 | 2.07min |
| e-45 | 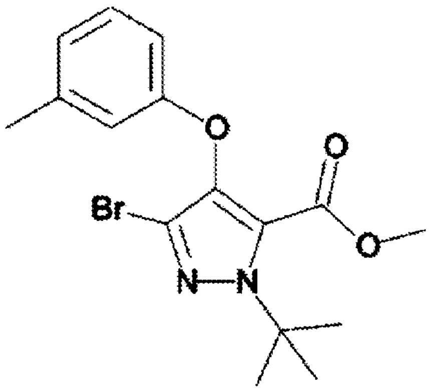 | 2.00min |
| e-46 | 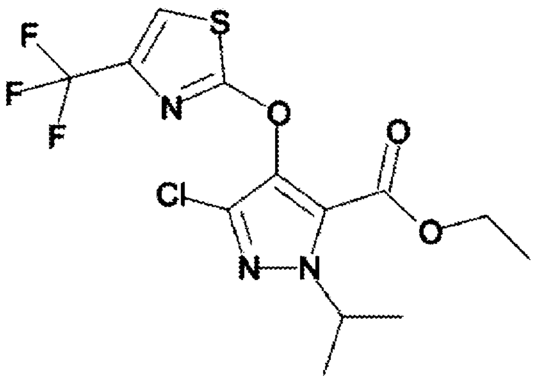 | 1.92min |

[Table 52]

| TABLE 4 | | |
|---|---|---|
| COMPOUND NO. | CHEMICAL FORMULA | LCMS(SQD2) RETENTION TIME |
| d-1 | 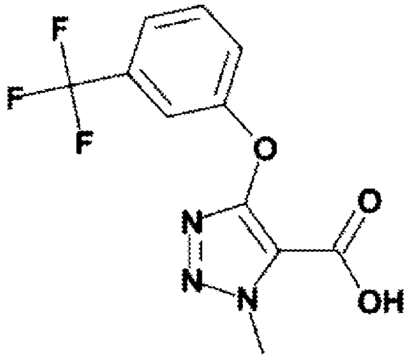 | 1. 19min |
| d-2 | 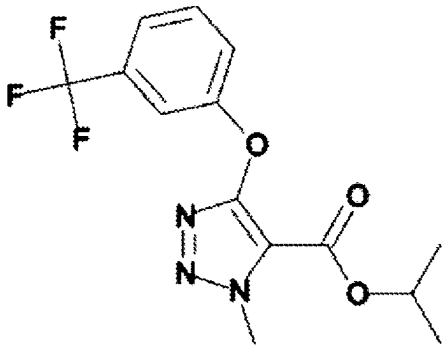 | 1.67min |
| d-3 | 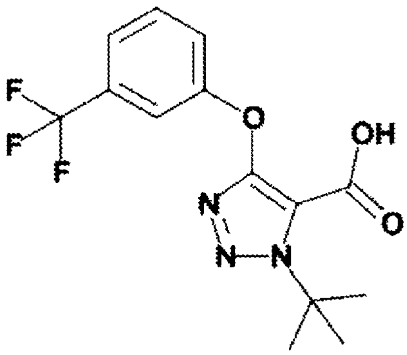 | 1.47min |
| d-4 | 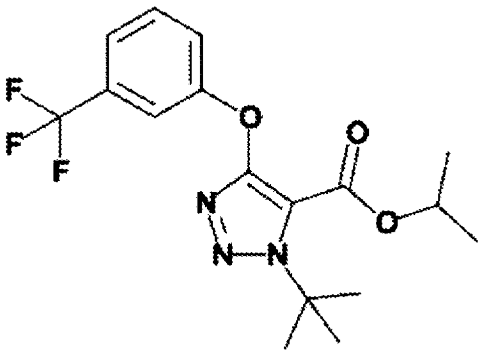 | 2.04min |

[Table 53]

| TABLE 4 (CONTINUED) | | |
|---|---|---|
| COMPOUND NO. | CHEMICAL FORMULA | LCMS(SQD2) RETENTION TIME |
| d-5 | 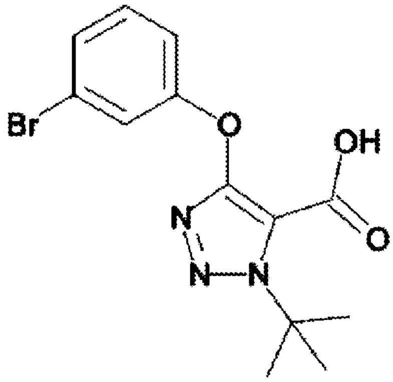 | 1.44min |
| d-6 | 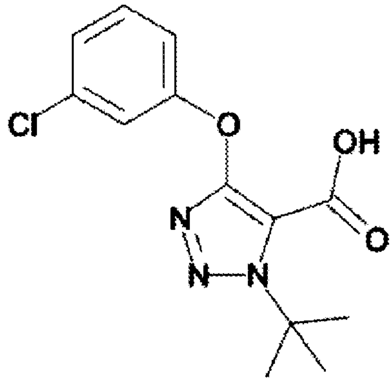 | 1.40min |
| d-7 | 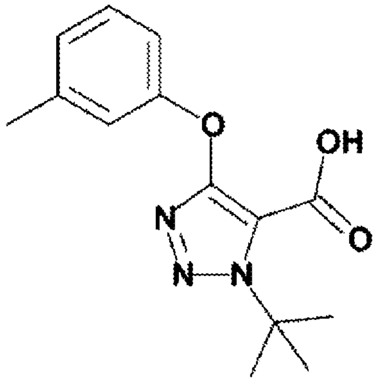 | 1.35min |
| d-8 | 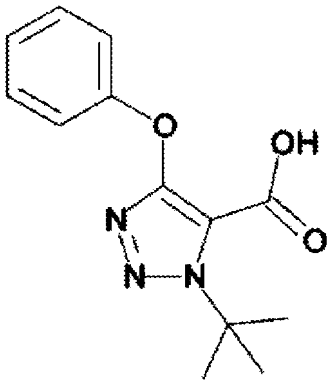 | 1.13min |

[Table 54]

| TABLE 4 (CONTINUED) | | |
|---|---|---|
| COMPOUND NO. | CHEMICAL FORMULA | LCMS(SQD2) RETENTION TIME |
| d-9 | 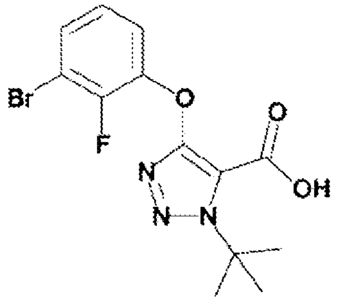 | 1.47min |
| d-10 | 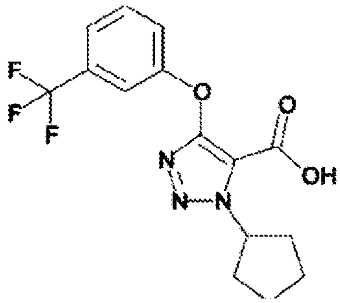 | 1.62min |
| d-11 | 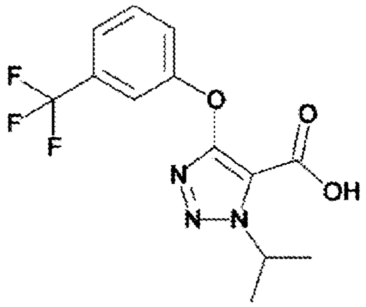 | 1.44min |
| d-12 | 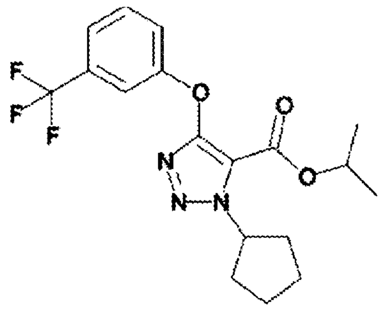 | 2.02min |
| d-13 | 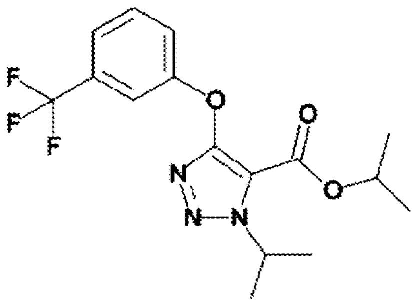 | 1.88min |

[Table 55]

| TABLE 5 | | |
|---|---|---|
| COMPOUND NO. | CHEMICAL FORMULA | LCMS(SQD2) RETENTION TIME |
| f-1 | 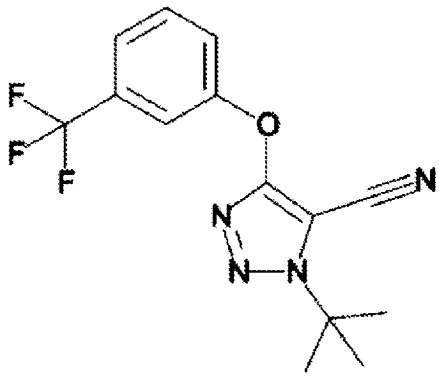 | 1.79min |
| f-2 | 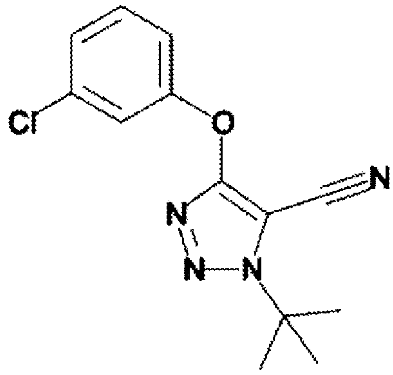 | 1.75min |
| f-3 | 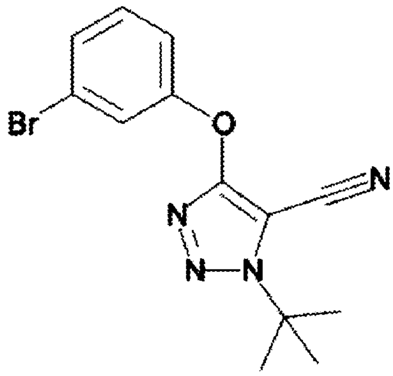 | 1.78min |
| f-4 | 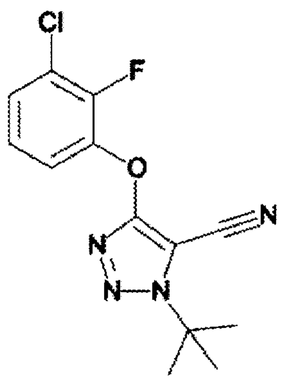 | 1.77min |

[Table 56]

| TABLE 5 (CONTINUED) | | |
|---|---|---|
| COMPOUND NO. | CHEMICAL FORMULA | LCMS(SQD2) RETENTION TIME |
| f-5 | 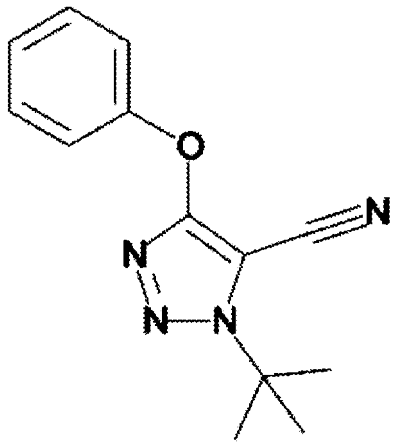 | 1.61min |
| f-6 | 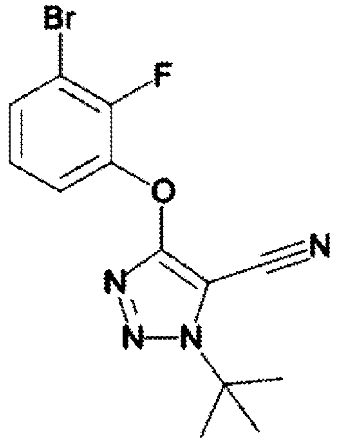 | 1.78min |

The effects of the present invention are exemplified by the following fungicidal effect test and disease control test.

(Fungicidal effect test against fungi causing gray mold)

A spore suspension of fungi causing gray mold disease (Botrytis cinerea, Vogel medium) was prepared. A five-membered heteroaryl compound according to the present invention was added to dimethyl sulfoxide to obtain a drug liquid. This drug liquid and the spore suspension were mixed in equal amounts (based on volume) to obtain a test liquid in which the concentration of the five-membered heteroaryl compound according to the present invention was 25 ppm by volume. The test liquid was added dropwise into a 96-well microplate (area treated with drug).

Dimethyl sulfoxide and the spore suspension were mixed in equal amounts (based on volume) to obtain a control liquid. The control liquid was added dropwise into a 96-well microplate (untreated area).

The microplates were left still in a dark place at 20°C for five days to carry out cultivation. The turbidity OD at a wavelength of 405 nm was measured using a microplate reader. The growth inhibition ratio was calculated by the following equation.

$$\text{Growth inhibition ratio (\%)} = 100\text{-}100\times \{[(\text{OD of area treated with drug after cultivation})\text{-}(\text{OD of area treated with drug before cultivation})] / [(\text{OD of untreated area after cultivation})\text{-}(\text{OD of untreated area before cultivation})]\}$$

Compound Nos. c-1, c-2, c-6, c-9 to c-11, c-13 to c-18, c-20, c-22 to c-24, c-26 to c-35, c-38 to c-45, c-47 to c-51, c-53 to c-60, c-62, c-64 to c-67, c-70 to c-83, c-86, c-87, c-89 to c-104, c-106 to c-118, c-121 to c-137, and c-139 to c-156 were subjected to the fungicidal effect test against fungi causing gray mold. The growth inhibition ratios of all compounds were at least 75%.

(Fungicidal effect test against fungi causing grape ripe rot disease)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing grape ripe rot disease (Glomerella cingulata, Vogel medium), and the temperature and the time period when left still were changed to 25°C and three days.

Compound Nos. c-1, c-2, c-4 to c-6, c-9 to c-11, c-13 to c-18, c-20, c-22 to c-24, c-26 to c-35, c-38 to c-67, c-70 to c-83, c-86 to c-104, c-106 to c-118, and c-121 to c-156 were subjected to the fungicidal effect test against fungi causing grape ripe rot disease. The growth inhibition ratios of all compounds were at least 75%.

(Fungicidal effect test against fungi causing apple anthracnose)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing apple anthracnose (Colletotrichum acutatum, Czapek medium), and the temperature and the time period when left still were changed to 25°C and four days.

Compound Nos. c-1, c-2, c-4 to c-6, c-9 to c-11, c-13 to c-18, c-20, c-22 to c-24, c-26 to c-35, c-38 to c-45, c-47 to c-51, c-53 to c-60, c-62, c-64 to c-67, c-70 to c-83, c-86, c-87, c-89 to c-104, c-106 to c-118, and c-121 to c-156 were subjected to a fungicidal effect test against fungi causing apple anthracnose. The growth inhibition ratios of all compounds were at least 75%.

(Fungicidal effect test against fungi causing wheat Fusarium head blight)

The turbidity was measured to calculate the growth inhibition ratio by the same method as the fungicidal effect test against fungi causing gray mold, except that the spore suspension of fungi causing gray mold disease (Botrytis cinerea, Vogel medium) was replaced with a spore suspension of fungi causing wheat Fusarium head blight disease (Fusarium graminearum, SD medium), and the temperature and the time period when left still were changed to 25°C and four days.

Compound Nos. c-14, c-15, c-18, c-20, c-22 to c-24, c-27, c-28, c-32, c-33, c-35, c-38 to c-44, c-48, c-49, c-53, c-55, c-56, c-60, c-62, c-64 to c-67, c-72 to c-74, and c-76 to c-83 were subjected to the fungicidal effect test against fungi causing wheat Fusarium head blight. The growth inhibition ratios of all compounds were at least 75%.

(Control test of cucumber gray mold)

5 parts by mass of a five-membered heteroaryl compound according to the present invention, 93.5 parts by mass of N,N-dimethylformamide, and 1.5 parts by mass of polyoxyethylene sorbitan monolaurate were mixed together to obtain an emulsion (1) having 5% by mass of the active ingredient.

Water was added to the emulsion (1) to obtain a drug liquid in which the concentration of the five-membered heteroaryl compound according to the present invention was 125 ppm by mass or 25 ppm by mass. Then, the drug liquid was sprayed onto young cucumber seedlings cultivated in a nursery pot (variety "Jihai"-based, cotyledon stage), and then air-dried. Then, a conidiospore suspension of fungi causing cucumber gray mold disease (Botrytis cinerea) was inoculated dropwise thereon (treated area). As a control, a conidiospore suspension of fungi causing cucumber gray mold disease

(Botrytis cinerea) was inoculated dropwise onto young cucumber seedlings unsprayed with the drug liquid (untreated area). They were left still in a moist room at 20°C. Four days after inoculation, leaves of the young cucumber seedlings were visually observed, and the control value was calculated from the lesion area ratio in accordance with the following equation.

$$\text{Control value (\%)} = 100-\{\text{the lesion area ratio at the treated area / the lesion area ratio at the untreated area}\} \times 100$$

Compound Nos. c-16 to c-18, c-20, c-22, c-23, c-27 to c-30, c-32 to c-34, c-38 to c-42, c-44, c-45, c-47 to c-49, c-55 to c-58, c-60, c-64 to c-67, c-70 to c-78, c-80 to c-83, c-86, c-87, c-90 to c-95, c-97 to c-103, c-106, c-109 to c-117, c-121, c-122, c-124, c-127 to c-136, c-140 to c-149, c-151 to c-153, c-155, and c-156 were subjected to the control test of cucumber gray mold at the concentration of 125 ppm by mass. The control values of all compounds were at least 75%.

Compound Nos. c-16 to c-18, c-20, c-22, c-23, c-27 to c-30, c-33, c-38 to c-42, c-44, c-45, c-49, c-55, c-60, c-64 to c-67, c-71, c-72, c-74, c-76 to c-78, c-80, c-81, c-83, c-86, c-87, c-90 to c-95, c-97 to c-103, c-106, and c-109 to c-116 were subjected to the control test of cucumber gray mold at the concentration of 25 ppm by mass. The control values of all compounds were at least 75%.

(Control test of apple scab disease)

Water was added to the emulsion (1) such that the concentration of the five-membered heteroaryl compound according to the present invention was 125 ppm by mass, followed by dissolving it therein to obtain a drug liquid. Then, the drug liquid was sprayed onto young apple seedlings cultivated in a nursery pot (variety "Orin", three- to four-leaf stage), and then air-dried. Thereafter, conidiospores of fungi causing apple scab disease (Venturia inaequalis) were inoculated thereon (treated area). As a control, the conidiospores were inoculated in a similar manner to the above on young apple seedlings unsprayed with the drug liquid (untreated area). They were left still at 20°C in a moist room with light and dark cycles repeated every 12 hours.

12 Days to 14 days after inoculation, leaves of young apple seedlings were visually observed, and the control value was calculated from the lesion area ratio in accordance with the equation used in the control test of cucumber gray mold.

Compound Nos. c-2, c-11, c-18 to c-20, c-23, c-27, c-28, c-33, c-34, c-39 to c-42, c-44, c-48, c-55, c-60, c-64, c-65, c-67, c-71 to c-74, c-77, c-82, c-83, c-87, c-91 to c-100, c-102, c-103, c-106, c-109, c-110, c-117, c-122, and c-124 to c-126 were subjected to the control test of apple scab disease. The control values of all compounds were at least 75%.

(Control test of wheat powdery mildew disease)

Water was added to the emulsion (1) such that the concentration of the five-membered heteroaryl compound according to the present invention was 125 ppm by mass, followed by dissolving it therein to obtain a drug liquid. Then, the drug liquid was sprayed onto young wheat seedlings cultivated in a nursery pot (variety "Chihoku", oneto two-leaf stage), and then air-dried. Thereafter, conidiospores of fungi causing wheat powdery mildew disease (Erysiphe graminis f.sp.tritici) were sprinkled on the young wheat seedlings to inoculate them (treated area). As a control, the conidiospores of fungi causing wheat powdery mildew disease (Erysiphe graminis f.sp.tritici) were sprinkled on young wheat seedlings unsprayed with the drug liquid (untreated area). They were left still in a greenhouse at 20°C. Six days after inoculation, leaves of young wheat seedlings were visually observed, and the lesion area ratio was determined to calculate the control value in a similar manner to the previous test.

Compound Nos. c-15 to c-18, c-20, c-22, c-23, c-27, c-28, c-33, c-38 to c-42, c-44, c-48, c-60, c-64, c-65, c-67, c-71 to c-74, c-76 to c-83, c-86, c-91 to c-95, c-97, c-98, c-100 to c-103, c-106, c-109 to c-113, c-124, c-125, c-127, and c-131 to c-136 were subjected to the control test of wheat powdery mildew disease. The control values of all compounds were at least 75%.

Since all compounds selected from the five-membered heteroaryl compounds according to the present invention at random exhibited the above-mentioned effects, it can be understood that the five-membered heteroaryl compounds according to the present invention, encompassing compounds not listed as examples, have fungicidal effects, and do not cause phytotoxicity to plants.

INDUSTRIAL APPLICABILITY

The five-membered heteroaryl compound according to the present invention exhibits excellent fungicidal activity against pathogens that affect agricultural and horticultural plants, has excellent safety, and can be synthesized in an industrially advantageous manner. The agricultural and horticultural fungicide according to the present invention can effectively prevent diseases in agricultural and horticultural plants.

## Claims

**1.** A compound of formula (Z-1) or a salt thereof,

[Chemical Formula 1]

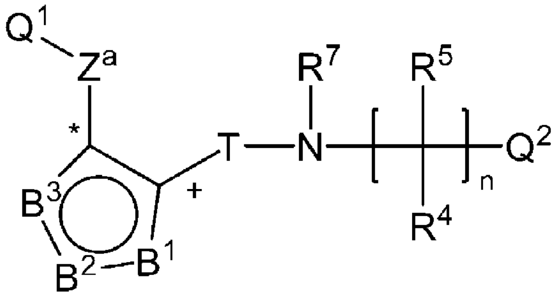

(Z − I)

in the formula (Z-1),
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in a partial structure of the following formula (III):

[Chemical Formula 2]

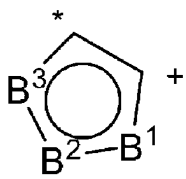

(III)

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,
$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted

naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure of the formula (III) correspond to carbon atoms marked with * and + in the formula (Z-1),
$Z^a$ is an oxygen atom or a sulfur atom,
T is a partial structure (T-1) or a partial structure (T-2),

[Chemical Formula 3]

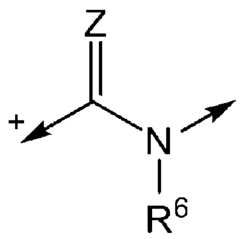
(T − 1)
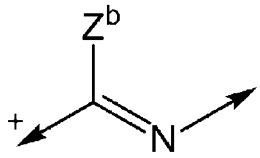
(T − 2)

in the partial structure (T-1) or the partial structure (T-2), an arrow marked with + is bonded to a carbon atom marked with + in the partial structure of the formula (III), and the other arrow is bonded to a nitrogen atom of NR7 in the formula (Z-1),
in the partial structure (T-1), Z is an oxygen atom or a sulfur atom,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
in the partial structure (T-2), $Z^b$ is a C1-6 alkoxy group or a C1-6 alkylthio group,
$R^7$ is a hydrogen atom, a substituted or unsubstituted C1-6 alkyl group, a substituted or unsubstituted C2-6 alkenyl group, a substituted or unsubstituted C2-6 alkynyl group, a formyl group, a group of formula: $R^c$-CO-, a group of formula: $R^c$-CS-, a group of formula: $R^c$-CO-CO-, a group of formula: $R^c$-$SO_2$-, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a cyano group,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group, and
n is an integer of 1 to 3.

**2.** The compound or the salt thereof according to claim 1, wherein the formula (Z-1) is formula (I),

[Chemical Formula 4]

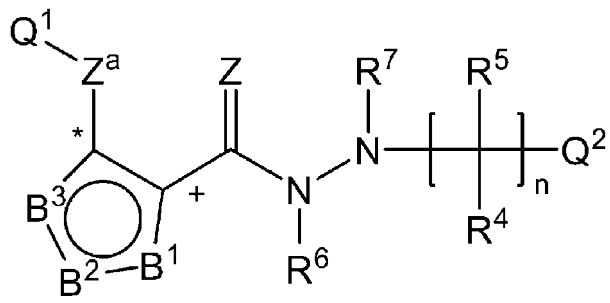

( I )

in the formula (I),
$Q^1$, $Q^2$, $B^1$, $B^2$, $B^3$, $Z^a$, $R^4$, $R^5$, $R^7$ and n are identical to $Q^1$, $Q^2$, $B^1$, $B^2$, $B^3$, $Z^a$ , $R^4$, $R^5$, $R^7$ and n in the formula (Z-1), and
Z and $R^6$ are identical to Z and $R^6$ in the partial structure (T-1).

**3.** The compound or the salt thereof according to claim 2, wherein

$Z^a$ is an oxygen atom,
Z is an oxygen atom,
$R^6$ is a hydrogen atom,
$R^7$ is a formyl group, a group of formula: $R^c$-CO-, a group of formula: $R^c$-CS-, a group of formula: $R^c$-CO-CO-, or a group of formula: $R^c$-$SO_2$-, and
n is 1.

**4.** An agricultural and horticultural fungicide comprising at least one selected from the group consisting of a compound and a salt thereof of any one of claims 1 to 3, as an active ingredient.

**5.** A method for producing a compound of formula (I-1), the method comprising a step in which a compound of formula (im-1) and a compound of formula (im-2) are reacted in the presence of an acid scavenger,

[Chemical Formula 5]

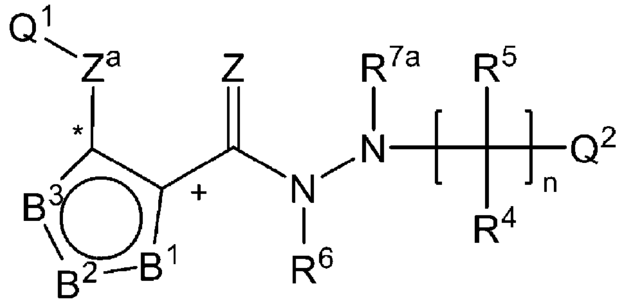

( I − 1 )

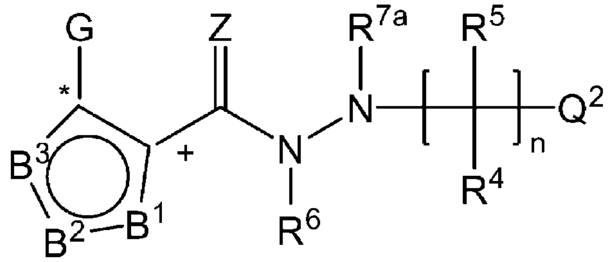

( i m − 1 )

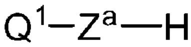

( i m − 2 )

in the formulae,
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in a partial structure shown below:

[Chemical Formula 6]

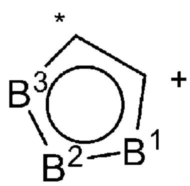

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,
$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or

unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-1) or the formula (im-1),
$Z^a$ is an oxygen atom or a sulfur atom,
Z is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group, and
G is a halogeno group.

**6.** A method for producing a compound of formula (I-2), the method comprising a step in which a compound of formula (im-3) and a compound of formula (im-4) are reacted in the presence of an organic base and a copper catalyst with oxygen as an oxidizing agent,

[Chemical Formula 7]

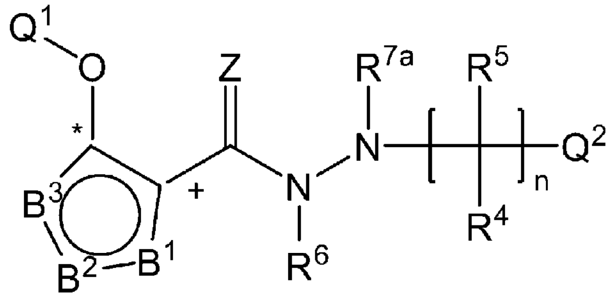

(I-2)

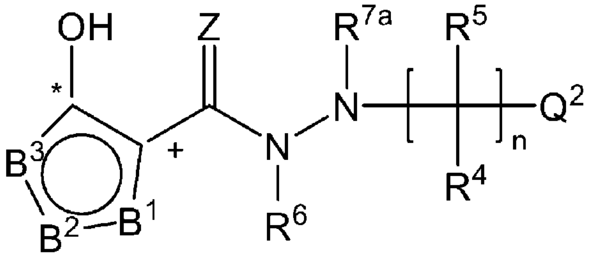

(im-3)

$Q^1-B(OH)_2$ (im-4)

in the formulae,

$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,

$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,

in a partial structure shown below:

[Chemical Formula 8]

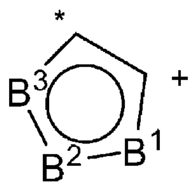

$B^1$ is an oxygen atom, a nitrogen atom, a group of formula: $NR^1$, or a group of formula: $CR^2$,

$B^2$ is an oxygen atom, a nitrogen atom, a group of formula: $NR^1$, or a group of formula: $CR^2$,

$B^3$ is an oxygen atom, a nitrogen atom, a group of formula: $NR^1$, or a group of formula: $CR^2$,

one of $B^1$, $B^2$ and $B^3$ is an oxygen atom, or a group of formula: $NR^1$, and remaining two thereof are a nitrogen atom or a group of formula: $CR^2$,

$R^1$ is each independently a substituted or unsubstituted C1-6 alkyl group, a substituted or unsubstituted C3-6 cycloalkyl group, or a substituted or unsubstituted five-membered or six-membered heterocyclyl group,

$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted

or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-2) or the formula (im-3),
Z is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
$R^{7a}$ is a formyl group, a group of formula: $R^{c-}$CO-, a group of formula: $R^{c-}$CS-, a group of formula: $R^{c-}$CO-CO-, or a group of formula: $R^{c-}SO_2$-, and
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted three-membered to six-membered heterocyclyl group, or a substituted or unsubstituted three-membered to six-membered heterocyclyloxy group.

**7.** A method for producing a compound of formula (1-3), the method comprising a step in which a compound of formula (im-5) and a compound of formula (im-6) are reacted in the presence of a condensing agent in a case in which $U^1$ in the formula (im-5) is a hydroxy group, or in the presence of an acid scavenger in a case in which $U^1$ is a halogeno group,

[Chemical Formula 9]

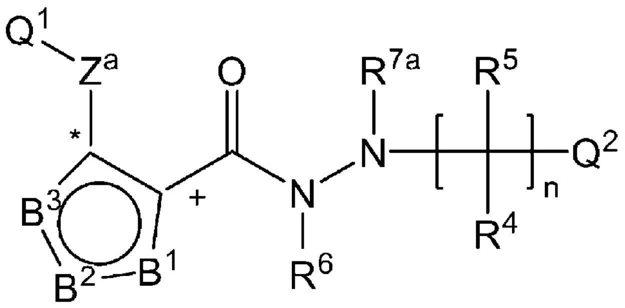

(I－3)

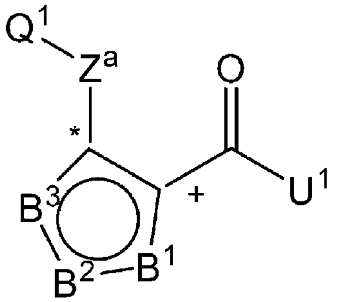

(im－5)

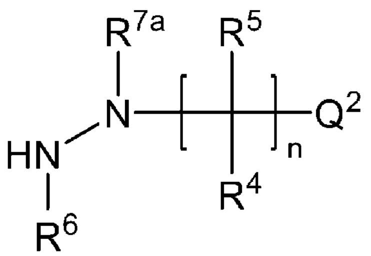

(im－6)

in the formulae,
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$Q^2$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
in a partial structure shown below:

[Chemical Formula 10]

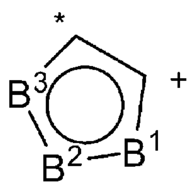

$B^1$ is an oxygen atom, a nitrogen atom, a group of formula: $NR^1$, or a group of formula: $CR^2$,
$B^2$ is an oxygen atom, a nitrogen atom, a group of formula: $NR^1$, or a group of formula: $CR^2$,
$B^3$ is an oxygen atom, a nitrogen atom, a group of formula: $NR^1$, or a group of formula: $CR^2$,
one of $B^1$, $B^2$ and $B^3$ is an oxygen atom, or a group of formula: $NR^1$, and remaining two thereof are a nitrogen atom or a group of formula: $CR^2$,
$R^1$ is each independently a substituted or unsubstituted C1-6 alkyl group, a substituted or unsubstituted C3-6 cycloalkyl group, or a substituted or unsubstituted five-membered or six-membered heterocyclyl group,
$R^2$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylcarbonyl group, a substituted or unsubstituted C1-6 alkoxycarbonyl group, a substituted or unsubstituted C1-6 alkylcarbonyloxy group, a substituted or unsubstituted C1-6 alkoxycarbonyloxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, a substituted or unsubstituted phenyloxy group, a substituted or unsubstituted naphthyloxy group, a substituted or unsubstituted five-membered or six-membered heterocyclyloxy group, a substituted or unsubstituted amino group, a substituted or unsubstituted aminocarbonyl group, a nitro group, a cyano group, or a halogeno group,
carbon atoms marked with * and + in the partial structure correspond to carbon atoms marked with * and + in the formula (I-3) or the formula (im-5),
$Z^a$ is an oxygen atom or a sulfur atom,
$R^4$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^5$ is each independently a hydrogen atom, a substituted or unsubstituted C1-6 alkyl group, a substituted or unsubstituted C2-6 alkenyl group, a substituted or unsubstituted C2-6 alkynyl group, a substituted or unsubstituted C3-6 cycloalkyl group, a substituted or unsubstituted phenyl group, a substituted or unsubstituted naphthyl group, a substituted or unsubstituted five-membered or six-membered heterocyclyl group, or a halogeno group,
$R^4$ and $R^5$ on an identical carbon atom may form together a divalent organic group,
n is an integer of 1 to 3,
$R^6$ is a hydrogen atom, a substituted or unsubstituted C1-6 alkyl group, a substituted or unsubstituted C2-6 alkenyl group, a substituted or unsubstituted C2-6 alkynyl group, a formyl group, a substituted or unsubstituted C1-6 alkylcarbonyl group, a substituted or unsubstituted C1-6 alkoxycarbonyl group, a substituted or unsubstituted C3-6 cycloalkyl group, a substituted or unsubstituted phenyl group, a substituted or unsubstituted naphthyl group, or a substituted or unsubstituted five-membered or six-membered heterocyclyl group,
$R^{7a}$ is a formyl group, a group of formula: $R^c$-CO-, a group of formula: $R^c$-CS-, a group of formula: $R^c$-CO-CO-, or a group of formula: $R^c$-$SO_2$-,
$R^c$ is each independently a substituted or unsubstituted C1-6 alkyl group, a substituted or unsubstituted C1-6 alkoxy group, a substituted or unsubstituted C3-6 cycloalkoxy group, a substituted or unsubstituted C1-6 alkylthio group, a substituted or unsubstituted amino group, a substituted or unsubstituted C3-6 cycloalkyl group, a substituted or unsubstituted phenyl group, a substituted or unsubstituted naphthyl group, a substituted or unsubstituted or unsubstituted three-membered to six-membered heterocyclyl group, or a substituted or unsubstituted three-membered to six-membered heterocyclyloxy group, and
$U^1$ is a hydroxy group or a halogeno group.

**8.** A compound of formula (im-5A) or a salt thereof,

[Chemical Formula 11]

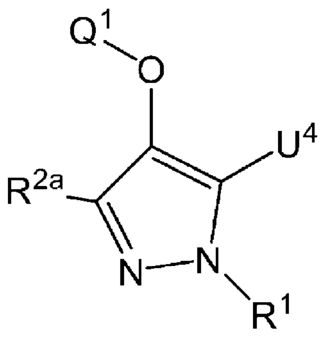
(im-5A)

in the formula (im-5A),
$Q^1$ is a substituted or unsubstituted phenyl group, a substituted or unsubstituted naphthyl group, a substituted or

unsubstituted five-membered or six-membered heterocyclyl group, or a substituted or unsubstituted nine-membered or ten-membered heterocyclyl group,
$R^1$ is a substituted or unsubstituted C1-6 alkyl group, a substituted or unsubstituted C3-6 cycloalkyl group, or a substituted or unsubstituted five-membered or six-membered heterocyclyl group,
$R^{2a}$ is a C1-6 haloalkyl group, a cyano group, or a halogeno group, and
$U^4$ is a carboxyl group, a C1-6 alkoxycarbonyl group, or a halogenocarbonyl group.

**9.** A compound of formula (im-5B) or a salt thereof,

[Chemical Formula 12]

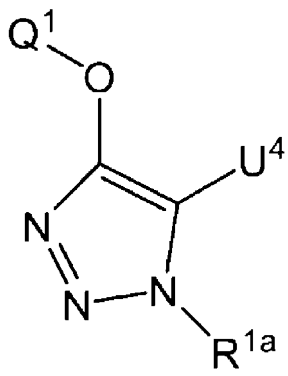

(im-5B)

in the formula (im-5B),
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group,
$R^{1a}$ is a substituted or unsubstituted C2-6 alkyl group, or a substituted or unsubstituted C3-6 cycloalkyl group, and
$U^4$ is a carboxyl group, a C1-6 alkoxycarbonyl group, or a halogenocarbonyl group.

**10.** A compound of formula (im-25) or a salt thereof,

[Chemical Formula 13]

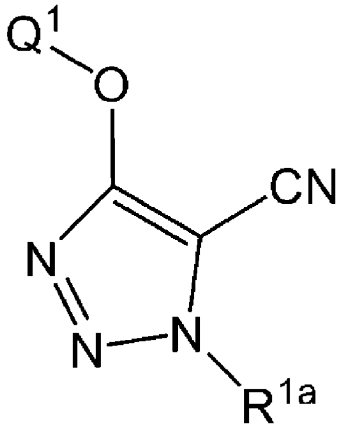

(im-25)

in the formula (im-25),
$Q^1$ is a substituted or unsubstituted phenyl group, a substituted or unsubstituted naphthyl group, a substituted or unsubstituted five-membered or six-membered heterocyclyl group, or a substituted or unsubstituted nine-membered or ten-membered heterocyclyl group, and
$R^{1a}$ is a substituted or unsubstituted C2-6 alkyl group, or a substituted or unsubstituted C3-6 cycloalkyl group.

## INTERNATIONAL SEARCH REPORT

International application No.
**PCT/JP2024/023210**

### A. CLASSIFICATION OF SUBJECT MATTER

***C07D 231/18***(2006.01)i; ***A01N 47/24***(2006.01)i; ***A01P 3/00***(2006.01)i; ***C07D 231/20***(2006.01)i; ***C07D 233/90***(2006.01)i; ***C07D 249/04***(2006.01)i; ***C07D 401/12***(2006.01)i; ***C07D 405/12***(2006.01)i; ***C07D 409/12***(2006.01)i; ***C07D 417/12***(2006.01)i
FI: C07D231/18 CSP; A01N47/24 Z; A01P3/00; C07D231/20 Z; C07D249/04 506; C07D401/12; C07D417/12; C07D233/90 Z; C07D409/12; C07D405/12

According to International Patent Classification (IPC) or to both national classification and IPC

### B. FIELDS SEARCHED

Minimum documentation searched (classification system followed by classification symbols)

C07D231/18; A01N47/24; A01P3/00; C07D231/20; C07D233/90; C07D249/04; C07D401/12; C07D405/12; C07D409/12; C07D417/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| A | JP 2022-508277 A (BAYER AKTIENGESELLSCHAFT) 19 January 2022 (2022-01-19) claims, examples | 1-10 |
| A | JP 2008-521856 A (BASF SE) 26 June 2008 (2008-06-26) claims, examples | 1-10 |
| A | JP 4-145081 A (KUMIAI CHEMICAL INDUSTRY CO., LTD.) 19 May 1992 (1992-05-19) claims, table 3, examples | 1-10 |
| A | US 5510320 A (E. I. DU PONT DE NEMOURS AND COMPANY) 23 April 1996 (1996-04-23) claims, table 4, examples | 1-10 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 September 2024** | **10 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/023210**

| Patent document cited in search report | Publication date (day/month/year) | Patent family member(s) | Publication date (day/month/year) |
|---|---|---|---|
| JP 2022-508277 A | 19 January 2022 | US 2023/0054449 A1<br>claims, examples<br>WO 2020/109391 A1<br>EP 3887362 A1<br>CN 113490663 A | |
| JP 2008-521856 A | 26 June 2008 | US 2007/0265231 A1<br>claims, examples<br>WO 2006/058730 A1<br>EP 1819224 A1<br>KR 10-2007-0090898 A<br>CN 101068468 A | |
| JP 4-145081 A | 19 May 1992 | (Family: none) | |
| US 5510320 A | 23 April 1996 | WO 1993/009100 A1<br>claims, table 4, examples<br>EP 610314 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023131664 A
- JP 2024007491 A
- JP 2024068592 A
- JP 2022508277 W
- JP 2022514651 A


### Non-patent literature cited in the description


- *CHEMICAL ABSTRACTS*, 943137-49-3